(19) Europäisches Patentamt
European Patent Office
Office européen des brevets

(11) EP 4 417 618 A1

(12) EUROPEAN PATENT APPLICATION
published in accordance with Art. 153(4) EPC

(43) Date of publication:
**21.08.2024 Bulletin 2024/34**

(21) Application number: **22880418.3**

(22) Date of filing: **14.10.2022**

(51) International Patent Classification (IPC):
***C07K 7/08** (2006.01)* ***A61P 35/00** (2006.01)*
***A61K 47/64** (2017.01)* ***C07K 14/00** (2006.01)*

(52) Cooperative Patent Classification (CPC):
**A61K 47/64; A61P 35/00; C07K 7/08; C07K 14/00**

(86) International application number:
**PCT/CN2022/125377**

(87) International publication number:
**WO 2023/061482 (20.04.2023 Gazette 2023/16)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **14.10.2021 CN 202111180715**
**24.01.2022 CN 202210081789**
**17.05.2022 CN 202210540531**
**27.06.2022 CN 202210737371**

(71) Applicant: **Xizang Haisco Pharmaceutical Co., Ltd.**
**Lhoka, Tibet 856099 (CN)**

(72) Inventors:
- **LI, Yao**
**Chengdu, Sichuan 611130 (CN)**
- **CHEN, Lei**
**Chengdu, Sichuan 611130 (CN)**
- **HUANG, Haitao**
**Chengdu, Sichuan 611130 (CN)**
- **WANG, Haodong**
**Chengdu, Sichuan 611130 (CN)**
- **TANG, Pingming**
**Chengdu, Sichuan 611130 (CN)**
- **YU, Yan**
**Chengdu, Sichuan 611130 (CN)**
- **ZHANG, Chen**
**Chengdu, Sichuan 611130 (CN)**
- **YAN, Pangke**
**Chengdu, Sichuan 611130 (CN)**

(74) Representative: **IP TRUST SERVICES**
**Europole - Le Grenat**
**3, avenue Doyen Louis Weil**
**38000 Grenoble (FR)**

Remarks:
The complete document including Reference Table(s) and the Sequence Listing(s) can be downloaded from the EPO website

# (54) EPHA2 BICYCLIC PEPTIDE LIGAND AND CONJUGATE THEREOF

(57) Disclosed are a bicyclic peptide ligand having high affinity for Eph receptor tyrosine kinase A2 (EphA2), a drug conjugate containing the bicyclic peptide ligand and a pharmaceutically acceptable salt thereof, and a pharmaceutical composition containing same, and the use of the drug conjugate and the pharmaceutical composition in the preparation of a drug for preventing and/or treating EphA2 overexpressed disease.

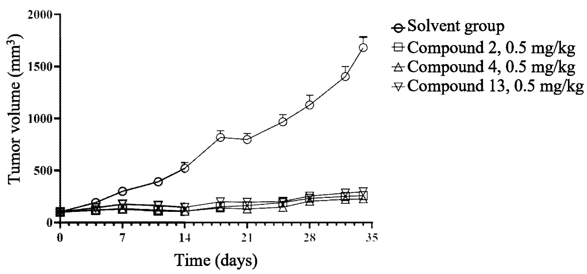


FIG. 1

EP 4 417 618 A1

## Description

### Technical Field

The present invention relates to a peptide ligand having high affinity for Eph receptor tyrosine kinase A2 (EphA2). The present invention also relates to a polypeptide-drug conjugate that conjugates the peptide ligand to one or more toxin molecules and the use of the peptide ligand and the drug conjugate in a drug for preventing and/or treating EphA2 overexpressed disease.

### Background Art

Eph receptors (Ephs) are an important class of receptor tyrosine kinases (RTKs). Eph receptor signalling is involved in various biological events and mainly results in cell-cell repulsion or cell-cell adhesion. Therefore, Eph receptors and corresponding ligands have important functions in embryonic tissue architecture, neuronal targeting, and vascular development. Moreover, high levels of Eph proteins are found in various malignant tumours, and the overexpression greatly promotes the development of cancer. Some Eph receptors, especially EphA2, have been shown to play an important role in the regulation of cancer development and tumour progression.

EphA2 is a transmembrane tyrosine kinase receptor and a member of the receptor tyrosine kinase (RTK) superfamily. Like other tyrosine kinases, the EphA2 molecule can be divided into three domains, including an extracellular ligand-binding region, an intracellular tyrosine kinase active functional region, and a transmembrane region. Unlike most Eph kinases, which are synthesized during development, EphA2 is primarily localized to proliferative epithelial cells in adults. Adult EphA2 is expressed in normal tissues only when there are highly proliferative epithelial cells. However, accumulating evidence indicates that human EphA2 is abundantly expressed in prostate cancer, lung cancer, oesophageal cancer, colorectal carcinoma, cervical cancer, ovarian cancer, breast cancer and skin cancer. The expression of EphA2 is associated with poor prognosis, increased metastatic potential and reduced survival of tumour patients. Therefore, the transcriptional pattern and functional relevance in malignant tumours of EphA2 make the protein an attractive target for cancer therapy.

EphA2-targeted therapies have emerged in clinical trials for various types and stages of malignant tumours. Clinical strategies to inhibit EphA2 include EphA2-targeting antibody-cytotoxic drug conjugates (ADCs) or peptide-drug conjugates (PDCs); tyrosine kinase inhibitors (TKIs), such as dasatinib, which has been approved for marketing; CAR-T cells that recognize and target EphA2 antigen; and nanocarriers designed to deliver EphA2-targeting siRNAs to tumour cells.

Although there is a wealth of data supporting the use of EphA2 drugs in cancer treatment, there still remain several challenges. EphA2 is expressed in various cell and tissue types, and its expression in normal tissues may lead to unexpected toxic and side effects. The diverse signalling modes of the EphA2 receptor also complicate the targeting strategies, and the most suitable approach may vary depending on the environment. In addition, because EphA2 may be kinase-independent, blocking the receptor's kinase activity with traditional small molecule inhibitors targeting RTKs may not inhibit EphA2 ligand-independent oncogenic effect. In view of the role of EphA2 in tumour development and the limitations of the existing EphA2-targeting drugs, it will be of important clinical significance to develop ligands that target EphA2 with good efficacy and high selectivity for the treatment of patients with EphA2 overexpressed tumours.

### Summary of the Invention

Firstly, the present invention provides a peptide ligand specific for EphA2, wherein the peptide ligand has a bicyclic peptide structure, demonstrates both high affinity for the target and exceptional selectivity and stability, and further has the advantage of being suitable for injection, inhalation, nasal, ocular, oral or topical administration.

A peptide ligand specific for EphA2, comprising a polypeptide and a non-aromatic molecular scaffold, wherein the polypeptide comprises at least three residues independently selected from Cys and Hcys, and wherein at least one of the residues is a Hcys residue, the three Cys and Hcys residues are separated by two loop sequences, and the Cys or Hcys residue of the polypeptide is connected to the scaffold via a thioether bond, thereby forming two polypeptide loops on the molecular scaffold;

in some specific embodiments, the peptide ligand comprises a polypeptide and a non-aromatic molecular scaffold, wherein the polypeptide comprises at least three residues independently selected from Cys and Hcys, and wherein two of the residues are Hcys residues, the three Cys and Hcys residues are separated by two loop sequences, and the Cys or Hcys residue of the polypeptide is connected to the scaffold via a thioether bond, thereby forming two polypeptide loops on the molecular scaffold;

in some specific embodiments, the peptide ligand comprises a polypeptide and a non-aromatic molecular scaffold, wherein the polypeptide comprises at least three residues independently selected from Cys and Hcys, and

wherein one of the residues is a Hcys residue, the three Cys and Hcys residues are separated by two loop sequences, and the Cys or Hcys residue of the polypeptide is connected to the scaffold via a thioether bond, thereby forming two polypeptide loops on the molecular scaffold;

in some specific embodiments, the peptide ligand comprises a polypeptide and a non-aromatic molecular scaffold, wherein the polypeptide comprises at least three residues independently selected from Cys and Hcys, and wherein two of the residues are Hcys residues, the three Cys and Hcys residues are separated by two loop sequences, the Cys or Hcys residue of the polypeptide is connected to the scaffold via a thioether bond, thereby forming two polypeptide loops on the molecular scaffold, and the molecular scaffold is selected from TATA;

in some specific embodiments, the peptide ligand comprises a polypeptide and a non-aromatic molecular scaffold, wherein the polypeptide comprises at least three residues independently selected from Cys and Hcys, and wherein one of the residues are a Hcys residue, the three Cys and Hcys residues are separated by two loop sequences, the Cys or Hcys residue of the polypeptide is connected to the scaffold via a thioether bond, thereby forming two polypeptide loops on the molecular scaffold, and the molecular scaffold is selected from TATA;

in some specific embodiments, the peptide ligand comprises an amino acid sequence selected from:

Xa1-A1-Xa2-A2-Xa3,

wherein A1 and A2 represent the amino acid residues between Xa1, Xa2 and Xa3, and A1 and A2 each independently comprise 5 or 6 amino acid residues; in some specific embodiments, the amino acid residue comprised in A1 and A2 is optionally selected from Hyp, Leu, Val, Asn, Pro, Leu, His, D-Asp, Trp, hArg, Ser, Thr, Tyr, Val, Ile and Gly, etc.; Xa1, Xa2 and Xa3 are independently a Cys or Hcys residue, and at least one of Xa1, Xa2 and Xa3 is a Hcys residue, and the non-aromatic molecular scaffold and Xa1, Xa2 and Xa3 of the polypeptide form a thioether bond, thereby forming two polypeptide loops on the molecular scaffold;

in some specific embodiments, the polypeptide comprises the amino acid sequence as shown below:

Xa1-Hyp-Leu-Val-Asn-Pro-Leu-Xa2-Leu-His-Pro-(D-Asp)-Trp-(hArg)-Xa3;

in some specific embodiments, the polypeptide comprises an amino acid sequence as shown in any one of SEQ ID NO:1-SEQ ID NO:7:

Hcys-Hyp-Leu-Val-Asn-Pro-Leu-Hcys-Leu-His-Pro-(D-Asp)-Trp-(hArg)-Hcys (SEQ ID NO:1);
Cys-Hyp-Leu-Val-Asn-Pro-Leu-Cys-Leu-His-Pro-(D-Asp)-Trp-(hArg)-Hcys (SEQ ID NO:2);
Cys-Hyp-Leu-Val-Asn-Pro-Leu-Hcys-Leu-His-Pro-(D-Asp)-Trp-(hArg)-Cys (SEQ ID NO:3);
Hcys-Hyp-Leu-Val-Asn-Pro-Leu-Cys-Leu-His-Pro-(D-Asp)-Trp-(hArg)-Cys (SEQ ID NO:4);
Cys-Hyp-Leu-Val-Asn-Pro-Leu-Hcys-Leu-His-Pro-(D-Asp)-Trp-(hArg)-Hcys (SEQ ID NO:5);
Hcys-Hyp-Leu-Val-Asn-Pro-Leu-Cys-Leu-His-Pro-(D-Asp)-Trp-(hArg)-Hcys (SEQ ID NO:6);
Hcys-Hyp-Leu-Val-Asn-Pro-Leu-Hcys-Leu-His-Pro-(D-Asp)-Trp-(hArg)-Cys (SEQ ID NO:7).

In some specific embodiments, the polypeptide sequence of the peptide ligand is selected from one of SEQ ID NO:8-SEQ ID NO:14:

(β-Ala)-$Sar_{10}$-Ala-hArg-Asp-Hcys-Hyp-Leu-Val-Asn-Pro-Leu-Hcys-Leu-His-Pro-(D-Asp)-Trp-(hArg)-Hcys (SEQ ID NO:8);
(β-Ala)-$Sar_{10}$-Ala-hArg-Asp-Cys-Hyp-Leu-Val-Asn-Pro-Leu-Cys-Leu-His-Pro-(D-Asp)-Trp-(hArg)-Hcys (SEQ ID NO:9);
(β-Ala)-$Sar_{10}$-Ala-hArg-Asp-Cys-Hyp-Leu-Val-Asn-Pro-Leu-Hcys-Leu-His-Pro-(D-Asp)-Trp-(hArg)-Cys (SEQ ID NO:10);
(β-Ala)-$Sar_{10}$-Ala-hArg-Asp-Hcys-Hyp-Leu-Val-Asn-Pro-Leu-Cys-Leu-His-Pro-(D-Asp)-Trp-(hArg)-Cys (SEQ ID NO:11);
(β-Ala)-$Sar_{10}$-Ala-hArg-Asp-Cys-Hyp-Leu-Val-Asn-Pro-Leu-Hcys-Leu-His-Pro-(D-Asp)-Trp-(hArg)-Hcys (SEQ ID NO: 12);
(β-Ala)-$Sar_{10}$-Ala-hArg-Asp-Hcys-Hyp-Leu-Val-Asn-Pro-Leu-Cys-Leu-His-Pro-(D-Asp)-Trp-(hArg)-Hcys (SEQ ID NO: 13);
(β-Ala)-$Sar_{10}$-Ala-hArg-Asp-Hcys-Hyp-Leu-Val-Asn-Pro-Leu-Hcys-Leu-His-Pro-(D-Asp)-Trp-(hArg)-Cys (SEQ ID NO: 14).

In some specific embodiments, the peptide ligand is a bicyclic peptide containing one of the following structures and optionally containing an additional amino acid sequence at either the N-terminus or C-terminus of the structures:

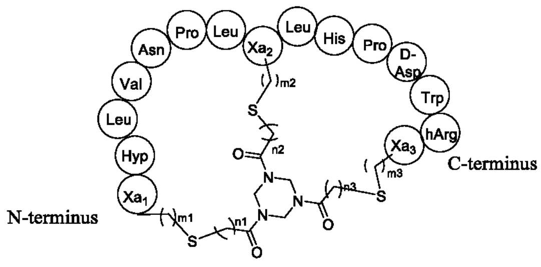

,

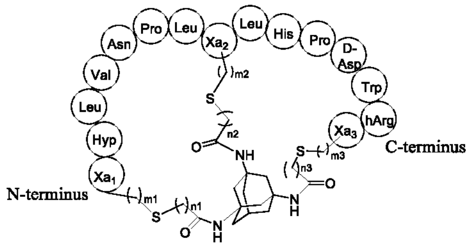

I-1 II-1

wherein the -SH group on the Xa1, Xa2 or Xa3 residue covalently binds to the molecular scaffold to form a thioether bond; thus, when Xa1, Xa2 or Xa3 is Cys, correspondingly, m1, m2 or m3 is 1;

when Xa1, Xa2 or Xa3 is Hcys, correspondingly, m1, m2 or m3 is 2;

n1, n2 and n3 are independently 1 or 2;

in some specific embodiments, the peptide ligand is a bicyclic peptide having one of the following structures, wherein the bicycle is as described above (such as 1-1 or II-1), and t is an integer of 5-15, such as 5, 6, 7, 8, 9, 10, 11, 12, 13, 14, or 15, in some embodiments, t is 10:

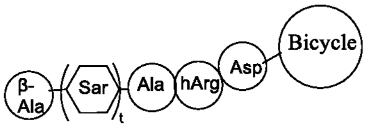

;

in some specific embodiments, the peptide ligand is a bicyclic peptide having one of the following structures:

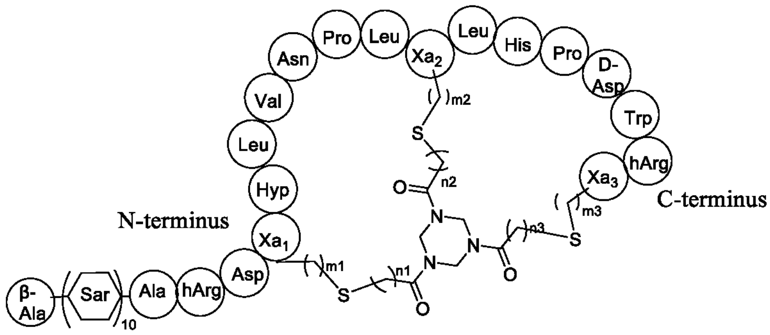

,

I-2

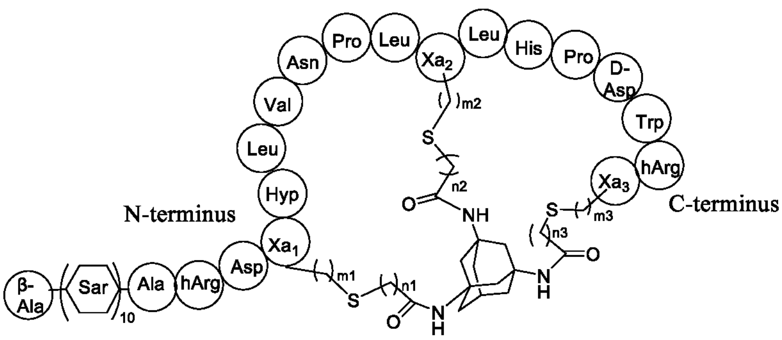

II-2.

In some specific embodiments, the peptide ligand is a bicyclic peptide of formula 1-2 or II-2, wherein

(1) Xa1 is Hcys, m1 is 2, and n1 is 1 or 2; or
(2) Xa2 is Hcys, m2 is 2, and n2 is 1 or 2; or
(3) Xa3 is Hcys, m3 is 2, and n3 is 1 or 2.

Secondly, the present invention also provides a drug conjugate or a pharmaceutically acceptable salt thereof, wherein the conjugate comprises the peptide ligand as described above conjugated to one or more effectors and/or functional groups via a linker; in some specific embodiments, the conjugate comprises the peptide ligand as described above conjugated to one effector and/or functional group via a linker; in some specific embodiments, the conjugate comprises the peptide ligand as described above conjugated to two effectors and/or functional groups via a linker;

in some specific embodiments, the effector is a cytotoxic agent; in some specific embodiments, the cytotoxic agent is selected from the following group: alkylating agents, antimetabolites, plant alkaloids, terpenoids, podophyllotoxins and a derivative thereof, taxanes and a derivative thereof, a topoisomerase inhibitor, and antitumour antibiotics; in some specific embodiments, the cytotoxic agent is selected from the following group: cisplatin, carboplatin, oxaliplatin, mechlorethamine, cyclophosphamide, chlorambucil, ifosfamide, azathioprine, mercaptopurine, a pyrimidine analogue, vincristine, vinblastine, vinorelbine, vindesine, etoposide, teniposide, taxol, camptothecine, irinotecan, topotecan, amsacrine, etoposide, etoposide phosphate, teniposide, actinomycin D, doxorubicin, epirubicin, epothilone and a derivative thereof, bleomycin and a derivative thereof, dactinomycin and a derivative thereof, plicamycin and a derivative thereof, mitomycin C, calicheamycin, maytansine and a derivative thereof, and auristatin and a derivative thereof; in some specific embodiments, the cytotoxic agent is selected from DM1, MMAE or SN38;

in some specific embodiments, the linker is a peptide linker, a disulphide linker, an enzyme-dependent linker, or a pH-dependent linker; and these linkers can be cleaved under certain conditions to release the effector molecule;

in some specific embodiments, the linker is a peptide linker, a disulphide linker or a pH-dependent linker; and these linkers can be cleaved under certain conditions to release the effector molecule;

in some specific embodiments, the disulphide linker is selected from DMDS, MDS, DSDM, NDMDS or a structure of formula III:

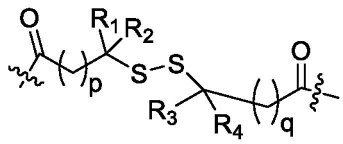

III

wherein $R_1$, $R_2$, $R_3$ and $R_4$ are independently selected from H, methyl, ethyl, propyl and isopropyl;
p and q are independently 1, 2, 3, 4 or 5;
the peptide linker is selected from: -Cit-Val-, -Phe-Lys- and -Val-Lys-;
the enzyme-dependent linker is selected from:

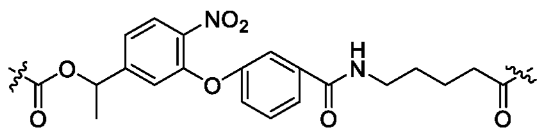
;

the pH-dependent linker is selected from a cis-aconitic anhydride;
the above-mentioned linkers mainly serve to connect the cytotoxic agent and the peptide ligand and release the cytotoxic agent by being cleaved under specific conditions; to control the cleavage rate and the accompanying release of the effector molecule, the linkers can be appropriately modified, for example, some groups can be connected at the connection site of the linker and the peptide ligand or effector to increase the length of the chain, and group modifications can be introduced around the cleavage bond to control the hindrance during the cleaving process, and the linkers of the present invention comprise the linker derivatives obtained by introducing modifications on the basis of the above-mentioned linkers;

in some specific embodiments, the linker is -PABC-Cit-Val-glutaryl-, -PABC-cyclobutyl-Ala-Cit-βAla-, or

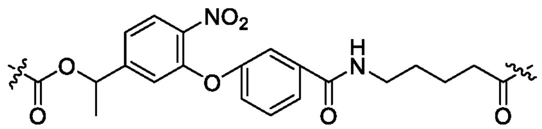

,

wherein PABC represents p-aminobenzylcarbamate; in some specific embodiments, the linker is (-PABC-Cit-Val-CO-$CH_2)_2N$-$CH_2(CH_2OCH_2)_2CH_2NH$-glutaryl-;

in some specific embodiments, the drug conjugate has a structure of formula I, formula II, formula III, formula IV, V or VI:

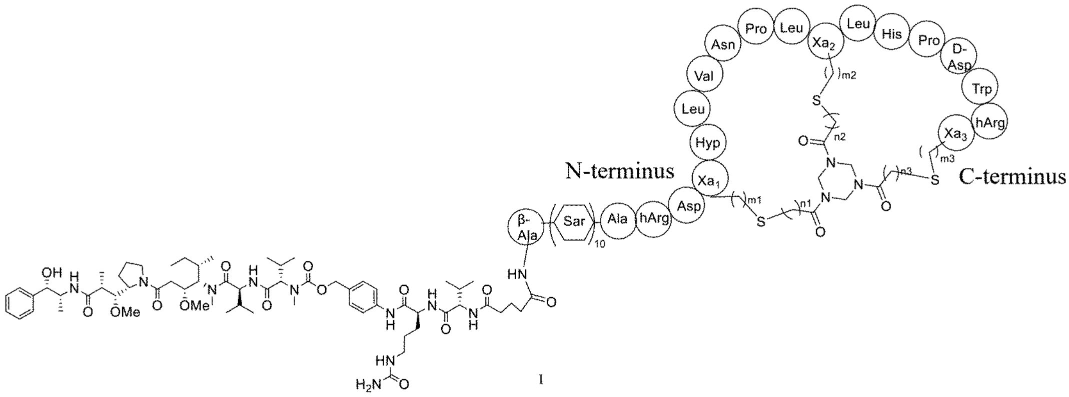

I

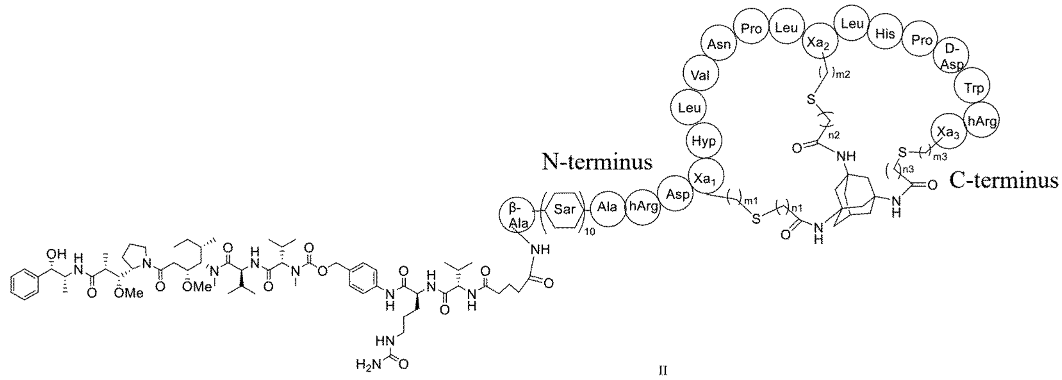

II

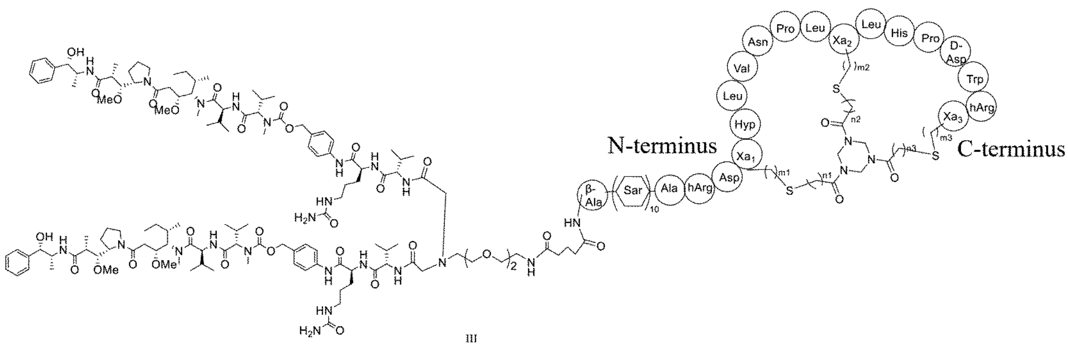

III

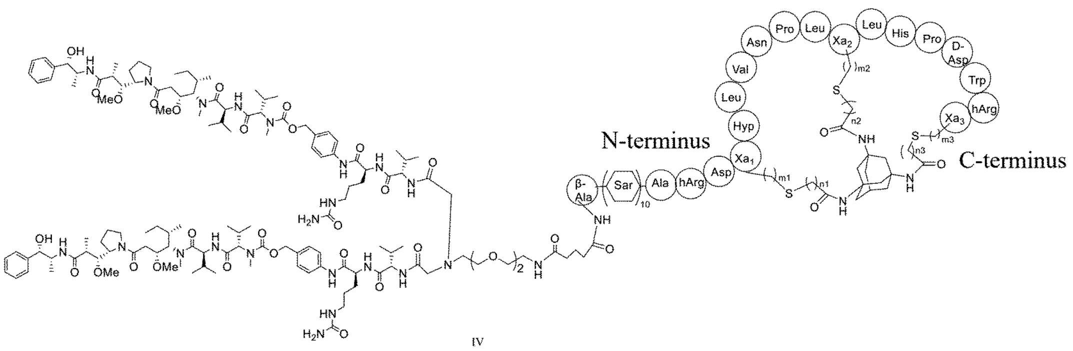

IV

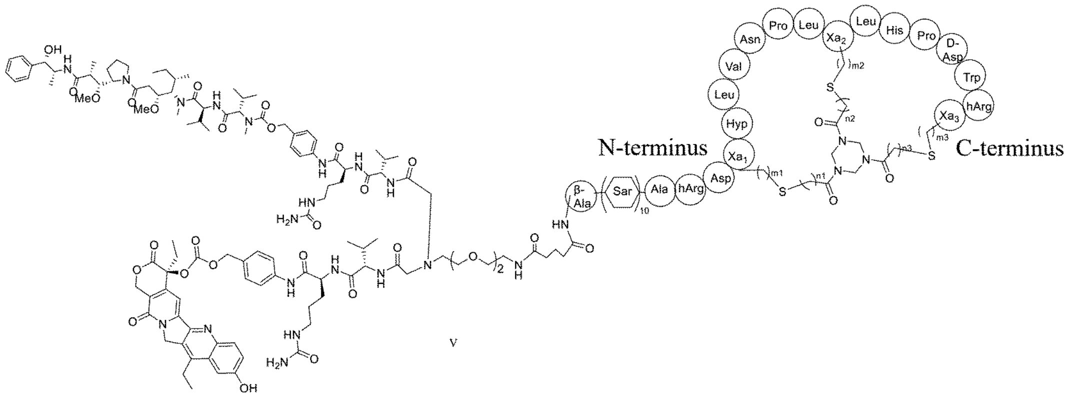

V

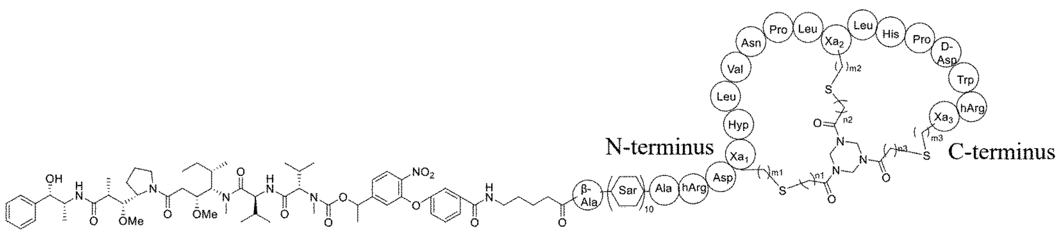

VI

wherein Xa1, Xa2 and Xa3 are independently a Cys or Hcys residue, and at least one of Xa1, Xa2 and Xa3 is a Hcys residue;
when Xa1, Xa2 or Xa3 is Cys, correspondingly, m1, m2 or m3 is 1;
when Xa1, Xa2 or Xa3 is Hcys, correspondingly, m1, m2 or m3 is 2;
n1, n2 and n3 are independently 1 or 2.

More specifically, as a first technical solution of the present invention, the present invention provides a peptide ligand specific for EphA2, comprising a polypeptide and a non-aromatic molecular scaffold, wherein the polypeptide comprises at least three residues independently selected from Cys and Hcys, and wherein at least one of the residues is a Hcys residue, the three Cys and Hcys residues are separated by two loop sequences, and the Cys or Hcys residue of the polypeptide is connected to the scaffold via a thioether bond, thereby forming two polypeptide loops on the molecular scaffold.

More specifically, as a second technical solution of the present invention, the peptide ligand specific for EphA2 comprises an amino acid sequence selected from:
Xa1-A1-Xa2-A2-Xa3

wherein A1 and A2 represent the amino acid residues between Xa1, Xa2 and Xa3, and A1 and A2 each independently comprise 5 or 6 amino acid residues;
Xa1, Xa2 and Xa3 are independently a Cys or Hcys residue, and at least one of Xa1, Xa2 and Xa3 is a Hcys residue, and the non-aromatic molecular scaffold and Xa1, Xa2 and Xa3 of the polypeptide form a thioether bond, thereby forming two polypeptide loops on the molecular scaffold.

More specifically, as a third technical solution of the present invention, the polypeptide of the first or second technical solution comprises the amino acid sequence as shown in SEQ ID NO:1:

Xa1-Hyp-Leu-Val-Asn-Pro-Leu-Xa2-Leu-His-Pro-(D-Asp)-Trp-(hArg)-Xa3 (SEQ ID NO:1).

More specifically, as a fourth technical solution of the present invention, the polypeptide comprises an amino acid sequence as shown in any one of SEQ ID NO:1-SEQ ID NO:7:

Hcys-Hyp-Leu-Val-Asn-Pro-Leu-Hcys-Leu-His-Pro-(D-Asp)-Trp-(hArg)-Hcys (SEQ ID NO:1);
Cys-Hyp-Leu-Val-Asn-Pro-Leu-Cys-Leu-His-Pro-(D-Asp)-Trp-(hArg)-Hcys (SEQ ID NO:2);
Cys-Hyp-Leu-Val-Asn-Pro-Leu-Hcys-Leu-His-Pro-(D-Asp)-Trp-(hArg)-Cys (SEQ ID NO:3);
Hcys-Hyp-Leu-Val-Asn-Pro-Leu-Cys-Leu-His-Pro-(D-Asp)-Trp-(hArg)-Cys (SEQ ID NO:4);
Cys-Hyp-Leu-Val-Asn-Pro-Leu-Hcys-Leu-His-Pro-(D-Asp)-Trp-(hArg)-Hcys (SEQ ID NO:5);
Hcys-Hyp-Leu-Val-Asn-Pro-Leu-Cys-Leu-His-Pro-(D-Asp)-Trp-(hArg)-Hcys (SEQ ID NO:6);
Hcys-Hyp-Leu-Val-Asn-Pro-Leu-Hcys-Leu-His-Pro-(D-Asp)-Trp-(hArg)-Cys (SEQ ID NO:7).

More specifically, as a fifth technical solution of the present invention, the polypeptide sequence of the peptide ligand is selected from one of SEQ ID NO:8-SEQ ID NO:14:

((β-Ala)-$Sar_{10}$-Ala-hArg-Asp-Hcys-Hyp-Leu-Val-Asn-Pro-Leu-Hcys-Leu-His-Pro-(D-Asp)-Trp-(hArg)-Hcys (SEQ ID NO:8);
(β-Ala)-$Sar_{10}$-Ala-hArg-Asp-Cys-Hyp-Leu-Val-Asn-Pro-Leu-Cys-Leu-His-Pro-(D-Asp)-Trp-(hArg)-Hcys (SEQ ID NO:9);
(β-Ala)-$Sar_{10}$-Ala-hArg-Asp-Cys-Hyp-Leu-Val-Asn-Pro-Leu-Hcys-Leu-His-Pro-(D-Asp)-Trp-(hArg)-Cys (SEQ ID NO:10);
(β-Ala)-$Sar_{10}$-Ala-hArg-Asp-Hcys-Hyp-Leu-Val-Asn-Pro-Leu-Cys-Leu-His-Pro-(D-Asp)-Trp-(hArg)-Cys (SEQ ID NO:11);
(β-Ala)-$Sar_{10}$-Ala-hArg-Asp-Cys-Hyp-Leu-Val-Asn-Pro-Leu-Hcys-Leu-His-Pro-(D-Asp)-Trp-(hArg)-Hcys (SEQ ID NO:12);
(β-Ala)-$Sar_{10}$-Ala-hArg-Asp-Hcys-Hyp-Leu-Val-Asn-Pro-Leu-Cys-Leu-His-Pro-(D-Asp)-Trp-(hArg)-Hcys (SEQ ID NO:13);
(β-Ala)-$Sar_{10}$-Ala-hArg-Asp-Hcys-Hyp-Leu-Val-Asn-Pro-Leu-Hcys-Leu-His-Pro-(D-Asp)-Trp-(hArg)-Cys (SEQ ID NO:14).

More specifically, as a sixth technical solution of the present invention, the peptide ligand is a bicyclic peptide containing one of the following structures:

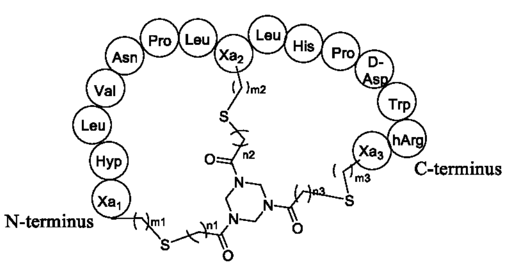

I-1

,

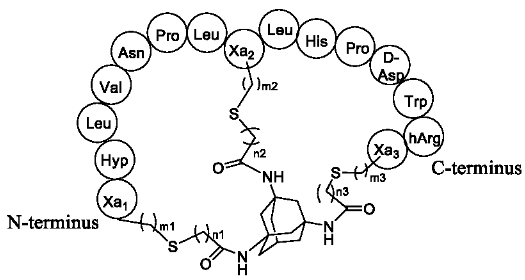

II-1

wherein, when Xa1, Xa2 or Xa3 is Cys, correspondingly, m1, m2 or m3 is 1;
when Xa1, Xa2 or Xa3 is Hcys, correspondingly, m1, m2 or m3 is 2;
n1, n2 and n3 are independently 1 or 2.

More specifically, as a seventh technical solution of the present invention, the peptide ligand is a bicyclic peptide having one of the following structures:

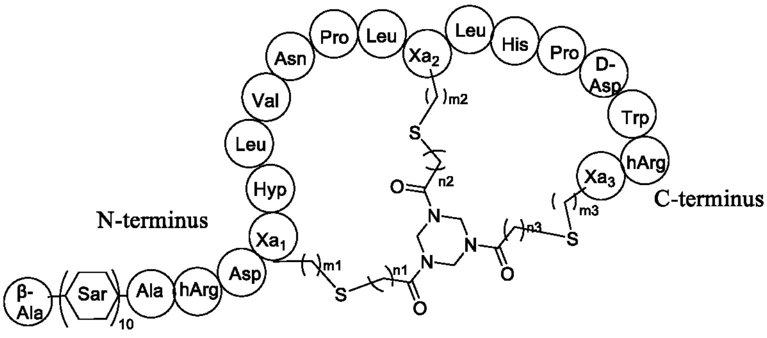

,

I-2

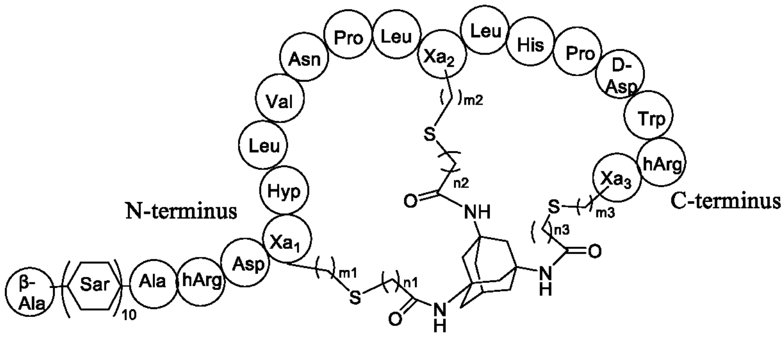

II-2.

More specifically, as an eighth technical solution of the present invention, provided is a peptide ligand of formula 1-2 or II-2, wherein

(1) Xa1 is Hcys, m1 is 2, and n1 is 1 or 2; or
(2) Xa2 is Hcys, m2 is 2, and n2 is 1 or 2; or
(3) Xa3 is Hcys, m3 is 2, and n3 is 1 or 2.

More specifically, as a ninth technical solution of the present invention, provided is a drug conjugate or a pharmaceutically acceptable salt thereof, wherein the conjugate comprises the peptide ligand according to any one of the first to eighth technical solutions conjugated to one or more effectors and/or functional groups via a linker.

More specifically, as a tenth technical solution of the present invention, provided is the drug conjugate or the pharmaceutically acceptable salt thereof according to the seventh technical solution, wherein the effector is a cytotoxic agent.

More specifically, as an eleventh technical solution of the present invention, provided is the drug conjugate or the pharmaceutically acceptable salt thereof according to the tenth technical solution, wherein the cytotoxic agent is selected from the following group: alkylating agents, antimetabolites, plant alkaloids, terpenoids, podophyllotoxins and a derivative thereof, taxanes and a derivative thereof, a topoisomerase inhibitor, and antitumour antibiotics.

More specifically, as a twelfth technical solution of the present invention, provided is the drug conjugate or the pharmaceutically acceptable salt thereof according to the tenth technical solution, wherein the cytotoxic agent is selected from the following group: cisplatin, carboplatin, oxaliplatin, mechlorethamine, cyclophosphamide, chlorambucil, ifosfamide, azathioprine, mercaptopurine, a pyrimidine analogue, vincristine, vinblastine, vinorelbine, vindesine, etoposide, teniposide, taxol, camptothecine, irinotecan, topotecan, amsacrine, etoposide, etoposide phosphate, teniposide, actinomycin D, doxorubicin, epirubicin, epothilone and a derivative thereof, bleomycin and a derivative thereof, dactinomycin and a derivative thereof, plicamycin and a derivative thereof, mitomycin C, calicheamycin, maytansine and a derivative thereof, and auristatin and a derivative thereof.

More specifically, as a thirteen technical solution of the present invention, provided is the drug conjugate or the pharmaceutically acceptable salt thereof according to the tenth technical solution, wherein the cytotoxic agent is selected

from DM1, MMAE or SN38.

More specifically, as a fourteenth technical solution of the present invention, provided is the drug conjugate or the pharmaceutically acceptable salt thereof according to the ninth technical solution, wherein the linker is a peptide linker, a disulphide linker, an enzyme-dependent linker, or a pH-dependent linker; in certain embodiments, the linker is a peptide linker, a disulphide linker, or a pH-dependent linker.

More specifically, as a fifteenth technical solution of the present invention, provided is the drug conjugate or the pharmaceutically acceptable salt thereof according to the fourteenth technical solution, wherein the disulphide linker is selected from DMDS, MDS, DSDM, NDMDS or a structure of formula III:

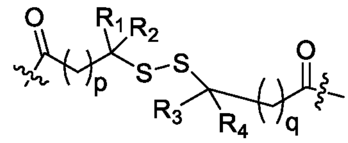

III

wherein $R_1$, $R_2$, $R_3$ and $R_4$ are independently selected from H, methyl, ethyl, propyl and isopropyl;
p and q are independently 1, 2, 3, 4 or 5;
the peptide linker is selected from: -Cit-Val-, -Phe-Lys- and -Val-Lys-;
the enzyme-dependent linker is selected from:

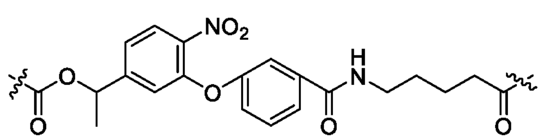

;

the pH-dependent linker is selected from a cis-aconitic anhydride.

More specifically, as a sixteenth technical solution of the present invention, provided is the drug conjugate or the pharmaceutically acceptable salt thereof according to the fourteenth technical solution, wherein the linker is -PABC-Cit-Val-glutaryl-, -PABC-cyclobutyl-Ala-Cit-βAla-, or (-PABC-Cit-Val-CO-$CH_2)_2$N-$CH_2(CH_2OCH_2)_2CH_2$NH-glutaryl-, wherein PABC represents p-aminobenzylcarbamate; or the linker is

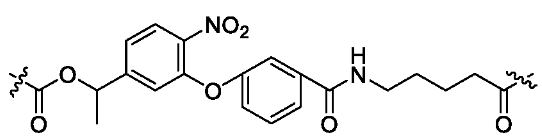

.

More specifically, as a seventeenth technical solution of the present invention, provided is the drug conjugate or the pharmaceutically acceptable salt thereof according to the ninth technical solution, wherein the drug conjugate has a structure of formula I, formula II, formula III, formula IV, formula V, or formula VI:

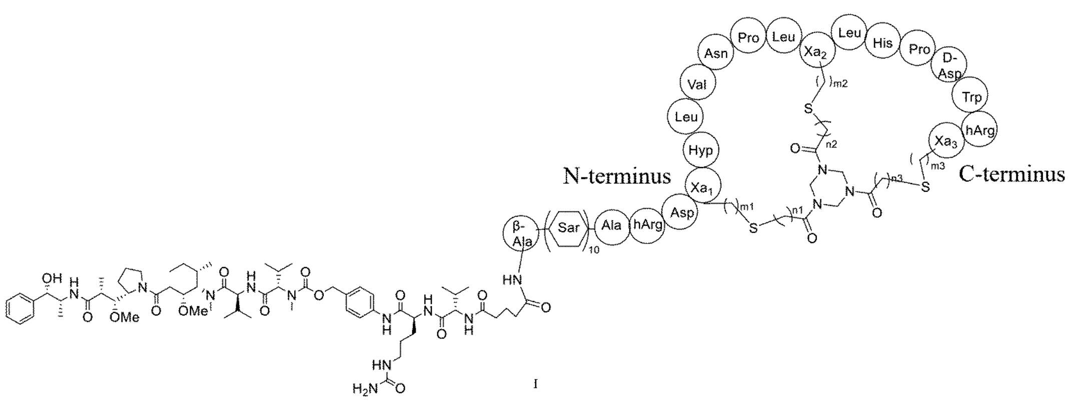

I

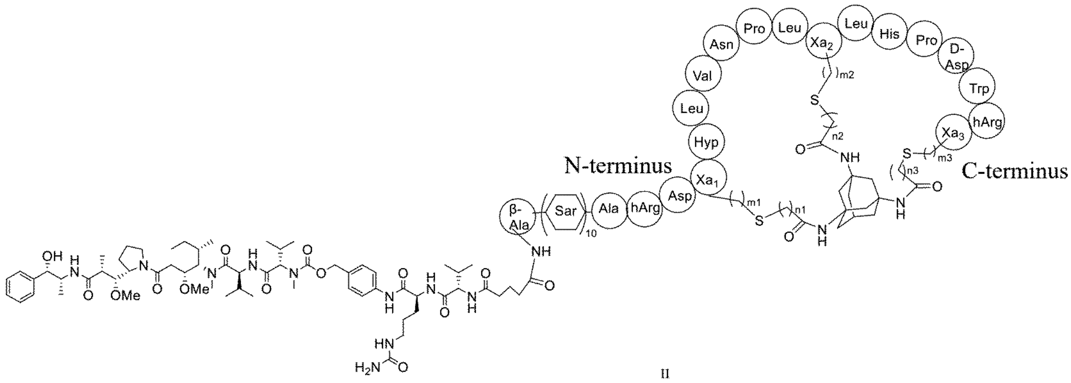

II

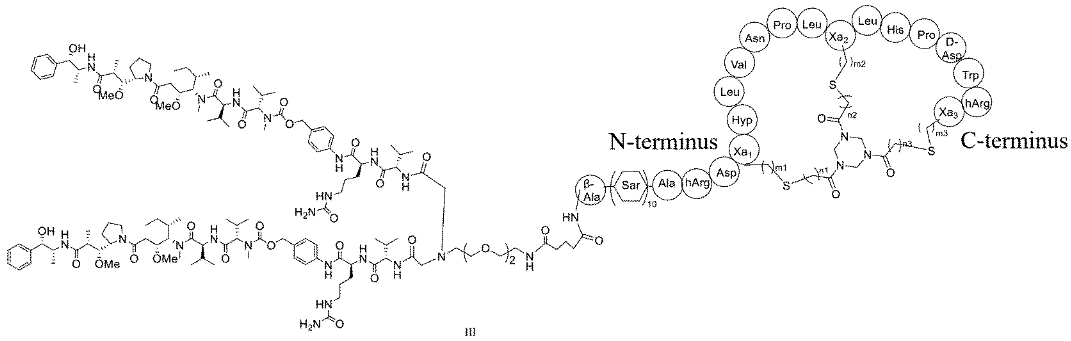

III

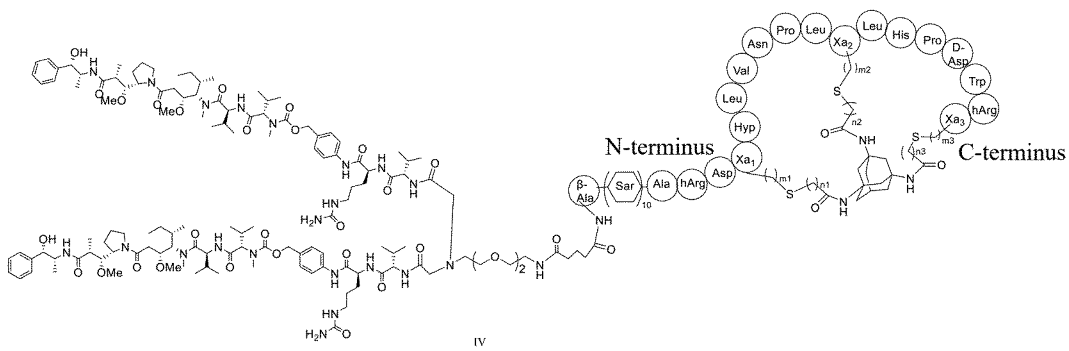

IV

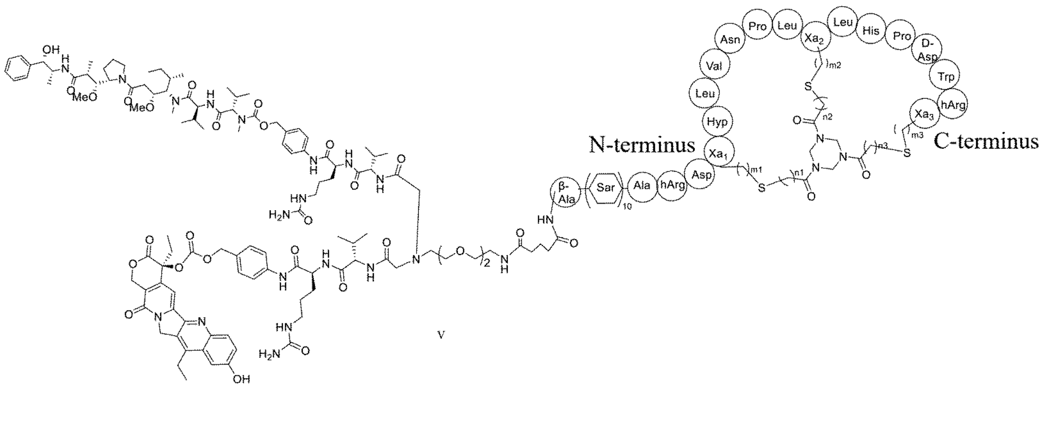

V

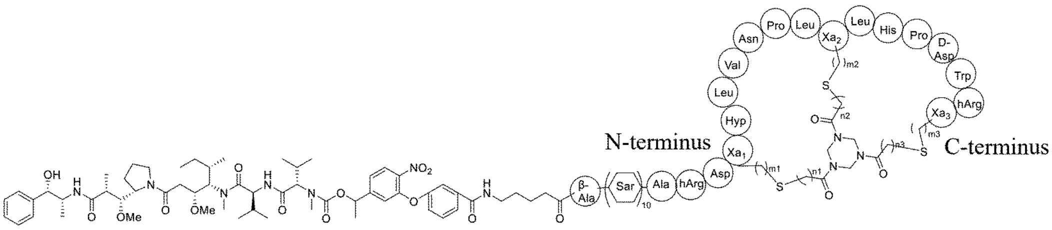

VI

wherein Xa1, Xa2 and Xa3 are independently a Cys or Hcys residue, and at least one of Xa1, Xa2 and Xa3 is a Hcys residue;
when Xa1, Xa2 or Xa3 is Cys, correspondingly, m1, m2 or m3 is 1;
when Xa1, Xa2 or Xa3 is Hcys, correspondingly, m1, m2 or m3 is 2;
n1, n2 and n3 are independently 1 or 2.

More specifically, as an eighteenth technical solution of the present invention, provided is a drug conjugate or a pharmaceutically acceptable salt thereof, wherein the drug conjugate is selected from one of the following structures:

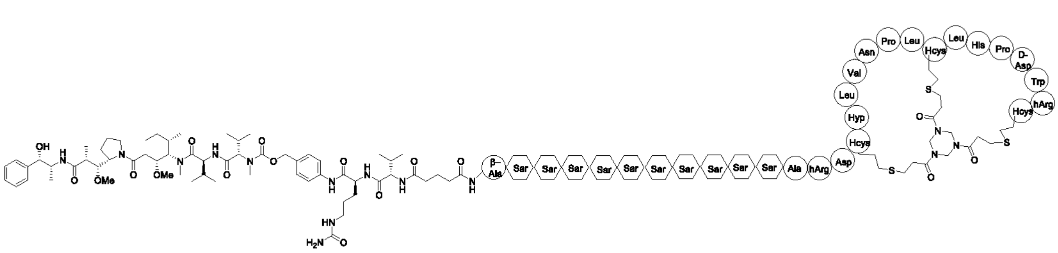

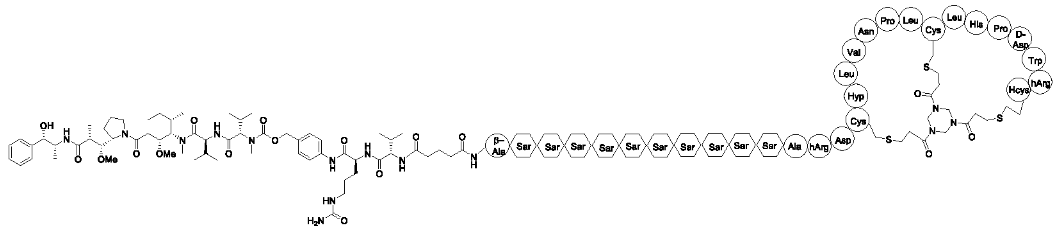

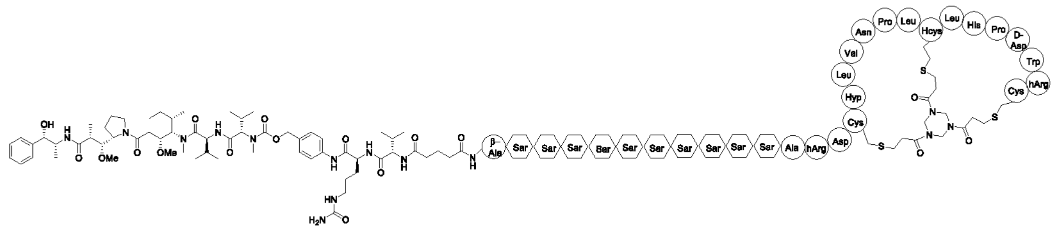

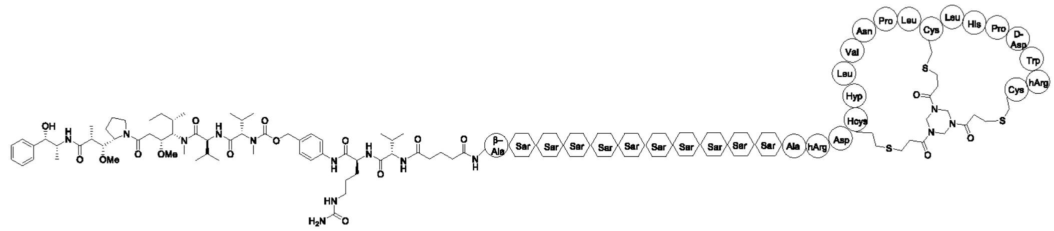

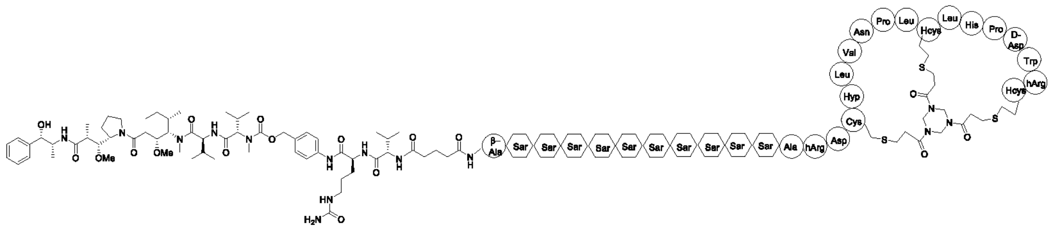

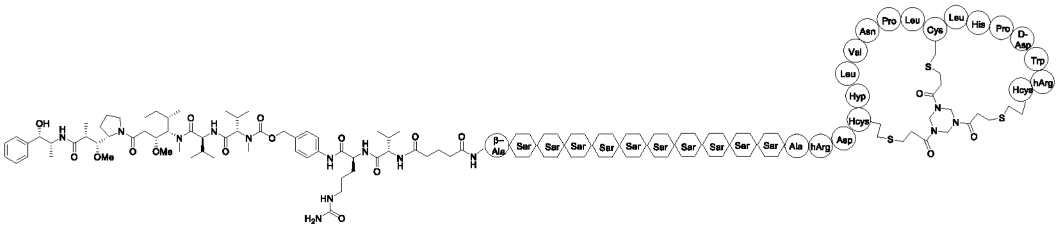

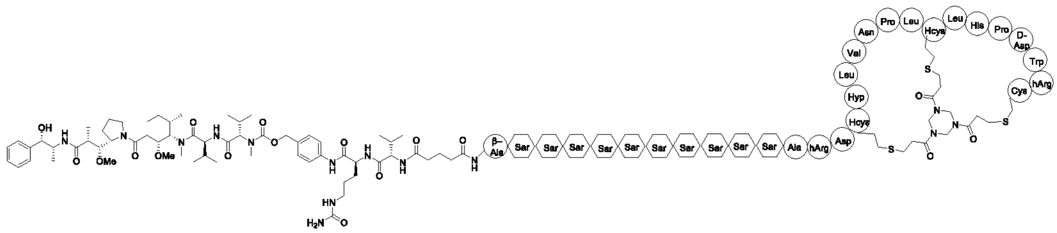

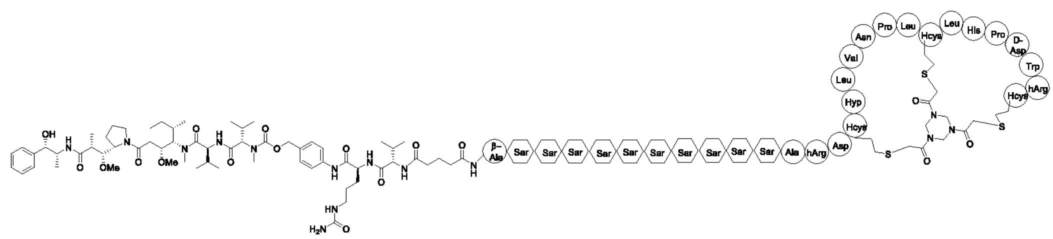

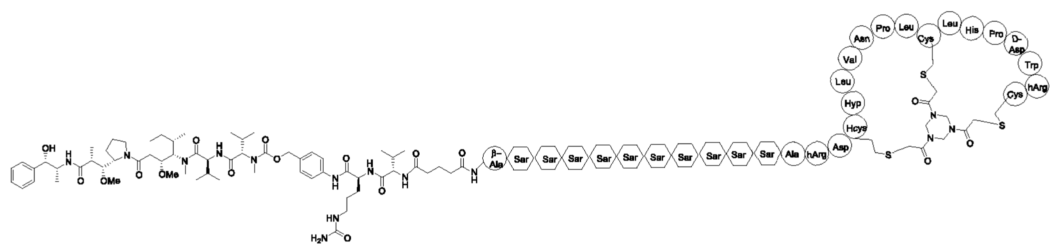

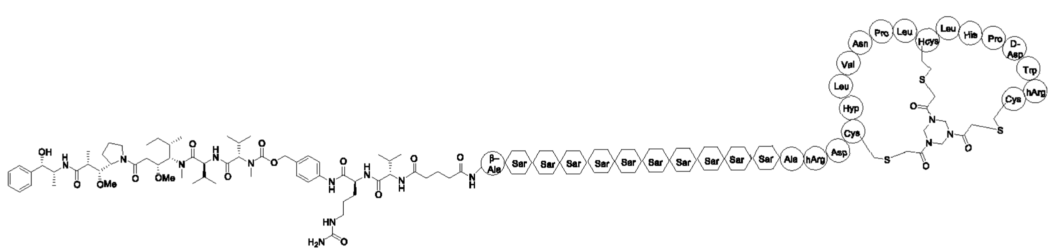

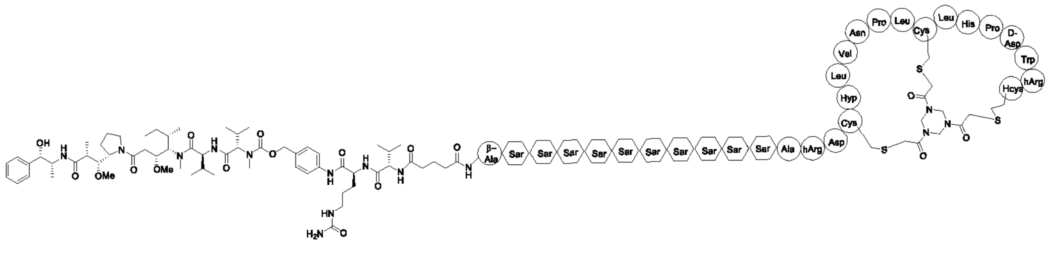

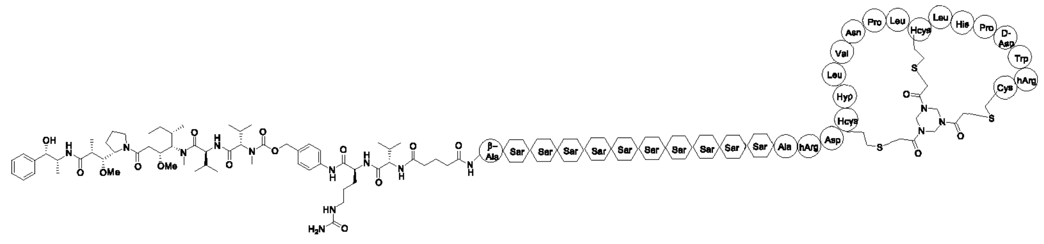

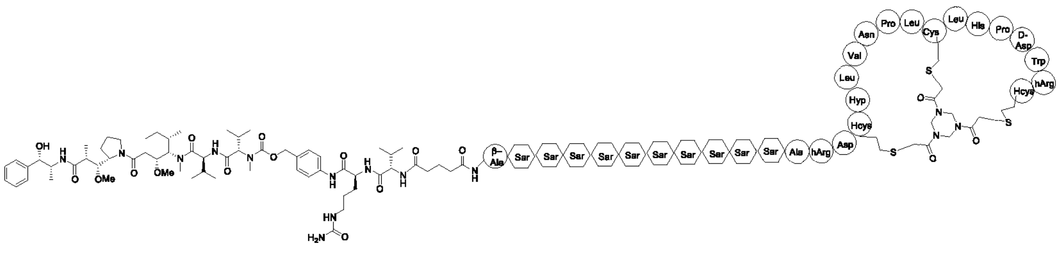

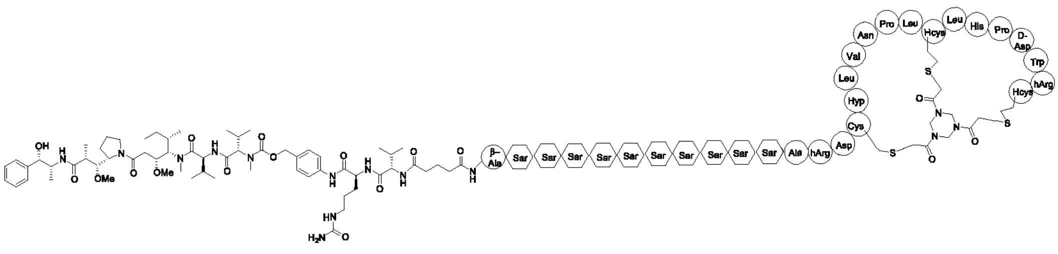

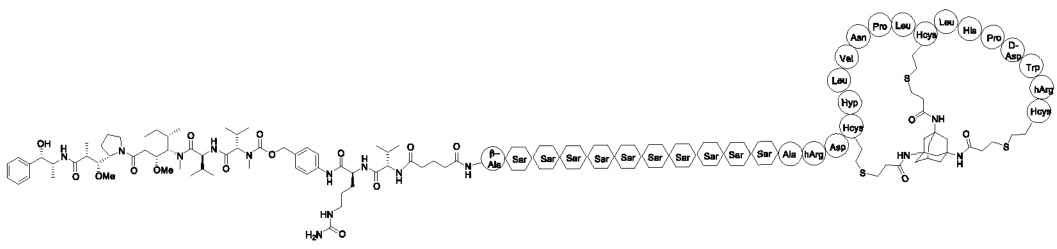

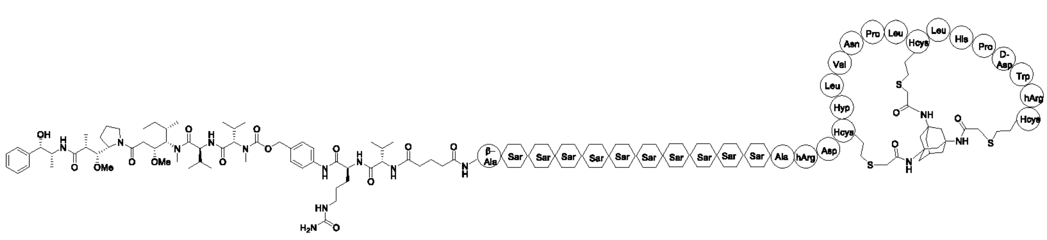

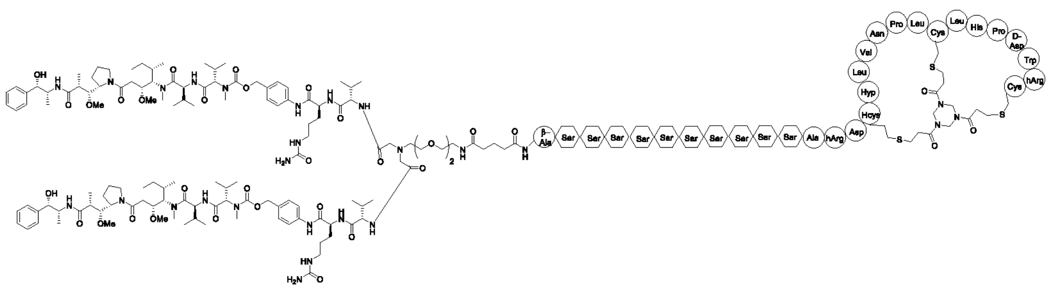

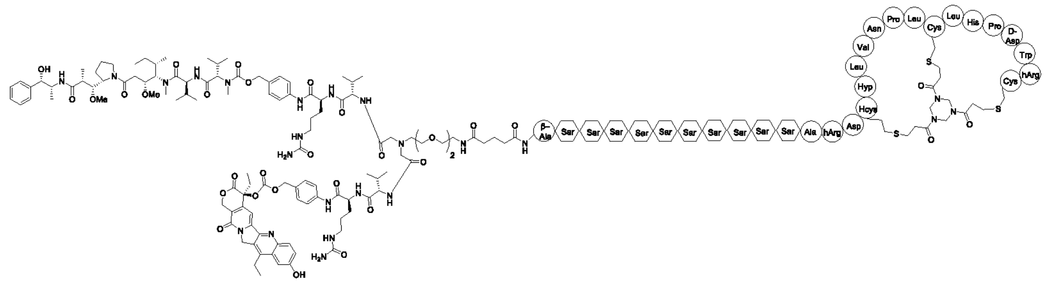

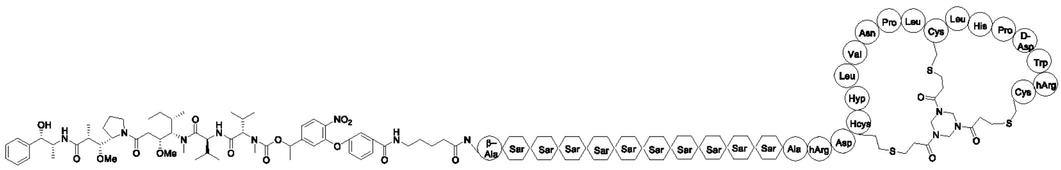

.

The present invention also relates to a pharmaceutical composition, comprising the drug conjugate or the pharmaceutically acceptable salt thereof according to any one of the preceding ninth to eighteenth technical solutions, and a pharmaceutically acceptable carrier and/or excipient.

Further, the pharmaceutical composition or pharmaceutical preparation comprises 1-1500 mg of the drug conjugate or the pharmaceutically acceptable salt thereof according to any one of the preceding technical solutions, and a pharmaceutically acceptable carrier and/or excipient.

The present invention also relates to the use of the drug conjugate or the pharmaceutically acceptable salt thereof according to any one of the preceding ninth to eighteenth technical solutions or a composition thereof in the preparation for preventing and/or treating EphA2 overexpressed disease.

Generally, the EphA2 overexpressed disease is cancer. Examples of cancers (and their benign counterparts) that can be treated (inhibited) include, but are not limited to tumours of epithelial origin (adenomas and carcinomas of various types including adenocarcinomas, squamous carcinomas, transitional cell carcinomas and other carcinomas) such as carcinomas of the bladder and urinary tract, breast, gastrointestinal tract (including the oesophagus, stomach (gastric), small intestine, colon, rectum and anus), liver (hepatocellular carcinoma), gall bladder and biliary system, exocrine pancreas, kidney, lung (for example adenocarcinomas, small cell lung carcinomas, non-small cell lung carcinomas, bronchioalveolar carcinomas and mesotheliomas), head and neck (for example cancers of the tongue, buccal cavity, larynx, pharynx, nasopharynx, tonsil, salivary glands, nasal cavity and paranasal sinuses), ovary, fallopian tubes, peritoneum, vagina, vulva, penis, cervix, myometrium, endometrium, thyroid (for example thyroid follicular carcinoma), kidney, prostate, skin and adnexa (for example melanoma, basal cell carcinoma, squamous cell carcinoma, keratoacanthoma, dysplastic naevus); haematological malignancies (i.e. leukaemia, lymphomas) and premalignant haematological disorders and disorders of borderline malignancy including haematological malignancies and related conditions of lymphoid lineage (for example acute lymphocytic leukaemia [ALL], chronic lymphocytic leukaemia [CLL], B-cell lymphomas such as diffuse large B-cell lymphoma [DLBCL], follicular lymphoma, Burkitt's lymphoma, mantle cell lymphoma, T-cell lymphomas and leukaemia, natural killer [NK] cell lymphomas, Hodgkin's lymphomas, hairy cell leukaemia, monoclonal gammopathy of uncertain significance, plasmacytoma, multiple myeloma, and post-transplant lymphoproliferative disorders), and haematological malignancies and related conditions of myeloid lineage (for example acute myelogenous leukaemia [AML], chronic myelogenous leukaemia [CML], chronic myelomonocytic leukaemia [CMML], hypereosinophilic syndrome, myeloproliferative disorders such as polycythaemia vera, essential thrombocythemia and primary myelofibrosis, myeloproliferative syndrome, myelodysplastic syndrome, and promyelocytic leukaemia); tumours of mesenchymal origin, for example sarcomas of soft tissue, bone or cartilage such as osteosarcomas, fibrosarcomas, chondrosarcomas, rhabdomyosarcomas, leiomyosarcomas, liposarcomas, angiosarcomas, Kaposi's sarcoma, Ewing's sarcoma, synovial sarcomas, epithelioid sarcomas, gastrointestinal stromal tumours, benign and malignant tissue sarcomas, and dermatofibrosarcoma protuberans; tumours of the central or peripheral nervous system (for example astrocytomas, gliomas and glioblastomas, meningiomas, ependymomas, pinealomas and schwannomas); endocrine tumours (for example pituitary tumours, adrenal tumours, islet cell tumours, parathyroid tumours, carcinoid tumours and medullary thyroid carcinoma); ocular and adnexal tumours (for example retinoblastoma); germ cell and trophoblastic tumours (for example teratomas, seminomas, dysgerminomas, hydatidiform moles and choriocarcinomas); paediatric and embryonal tumours (for example medulloblastoma, neuroblastoma, Wilms tumour, and primitive neuroectodermal tumours); or syndromes, congenital or otherwise, which leave the patient susceptible to malignancy (for example Xeroderma Pigmentosum).

The EphA2 overexpressed disease is further selected from: prostate cancer, lung cancer, breast cancer, gastric cancer, ovarian cancer, oesophageal cancer, multiple myeloma and fibrosarcoma.

The present invention also provides a method for treating a tumour, comprising administering to a subject a therapeutically effective amount of the drug conjugate or the pharmaceutically acceptable salt thereof according to any one of the preceding technical solutions, and a pharmaceutically acceptable carrier and/ or excipient, wherein the subject comprises a mammal or human, the therapeutically effective amount is preferably 1-1500 mg; and the tumour is preferably prostate cancer, lung cancer, breast cancer, gastric cancer, ovarian cancer, oesophageal cancer, multiple myeloma and fibrosarcoma.

The present invention also provides a method for treating a tumour, comprising administering to the mammal or human a therapeutically effective amount of the drug conjugate or the pharmaceutically acceptable salt thereof or a co-crystal or the pharmaceutical composition according to the present invention.

The "effective amount" or "therapeutically effective amount" as described in the present application refers to administration of a sufficient amount of the compound disclosed in the present application that will alleviate to some extent one or more symptoms of the diseases or conditions being treated. In some embodiments, the outcome is the reduction and/or remission of signs, symptoms or causes of the disease, or any other desired change in the biological system. For example, an "effective amount" in terms of the therapeutic use is an amount of the composition comprising the compound disclosed in the present application that is required to provide clinically significant reduction of the symptoms of the disease. Examples of the therapeutically effective amount include, but are not limited to 1-1500 mg, 1-1400 mg, 1-1300 mg, 1-1200 mg, 1-1000 mg, 1-900 mg, 1-800 mg, 1-700 mg, 1-600 mg, 1-500 mg, 1-400 mg, 1-300 mg, 1-250 mg, 1-200 mg, 1-150 mg, 1-125 mg, 1-100 mg, 1-80 mg, 1-60 mg, 1-50 mg, 1-40 mg, 1-25 mg, 1-20 mg, 5-1500 mg, 5-1000 mg, 5-900 mg, 5-800 mg, 5-700 mg, 5-600 mg, 5-500 mg, 5-400 mg, 5-300 mg, 5-250 mg, 5-200 mg, 5-150 mg, 5-125 mg, 5-100 mg, 5-90 mg, 5-70 mg, 5-80 mg, 5-60 mg, 5-50 mg, 5-40 mg, 5-30 mg, 5-25 mg, 5-20 mg, 10-1500 mg, 10-1000 mg, 10-900 mg, 10-800 mg, 10-700 mg, 10-600 mg, 10-500 mg, 10-450 mg, 10-400 mg, 10-300 mg, 10-250 mg, 10-200 mg, 10-150 mg, 10-125 mg, 10-100 mg, 10-90 mg, 10-80 mg, 10-70 mg, 10-60 mg, 10-50 mg, 10-40 mg, 10-30 mg, 10-20 mg; 20-1500 mg, 20-1000 mg, 20-900 mg, 20-800 mg, 20-700 mg, 20-600 mg, 20-500 mg, 20-400 mg, 20-350 mg, 20-300 mg, 20-250 mg, 20-200 mg, 20-150 mg, 20-125 mg, 20-100 mg, 20-90 mg, 20-80 mg, 20-70 mg, 20-60 mg, 20-50 mg, 20-40 mg, 20-30 mg; 50-1500 mg, 50-1000 mg, 50-900 mg, 50-800 mg, 50-700 mg, 50-600 mg, 50-500 mg, 50-400 mg, 50-300 mg, 50-250 mg, 50-200 mg, 50-150 mg, 50-125 mg, 50-100 mg; 100-1500 mg, 100-1000 mg, 100-900 mg, 100-800 mg, 100-700 mg, 100-600 mg, 100-500 mg, 100-400 mg, 100-300 mg, 100-250 mg, or 100-200 mg.

The present invention relates to a pharmaceutical composition or pharmaceutical preparation, comprising a therapeutically effective amount of the drug conjugate or the pharmaceutically acceptable salt or co-crystal thereof according to the present invention, and a carrier and/or excipient. The pharmaceutical composition can be in a unit preparation form (the amount of the drug substance in the unit preparation is also referred to as the "preparation strength"). In some embodiments, the pharmaceutical composition comprises the compound, or the stereoisomer, deuterated compound, solvate, pharmaceutically acceptable salt, or co-crystal thereof according to the present invention in an amount including but not limited to 1-1500 mg, 5-1000 mg, 10-800 mg, 20-600 mg, 25-500 mg, 40-200 mg, 50-100 mg, 1 mg, 1.25 mg, 2.5 mg, 5 mg, 10 mg, 12.5 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, 275 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 850 mg, 900 mg, 950 mg, 1000 mg, 1100 mg, 1200 mg, 1300 mg, 1400 mg, and 1500 mg.

The present invention also relates to a method for treating a disease in a mammal or human, comprising administering to a subject a therapeutically effective amount of the drug conjugate or the pharmaceutically acceptable salt or co-crystal thereof according to the present invention, and a pharmaceutically acceptable carrier and/or excipient, wherein the therapeutically effective amount is preferably 1-1500 mg; and the disease is preferably prostate cancer,

lung cancer, breast cancer, gastric cancer, ovarian cancer, oesophageal cancer, multiple myeloma and fibrosarcoma.

The present invention also relates to a method for treating a disease in a mammal or human, comprising administering to a subject a drug, i.e., the conjugate or the pharmaceutically acceptable salt or co-crystal thereof according to the present invention, and a pharmaceutically acceptable carrier and/excipient in a daily dose of 1-1500 mg/day, wherein the daily dose can be a single dose or divided doses; in some embodiments, the daily dose includes, but is not limited to 10-1500 mg/day, 20-1500 mg/day, 25-1500 mg/day, 50-1500 mg/day, 75-1500 mg/day, 100-1500 mg/day, 200-1500 mg/day, 10-1000 mg/day, 20-1000 mg/day, 25-1000 mg/day, 50-1000 mg/day, 75-1000 mg/day, 100-1000 mg/day, 200-1000 mg/day, 25-800 mg/day, 50-800 mg/day, 100-800 mg/day, 200-800 mg/day, 25-400 mg/day, 50-400 mg/day, 100-400 mg/day, or 200-400 mg/day; in some embodiments, the daily dose includes, but is not limited to 1 mg/day, 5 mg/day, 10 mg/day, 20 mg/day, 25 mg/day, 50 mg/day, 75 mg/day, 100 mg/day, 125 mg/day, 150 mg/day, 200 mg/day, 400 mg/day, 600 mg/day, 800 mg/day, 1000 mg/day, 1200 mg/day, 1400 mg/day, or 1500 mg/day.

The present invention relates to a kit, wherein the kit can comprise a composition in the form of a single dose or multiple doses and comprises the conjugate or the pharmaceutically acceptable salt or co-crystal thereof according to the present invention, and the amount of the conjugate or the pharmaceutically acceptable salt or co-crystal thereof according to the present invention is identical to the amount of same in the above-mentioned pharmaceutical composition.

In the present invention, the amount of the conjugate or the pharmaceutically acceptable salt or co-crystal thereof according to the present invention is calculated in the form of a free base in each case.

The term "preparation strength" refers to the weight of the drug substance contained in each vial, tablet or other unit preparation.

**Synthesis**

Those skilled in the art would have been able to prepare the compounds of the present invention according to known organic synthesis techniques, and the starting materials used therein are commercially available chemicals and (or) compounds described in chemical documents. "Commercially available chemicals" are obtained from regular commercial sources, and suppliers include: Titan Technology Co., Ltd., Energy Chemical Co., Ltd., Shanghai Demo Co., Ltd., Chengdu Kelong Chemical Co., Ltd., Accela ChemBio Co., Ltd., PharmaBlock Sciences (Nanjing), Inc., WuXi Apptec Co., Ltd., J&K Scientific Co., Ltd., etc.

The peptides of the present invention can be prepared synthetically by standard techniques, followed by reaction with a molecular scaffold in vitro. When this is performed, standard chemistry may be used. This enables the rapid large-scale preparation of soluble material for further downstream experiments or validation. Such methods could be accomplished using conventional chemistry such as that disclosed in Timmerman et al. (2005, ChemBioChem).

In some embodiments, the drug conjugate of the present invention can be synthesized according to the following route:

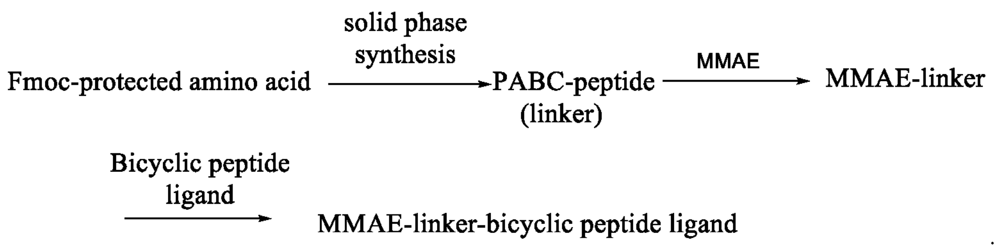


More specific synthesis steps are described in the Examples.

**Term**

The term "peptide ligand" refers to a peptide covalently bound to a molecular scaffold. Generally, such peptides comprise two or more reactive groups (i.e., cysteine residues or homocysteine residues) which are capable of forming covalent bonds (such as a thioether bond) with the scaffold, and a sequence subtended between the reactive groups which is referred to as the loop sequence, since it forms a loop when the peptide is bound to the scaffold. In the present case, the peptides comprise at least three cysteine residues or homocysteine residues (Cys residues or Hcys residues), and form at least two loops on the scaffold.

The molecular scaffold comprises a non-aromatic molecular scaffold. The term "non-aromatic molecular scaffold" refers to any molecular scaffold as defined herein which does not contain an aromatic carbocyclic or heterocyclic ring system. Suitable examples of non-aromatic molecular scaffolds are described in Heinis et al. (2014) Angewandte Chemie, International Edition 53(6) 1602-1606. The molecular scaffold may be a small molecule, such as a small organic

molecule. The molecular scaffold may be a macromolecule. In some cases, the molecular scaffold is a macromolecule composed of amino acids, nucleotides or carbohydrates. In some cases, the molecular scaffold comprises reactive groups that are capable of reacting with functional group(s) of the polypeptide to form covalent bonds. The molecular scaffold may comprise chemical groups which form the linkage with a peptide, such as amines, thiols, alcohols, ketones, aldehydes, nitriles, carboxylic acids, esters, alkenes, alkynes, azides, anhydrides, succinimides, maleimides, alkyl halides and acyl halides. An example of an αβ unsaturated carbonyl containing compound is 1,1',1"-(1,3,5-triazinane-1,3,5-triyl)triprop-2-en-1-one (TATA) (Angewandte Chemie, International Edition (2014), 53(6), 1602-1606).

The term "polypeptide" refers to a compound formed by three or more amino acid molecules linked together via peptide bonds. The amino acid units in a polypeptide are referred to as amino acid residues.

Effectors and/or functional groups are molecules or fragments having pharmacological effects or specific functions that can be connected (via linkers), for example, to the N and/or C termini of the polypeptide, to an amino acid within the polypeptide, or to the molecular scaffold. Suitable effectors and/or functional groups comprise an antibody and a moiety or fragment thereof, a cytotoxic molecule or a fragment thereof, an enzyme inhibitor molecule or a fragment thereof, a metal chelator, etc. In some cases, the effector and/or functional group is a drug. The effector and/or functional group is particularly a cytotoxic agent, including alkylating agents, antimetabolites, plant alkaloids, terpenoids, podophyllotoxins and a derivative thereof, taxanes and a derivative thereof, a topoisomerase inhibitor, antitumour antibiotics, etc. The effector and/or functional group is more particularly cisplatin, carboplatin, oxaliplatin, mechlorethamine, cyclophosphamide, chlorambucil, ifosfamide, azathioprine, mercaptopurine, a pyrimidine analogue, vincristine, vinblastine, vinorelbine, vindesine, etoposide, teniposide, taxol, camptothecine, irinotecan, topotecan, amsacrine, etoposide, etoposide phosphate, teniposide, actinomycin D, doxorubicin, epirubicin, epothilone and a derivative thereof, bleomycin and a derivative thereof, dactinomycin and a derivative thereof, plicamycin and a derivative thereof, mitomycin C, calicheamycin, maytansine and a derivative thereof, and auristatin and a derivative thereof.

The term "derivative" refers to a product derived from the substitution of hydrogen atoms or atomic groups in a compound by other atoms or atomic groups.

Unless otherwise specified, all the amino acids are used in the L-configuration.

β-Ala: Beta-alanine
Sar: Sarcosine
Cys: Cysteine
Hcys: Homocysteine
Hyp: Hydroxyproline
Asn: Asparagine
Pro: Proline
Leu: Leucine
His: Histidine
D-Asp: D-aspartic acid
Trp: Tryptophan
hArg: Homoarginine
Ser: Serine
Thr: Threonine
Phe: Phenylalanine
Tyr: Tyrosine
Val: Valine
Ile: Isoleucine
Lys: Lysine
Gly: Glycine
Cit: Citrulline
DM1:

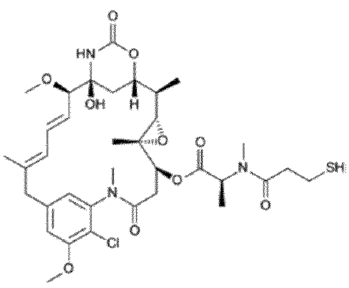

MMAE:

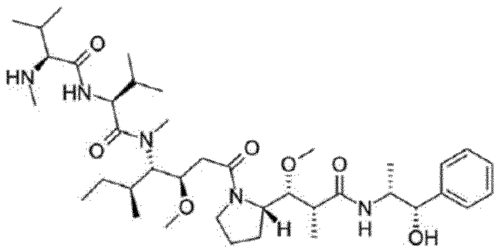

SN3 8: 7-ethyl-10-hydroxycamptothecine
DMDS:

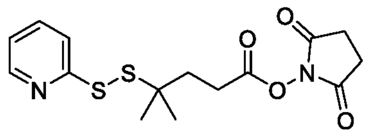

MDS:

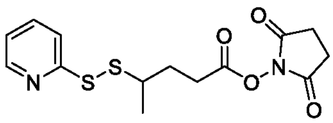

DSDM:

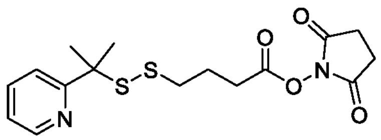

NDMDS:

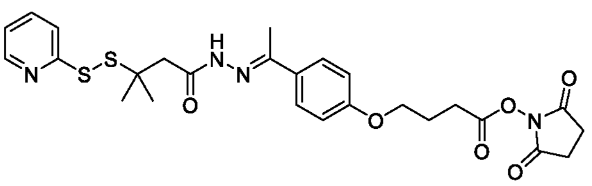

TATB:

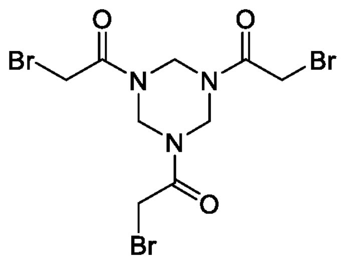

TATA:

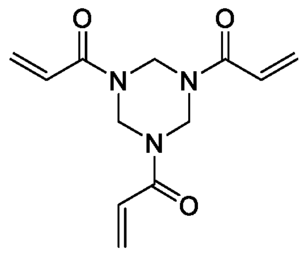

The carbon, hydrogen, oxygen, sulphur, nitrogen and halogen involved in the groups and compounds of the present invention all comprise isotopes thereof, and are optionally further substituted with one or more of the corresponding isotopes thereof, wherein the isotopes of carbon comprise $^{12}C$, $^{13}C$ and $^{14}C$; the isotopes of hydrogen comprise protium (H), deuterium (deuterium, also known as heavy hydrogen), and tritium (T, also known as superheavy hydrogen); the isotopes of oxygen comprise $^{16}O$, $^{17}O$ and $^{18}O$; the isotopes of sulphur comprise $^{32}S$, $^{33}S$, $^{34}S$ and $^{36}S$; the isotopes of nitrogen comprise $^{14}N$ and $^{15}N$; the isotope of fluorine comprises $^{19}F$; the isotopes of chlorine comprise $^{35}Cl$ and $^{37}Cl$; and the isotopes of bromine comprise $^{79}Br$ and $^{81}Br$.

The term "pharmaceutically acceptable salt" refers to a salt of the compound of the present invention, which salt maintains the biological effectiveness and characteristics of a free acid or a free base and is obtained by reacting the free acid with a non-toxic inorganic base or organic base, or reacting the free base with a non-toxic inorganic acid or organic acid.

The term "pharmaceutical composition" represents a mixture of one or more compounds described herein and the stereoisomers, solvates, pharmaceutically acceptable salts or co-crystals thereof and other components comprising physiologically/pharmaceutically acceptable carriers and/or excipients.

The term "carrier" refers to: a system that does not cause significant irritation to the organism and does not eliminate the biological activity and characteristics of the administered compound, and can change the way the drug enters the human body and the distribution of the drug in the body, control the release rate of the drug and delivery the drug to targeted organs. Non-limiting examples of the carrier include microcapsule, microsphere, nanoparticle, liposome, etc.

The term "excipient" refers to: a substance that is not a therapeutic agent per se, but used as a diluent, adjuvant, adhesive and/or vehicle for addition to a pharmaceutical composition, thereby improving the disposal or storage properties thereof, or allowing to or promoting the formation of a compound or a pharmaceutical composition into a unit dosage form for administration. As is known to those skilled in the art, a pharmaceutically acceptable excipient can provide various functions and can be described as a wetting agent, a buffer, a suspending agent, a lubricant, an emulsifier, a disintegrating agent, an absorbent, a preservative, a surfactant, a colorant, a flavouring agent and a sweetening agent. Examples of pharmaceutically acceptable excipients include, but are not limited to: (1) sugars, such as lactose, glucose and sucrose; (2) starch, such as corn starch and potato starch; (3) cellulose and its derivatives, such as sodium carboxymethyl cellulose, ethyl cellulose, cellulose acetate, hydroxypropyl methylcellulose, hydroxypropyl cellulose, microcrystalline cellulose and croscarmellose (such as croscarmellose sodium); (4) tragacanth powder; (5) malt; (6) gelatine; (7) talc; (8) excipients, such as cocoa butter or suppository wax; (9) oils, such as peanut oil, cottonseed oil, safflower oil, sesame oil, olive oil, corn oil and soybean oil; (10) diols, such as propylene glycol; (11) polyols, such as glycerine, sorbitol, mannitol and polyethylene glycol; (12) esters, such as ethyl oleate and ethyl laurate; (13) agar; (14) buffers, such as magnesium hydroxide and aluminium hydroxide; (15) alginic acid; (16) pyrogen-free water; (17) isotonic saline; (18) Ringer's solution; (19) ethanol; (20) pH buffered solution; (21) polyester, polycarbonate and/or polyanhydride; and (22) other non-toxic compatible substances used in a pharmaceutical preparation.

Brief Description of the Drawings

Figure 1 shows the tumour growth curve of the mouse PC3 subcutaneous in vivo transplanted tumour model.
Figure 2 shows the animal body weight change curve of the mouse PC3 subcutaneous in vivo transplanted tumour model.

Detailed Description of Embodiments

The content of the present invention is described in detail with the following examples. If a specific condition is not indicated in the examples, a conventional condition is used in an experimental method. The listed examples are intended to better illustrate the content of the present invention, but should not be construed as limiting the content of the present invention. According to the above-mentioned content of the invention, those skilled in the art can make unsubstantial modifications and adjustments to the embodiments, which still fall within the protection scope of the present invention.

**Preparation of compounds**

Synthesis of bicyclic peptide HSK-P14

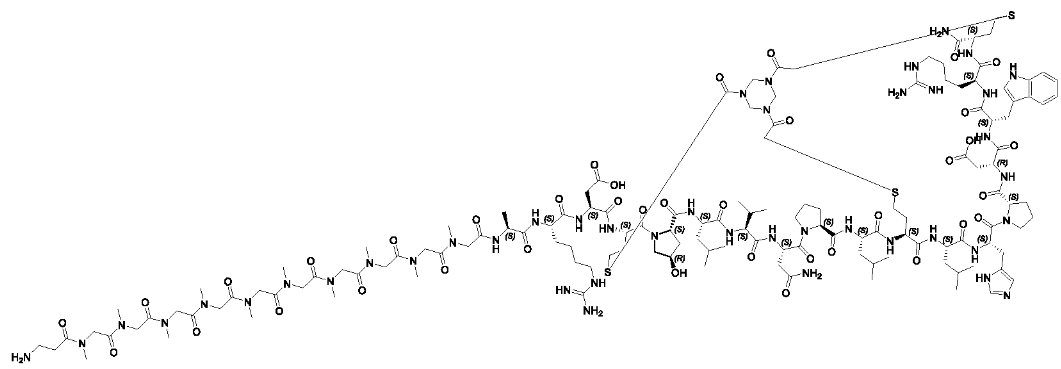

HSK-P14

Synthetic method for P14:

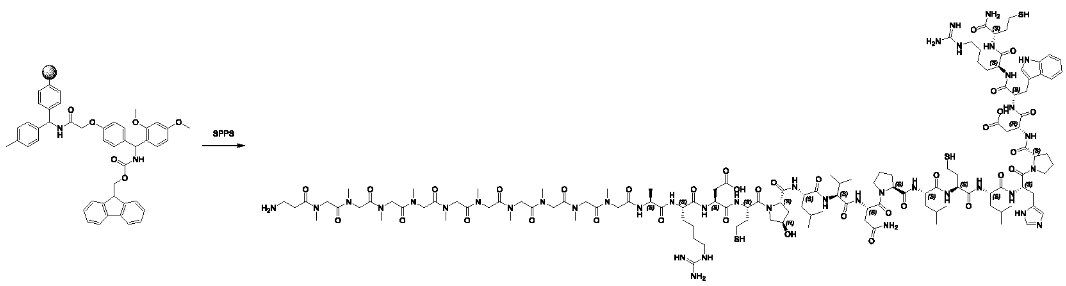


P14

The polypeptide was synthesized using standard Fmoc chemistry:

1. MBHA resin (0.5 mmol, 1.85 g, sub: 0.27 mmol/g) and DMF solvent were added to a reactor and the mixture was shaken for 2 hours.
2. The solvent was pumped off and the resin was rinsed with DMF 3 times.
3. 20% Piperidine/DMF was added and the mixture was mixed for 30 minutes.

4. The solvent was pumped off and the resin was rinsed with DMF 5 times.
5. Fmoc-protected amino acid solution was added, the mixture was mixed for 30 seconds; then a coupling reagent was added and the mixture was bubbled with nitrogen for 1 hour.
6. Steps 2-5 were repeated for the coupling of the subsequent amino acid.

| # | Raw materials | Coupling reagents |
|---|---|---|
| 1 | Fmoc-HCys(Trt)-OH (2.0 eq.) | HATU (1.90 eq.) and DIEA (4.0 eq.) |
| 2 | Fmoc-HArg(Pbf)-OH (2.0 eq.) | HATU (1.90 eq.) and DIEA (4.0 eq.) |
| 3 | Fmoc-Trp(Boc)-OH (3.0 eq.) | HATU (2.85 eq.) and DIEA (6.0 eq.) |
| 4 | Fmoc-D-Asp(OtBu)-OH (3.0 eq.) | HATU (2.85 eq.) and DIEA (6.0 eq.) |
| 5 | Fmoc-Pro-OH (3.0 eq.) | HATU (2.85 eq.) and DIEA (6.0 eq.) |
| 6 | Fmoc-His(Trt)-OH (3.0 eq.) | HATU (2.85 eq.) and DIEA (6.0 eq.) |
| 7 | Fmoc-Leu-OH (3.0 eq.) | HATU (2.85 eq.) and DIEA (6.0 eq.) |
| 8 | Fmoc-HCys(Trt)-OH (2.0 eq.) | HATU (1.90 eq.) and DIEA (4.0 eq.) |
| 9 | Fmoc-Leu-OH (3.0 eq.) | HATU (2.85 eq.) and DIEA (6.0 eq.) |
| 10 | Fmoc-Pro-OH (3.0 eq.) | HATU (2.85 eq.) and DIEA (6.0 eq.) |
| 11 | Fmoc-Asn(Trt)-OH (3.0 eq.) | HATU (2.85 eq.) and DIEA (6.0 eq.) |
| 12 | Fmoc-Val-OH (3.0 eq.) | HATU (2.85 eq.) and DIEA (6.0 eq.) |
| 13 | Fmoc-Leu-OH (3.0 eq.) | HATU (2.85 eq.) and DIEA (6.0 eq.) |
| 14 | Fmoc-Hyp-OH (3.0 eq.) | HATU (2.85 eq.) and DIEA (6.0 eq.) |
| 15 | Fmoc-HCys(Trt)-OH (2.0 eq.) | HATU (1.90 eq.) and DIEA (4.0 eq.) |
| 16 | Fmoc-Asp(OtBu)-OH (3.0 eq.) | HATU (2.85 eq.) and DIEA (6.0 eq.) |
| 17 | Fmoc-HArg(Pbf)-OH (2.0 eq.) | HATU (1.90 eq.) and DIEA (4.0 eq.) |
| 18 | Fmoc-Sar-OH (3.0 eq.) | HATU (2.85 eq.) and DIEA (6.0 eq.) |
| 19 | Fmoc-Sar-OH (3.0 eq.) | HATU (2.85 eq.) and DIEA (6.0 eq.) |
| 20 | Fmoc-Sar-OH (3.0 eq.) | HATU (2.85 eq.) and DIEA (6.0 eq.) |
| 21 | Fmoc-Sar-OH (3.0 eq.) | HATU (2.85 eq.) and DIEA (6.0 eq.) |
| 22 | Fmoc-Sar-OH (3.0 eq.) | HATU (2.85 eq.) and DIEA (6.0 eq.) |
| 23 | Fmoc-Sar-OH (3.0 eq.) | HATU (2.85 eq.) and DIEA (6.0 eq.) |
| 24 | Fmoc-Sar-OH (3.0 eq.) | HATU (2.85 eq.) and DIEA (6.0 eq.) |
| 25 | Fmoc-Sar-OH (3.0 eq.) | HATU (2.85 eq.) and DIEA (6.0 eq.) |
| 26 | Fmoc-Sar-OH (3.0 eq.) | HATU (2.85 eq.) and DIEA (6.0 eq.) |
| 27 | Fmoc-Sar-OH (3.0 eq.) | HATU (2.85 eq.) and DIEA (6.0 eq.) |
| 28 | Fmoc-β-Ala-OH (3.0 eq.) | HATU (2.85 eq.) and DIEA (6.0 eq.) |

Removal of the Fmoc protecting agent was carried out using 20% piperidine/DMF solution for 30 minutes. The coupling reaction was monitored using ninhydrin, and the resin was washed with DMF 5 times.
Peptide cleavage and purification:

1. Side chain-protected polypeptide was added to a reaction flask, a cleavage buffer (90% TFA/2.5% TIS/2.5% $H_2O$/5.0% DTT) was added and the mixture was stirred at room temperature for 2 hours.
2. Ice-cold diisopropyl ether was added to precipitate out the polypeptide, and centrifugation (3 min at 3000 rpm) was carried out.

3. The polypeptide was washed with diisopropyl ether twice.
4. The resulting product was dried in vacuo to obtain crude peptide compound P14 (1.50 g, crude) as a white solid.

Synthetic method for HSK-P14:

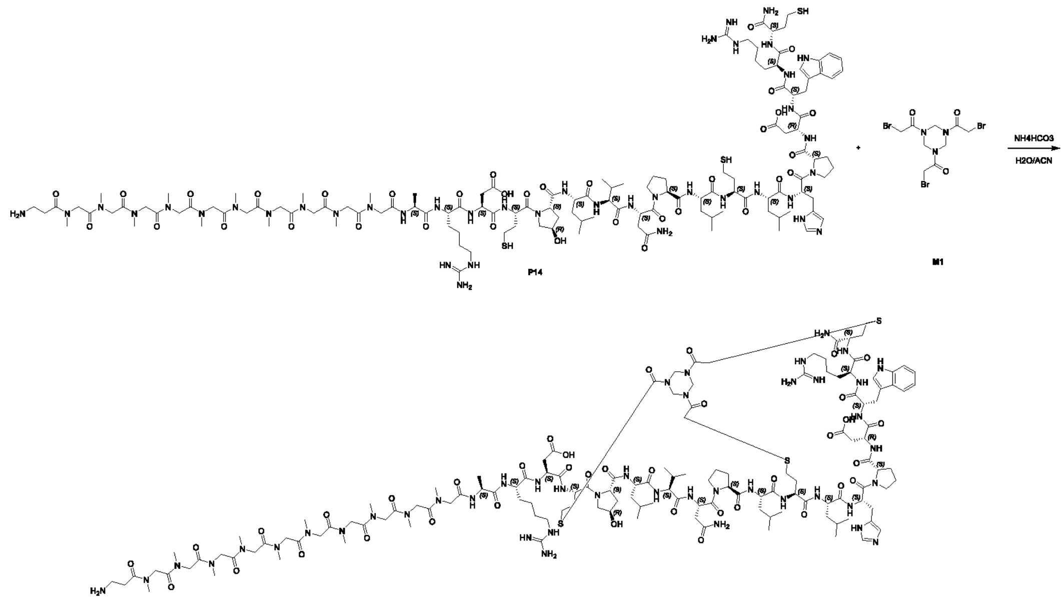


P14

M1

HSK-P14

The crude peptide P14 was dissolved in 50% MeCN/$H_2O$ (500 mL), and TATB (purchased from PharmaBlock Sciences (Nanjing), Inc., 270 mg, 0.60 mmol) was slowly added for at least 30 minutes at room temperature under stirring. After the addition, the mixture was stirred at room temperature for 30 minutes, ammonium bicarbonate was added to adjust the mixture to pH 8, and the reaction solution was stirred at room temperature for 12 hours. When the reaction was completed as shown by LC-MS, the resulting product was purified by preparative HPLC (mobile phase A: 0.075% TFA in $H_2O$, B: $CH_3CN$) to obtain HSK-P14 (135 mg, purity: 95.3%) as a white solid.

Synthesis of bicyclic peptide HSK-P15

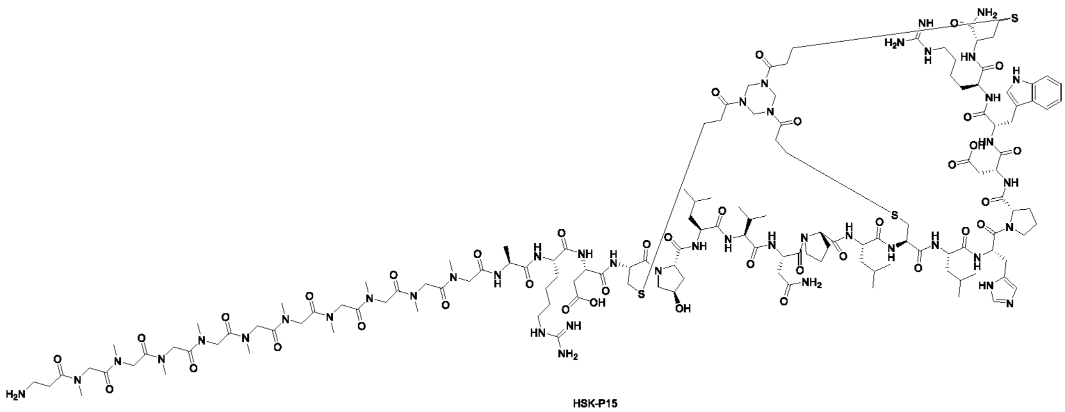

HSK-P15

Synthetic method for P15:

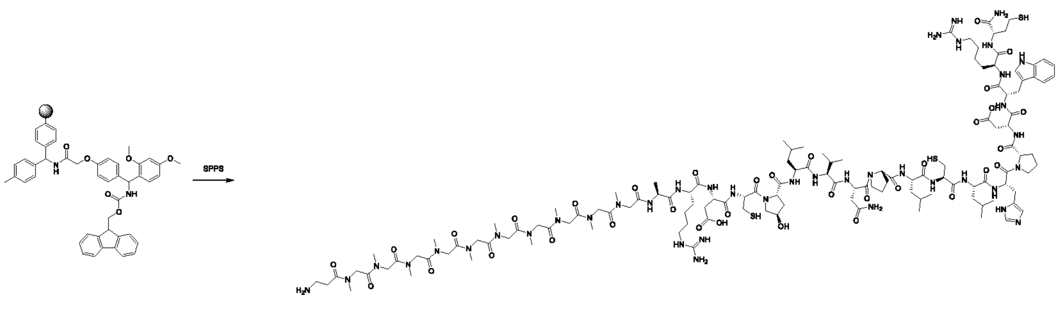


P16

The polypeptide was synthesized using standard Fmoc chemistry:

1. MBHA resin (0.5 mmol, 1.85 g, sub: 0.27 mmol/g) and DMF solvent were added to a reactor and the mixture was shaken for 2 hours.
2. The solvent was pumped off and the resin was rinsed with DMF 3 times.
3. 20% Piperidine/DMF was added and the mixture was mixed for 30 minutes.
4. The solvent was pumped off and the resin was rinsed with DMF 5 times.
5. Fmoc-protected amino acid solution was added, the mixture was mixed for 30 seconds; then a coupling reagent was added and the mixture was bubbled with nitrogen for 1 hour.
6. Steps 2-5 were repeated for the coupling of the subsequent amino acid.

| # | Raw materials | Coupling reagents |
|---|---|---|
| 1 | Fmoc-HCys(Trt)-OH (2.0 eq.) | HATU (1.90 eq.) and DIEA (4.0 eq.) |
| 2 | Fmoc-HArg(Pbf)-OH (2.0 eq.) | HATU (1.90 eq.) and DIEA (4.0 eq.) |
| 3 | Fmoc-Trp(Boc)-OH (3.0 eq.) | HATU (2.85 eq.) and DIEA (6.0 eq.) |
| 4 | Fmoc-D-Asp(OtBu)-OH (3.0 eq.) | HATU (2.85 eq.) and DIEA (6.0 eq.) |
| 5 | Fmoc-Pro-OH (3.0 eq.) | HATU (2.85 eq.) and DIEA (6.0 eq.) |
| 6 | Fmoc-His(Trt)-OH (3.0 eq.) | HATU (2.85 eq.) and DIEA (6.0 eq.) |
| 7 | Fmoc-Leu-OH (3.0 eq.) | HATU (2.85 eq.) and DIEA (6.0 eq.) |
| 8 | Fmoc-Cys(Trt)-OH (3.0 eq.) | HATU (2.85 eq.) and DIEA (6.0 eq.) |
| 9 | Fmoc-Leu-OH (3.0 eq.) | HATU (2.85 eq.) and DIEA (6.0 eq.) |
| 10 | Fmoc-Pro-OH (3.0 eq.) | HATU (2.85 eq.) and DIEA (6.0 eq.) |
| 11 | Fmoc-Asn(Trt)-OH (3.0 eq.) | HATU (2.85 eq.) and DIEA (6.0 eq.) |
| 12 | Fmoc-Val-OH (3.0 eq.) | HATU (2.85 eq.) and DIEA (6.0 eq.) |
| 13 | Fmoc-Leu-OH (3.0 eq.) | HATU (2.85 eq.) and DIEA (6.0 eq.) |
| 14 | Fmoc-Hyp-OH (3.0 eq.) | HATU (2.85 eq.) and DIEA (6.0 eq.) |
| 15 | Fmoc-Cys(Trt)-OH (3.0 eq.) | HATU (2.85 eq.) and DIEA (6.0 eq.) |
| 16 | Fmoc-Asp(OtBu)-OH (3.0 eq.) | HATU (2.85 eq.) and DIEA (6.0 eq.) |
| 17 | Fmoc-HArg(Pbf)-OH (2.0 eq.) | HATU (1.90 eq.) and DIEA (4.0 eq.) |
| 18 | Fmoc-Sar-OH (3.0 eq.) | HATU (2.85 eq.) and DIEA (6.0 eq.) |
| 19 | Fmoc-Sar-OH (3.0 eq.) | HATU (2.85 eq.) and DIEA (6.0 eq.) |
| 20 | Fmoc-Sar-OH (3.0 eq.) | HATU (2.85 eq.) and DIEA (6.0 eq.) |
| 21 | Fmoc-Sar-OH (3.0 eq.) | HATU (2.85 eq.) and DIEA (6.0 eq.) |
| 22 | Fmoc-Sar-OH (3.0 eq.) | HATU (2.85 eq.) and DIEA (6.0 eq.) |

(continued)

| # | Raw materials | Coupling reagents |
|---|---|---|
| 23 | Fmoc-Sar-OH (3.0 eq.) | HATU (2.85 eq.) and DIEA (6.0 eq.) |
| 24 | Fmoc-Sar-OH (3.0 eq.) | HATU (2.85 eq.) and DIEA (6.0 eq.) |
| 25 | Fmoc-Sar-OH (3.0 eq.) | HATU (2.85 eq.) and DIEA (6.0 eq.) |
| 26 | Fmoc-Sar-OH (3.0 eq.) | HATU (2.85 eq.) and DIEA (6.0 eq.) |
| 27 | Fmoc-Sar-OH (3.0 eq.) | HATU (2.85 eq.) and DIEA (6.0 eq.) |
| 28 | Fmoc-β-Ala-OH (3.0 eq.) | HATU (2.85 eq.) and DIEA (6.0 eq.) |

Removal of the Fmoc protecting agent was carried out using 20% piperidine/DMF solution for 30 minutes. The coupling reaction was monitored using ninhydrin, and the resin was washed with DMF 5 times.

Peptide cleavage and purification:

1. Side chain-protected polypeptide was added to a reaction flask, a cleavage buffer (90% TFA/2.5% TIS/2.5% $H_2O$/5.0% DTT) was added and the mixture was stirred at room temperature for 2 hours.
2. Ice-cold diisopropyl ether was added to precipitate out the polypeptide, and centrifugation (3 min at 3000 rpm) was carried out.
3. The polypeptide was washed with diisopropyl ether twice.
4. The resulting product was dried in vacuo to obtain crude peptide compound P15 (1.60 g, crude) as a white solid.

Synthetic method for HSK-P15:

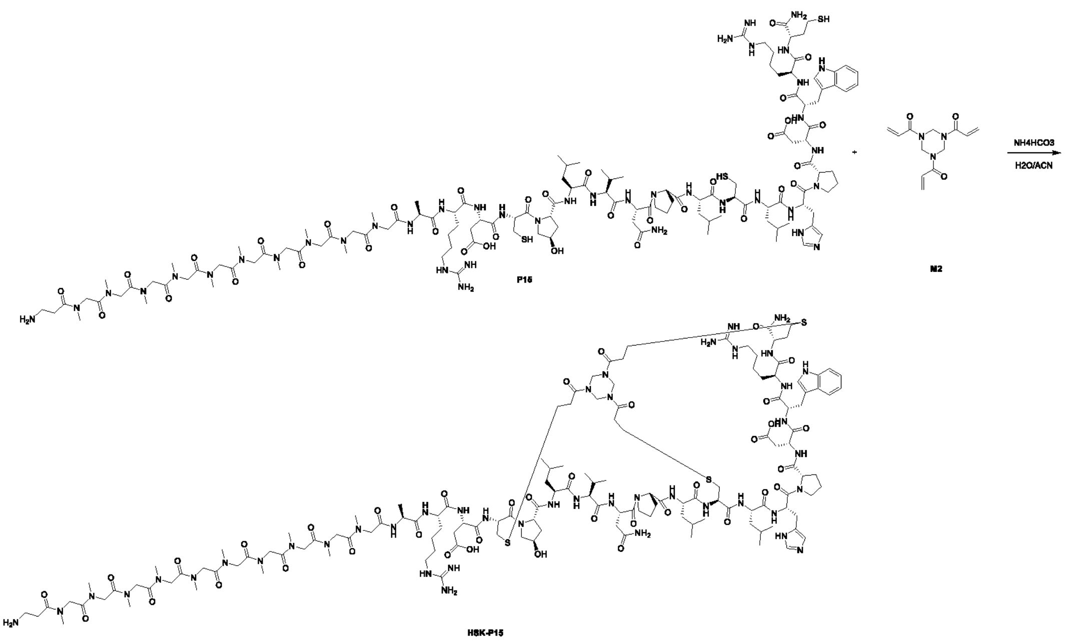


The crude peptide P15 was dissolved in 50% MeCN/$H_2O$ (500 mL), and TATA (purchased from PharmaBlock Sciences (Nanjing), Inc., 150 mg, 0.60 mmol) was slowly added for at least 30 minutes at room temperature under stirring. After the addition, the mixture was stirred at room temperature for 30 minutes, ammonium bicarbonate was added to adjust the mixture to pH 8, and the reaction solution was stirred at room temperature for 12 hours. When the reaction was completed as shown by LC-MS, the resulting product was purified by preparative HPLC (mobile phase A: 0.075% TFA in $H_2O$, B: $CH_3CN$) to obtain HSK-P15 (115 mg, purity: 95.8%) as a white solid.

Synthesis of bicyclic peptide HSK-P17

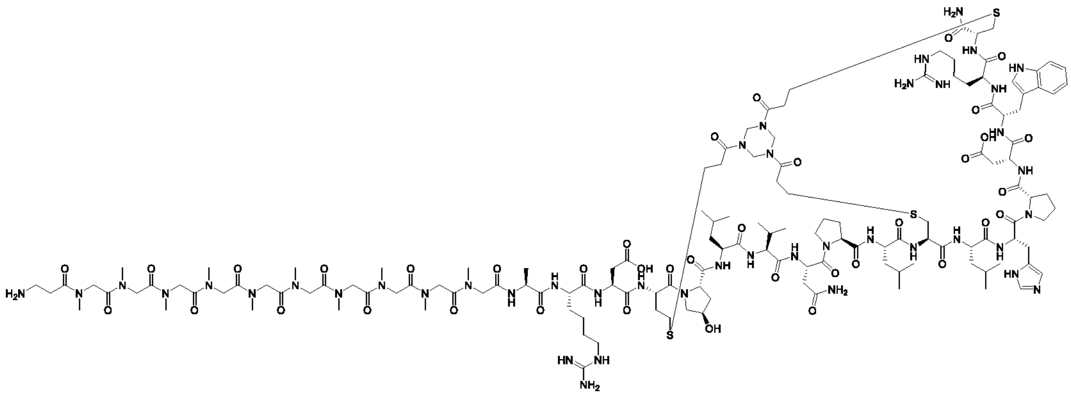

HSK-P17

Synthetic method for P17:

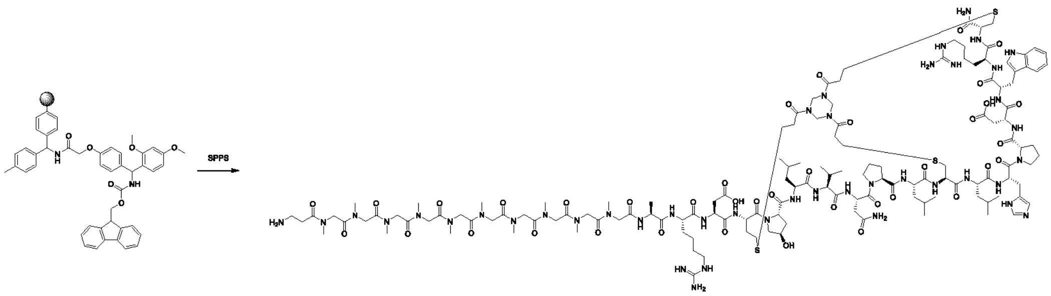


P17

The polypeptide was synthesized using standard Fmoc chemistry:

1. MBHA resin (0.5 mmol, 1.85 g, sub: 0.27 mmol/g) and DMF solvent were added to a reactor and the mixture was shaken for 2 hours.
2. The solvent was pumped off and the resin was rinsed with DMF 3 times.
3. 20% Piperidine/DMF was added and the mixture was mixed for 30 minutes.
4. The solvent was pumped off and the resin was rinsed with DMF 5 times.
5. Fmoc-protected amino acid solution was added, the mixture was mixed for 30 seconds; then a coupling reagent was added and the mixture was bubbled with nitrogen for 1 hour.
6. Steps 2-5 were repeated for the coupling of the subsequent amino acid.

| # | Raw materials | Coupling reagents |
|---|---|---|
| 1 | Fmoc-Cys(Trt)-OH (3.0 eq.) | HATU (2.85 eq.) and DIEA (6.0 eq.) |
| 2 | Fmoc-HArg(Pbf)-OH (2.0 eq.) | HATU (1.90 eq.) and DIEA (4.0 eq.) |
| 3 | Fmoc-Trp(Boc)-OH (3.0 eq.) | HATU (2.85 eq.) and DIEA (6.0 eq.) |
| 4 | Fmoc-D-Asp(OtBu)-OH (3.0 eq.) | HATU (2.85 eq.) and DIEA (6.0 eq.) |
| 5 | Fmoc-Pro-OH (3.0 eq.) | HATU (2.85 eq.) and DIEA (6.0 eq.) |

(continued)

| # | Raw materials | Coupling reagents |
|---|---|---|
| 6 | Fmoc-His(Trt)-OH (3.0 eq.) | HATU (2.85 eq.) and DIEA (6.0 eq.) |
| 7 | Fmoc-Leu-OH (3.0 eq.) | HATU (2.85 eq.) and DIEA (6.0 eq.) |
| 8 | Fmoc-Cys(Trt)-OH (3.0 eq.) | HATU (2.85 eq.) and DIEA (6.0 eq.) |
| 9 | Fmoc-Leu-OH (3.0 eq.) | HATU (2.85 eq.) and DIEA (6.0 eq.) |
| 10 | Fmoc-Pro-OH (3.0 eq.) | HATU (2.85 eq.) and DIEA (6.0 eq.) |
| 11 | Fmoc-Asn(Trt)-OH (3.0 eq.) | HATU (2.85 eq.) and DIEA (6.0 eq.) |
| 12 | Fmoc-Val-OH (3.0 eq.) | HATU (2.85 eq.) and DIEA (6.0 eq.) |
| 13 | Fmoc-Leu-OH (3.0 eq.) | HATU (2.85 eq.) and DIEA (6.0 eq.) |
| 14 | Fmoc-Hyp-OH (3.0 eq.) | HATU (2.85 eq.) and DIEA (6.0 eq.) |
| 15 | Fmoc-HCys(Trt)-OH (2.0 eq.) | HATU (1.90 eq.) and DIEA (4.0 eq.) |
| 16 | Fmoc-Asp(OtBu)-OH (3.0 eq.) | HATU (2.85 eq.) and DIEA (6.0 eq.) |
| 17 | Fmoc-HArg(Pbf)-OH (2.0 eq.) | HATU (1.90 eq.) and DIEA (4.0 eq.) |
| 18 | Fmoc-Sar-OH (3.0 eq.) | HATU (2.85 eq.) and DIEA (6.0 eq.) |
| 19 | Fmoc-Sar-OH (3.0 eq.) | HATU (2.85 eq.) and DIEA (6.0 eq.) |
| 20 | Fmoc-Sar-OH (3.0 eq.) | HATU (2.85 eq.) and DIEA (6.0 eq.) |
| 21 | Fmoc-Sar-OH (3.0 eq.) | HATU (2.85 eq.) and DIEA (6.0 eq.) |
| 22 | Fmoc-Sar-OH (3.0 eq.) | HATU (2.85 eq.) and DIEA (6.0 eq.) |
| 23 | Fmoc-Sar-OH (3.0 eq.) | HATU (2.85 eq.) and DIEA (6.0 eq.) |
| 24 | Fmoc-Sar-OH (3.0 eq.) | HATU (2.85 eq.) and DIEA (6.0 eq.) |
| 25 | Fmoc-Sar-OH (3.0 eq.) | HATU (2.85 eq.) and DIEA (6.0 eq.) |
| 26 | Fmoc-Sar-OH (3.0 eq.) | HATU (2.85 eq.) and DIEA (6.0 eq.) |
| 27 | Fmoc-Sar-OH (3.0 eq.) | HATU (2.85 eq.) and DIEA (6.0 eq.) |
| 28 | Fmoc-β-Ala-OH (3.0 eq.) | HATU (2.85 eq.) and DIEA (6.0 eq.) |

Removal of the Fmoc protecting agent was carried out using 20% piperidine/DMF solution for 30 minutes. The coupling reaction was monitored using ninhydrin, and the resin was washed with DMF 5 times.

Peptide cleavage and purification:

1. Side chain-protected polypeptide was added to a reaction flask, a cleavage buffer (90% TFA/2.5% TIS/2.5% $H_2O$/5.0% DTT) was added and the mixture was stirred at room temperature for 2 hours.
2. Ice-cold diisopropyl ether was added to precipitate out the polypeptide, and centrifugation (3 min at 3000 rpm) was carried out.
3. The polypeptide was washed with diisopropyl ether twice.
4. The resulting product was dried in vacuo to obtain crude peptide compound P17 (1.75 g, crude) as a white solid.

Synthetic method for HSK-P17:

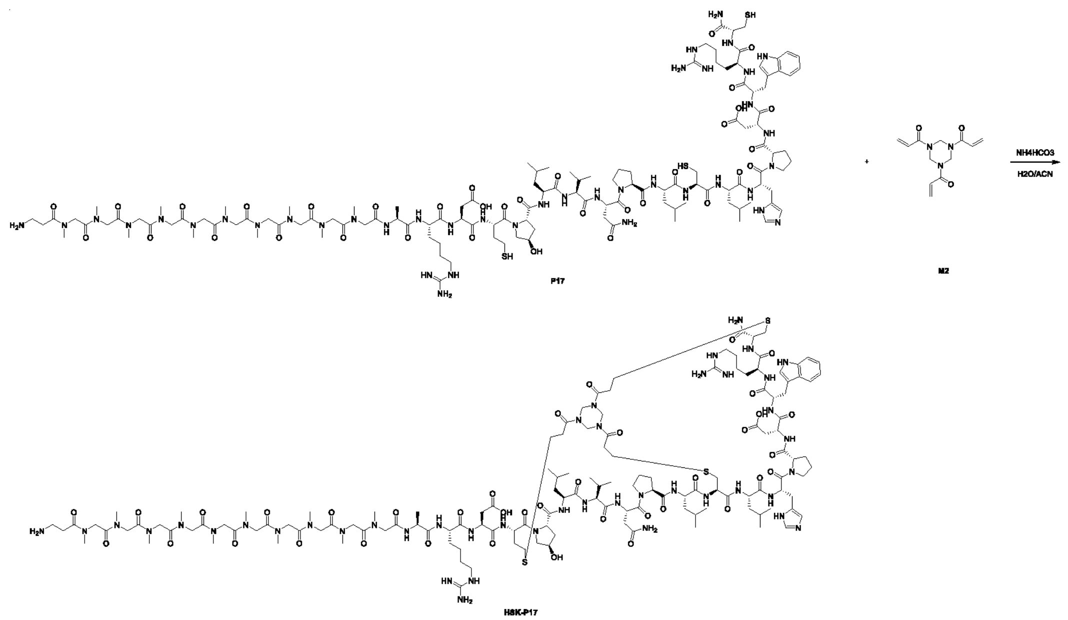


P17

M2

HSK-P17

The crude peptide P17 was dissolved in 50% MeCN/$H_2O$ (500 mL), and TATA (purchased from PharmaBlock Sciences (Nanjing), Inc., 150 mg, 0.60 mmol) was slowly added for at least 30 minutes at room temperature under stirring. After the addition, the mixture was stirred at room temperature for 30 minutes, ammonium bicarbonate was added to adjust the mixture to pH 8, and the reaction solution was stirred at room temperature for 12 hours. When the reaction was completed as shown by LC-MS, the resulting product was purified by preparative HPLC (mobile phase A: 0.075% TFA in $H_2O$, B: $CH_3CN$) to obtain HSK-P17 (110 mg, purity: 96.8%) as a white solid.

Synthesis of bicyclic peptide HSK-P30

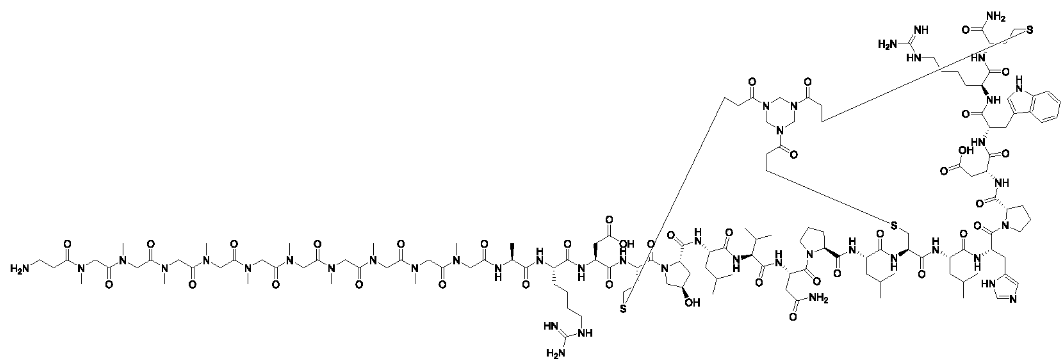

HSK-P30

Synthetic method for P30:

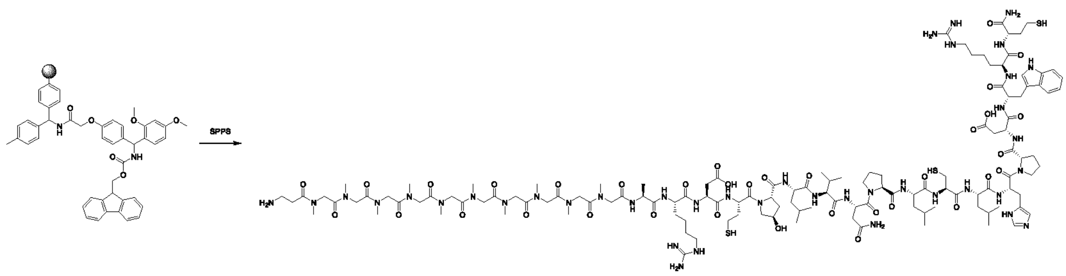


P30

The polypeptide was synthesized using standard Fmoc chemistry:

1. MBHA resin (0.5 mmol, 1.85 g, sub: 0.27 mmol/g) and DMF solvent were added to a reactor and the mixture was shaken for 2 hours.
2. The solvent was pumped off and the resin was rinsed with DMF 3 times.
3. 20% Piperidine/DMF was added and the mixture was mixed for 30 minutes.
4. The solvent was pumped off and the resin was rinsed with DMF 5 times.
5. Fmoc-protected amino acid solution was added, the mixture was mixed for 30 seconds; then a coupling reagent was added and the mixture was bubbled with nitrogen for 1 hour.
6. Steps 2-5 were repeated for the coupling of the subsequent amino acid.

| # | Raw materials | Coupling reagents |
|---|---|---|
| 1 | Fmoc-HCys(Trt)-OH (2.0 eq.) | HATU (1.90 eq.) and DIEA (4.0 eq.) |
| 2 | Fmoc-HArg(Pbf)-OH (2.0 eq.) | HATU (1.90 eq.) and DIEA (4.0 eq.) |
| 3 | Fmoc-Trp(Boc)-OH (3.0 eq.) | HATU (2.85 eq.) and DIEA (6.0 eq.) |
| 4 | Fmoc-D-Asp(OtBu)-OH (3.0 eq.) | HATU (2.85 eq.) and DIEA (6.0 eq.) |
| 5 | Fmoc-Pro-OH (3.0 eq.) | HATU (2.85 eq.) and DIEA (6.0 eq.) |
| 6 | Fmoc-His(Trt)-OH (3.0 eq.) | HATU (2.85 eq.) and DIEA (6.0 eq.) |
| 7 | Fmoc-Leu-OH (3.0 eq.) | HATU (2.85 eq.) and DIEA (6.0 eq.) |
| 8 | Fmoc-Cys(Trt)-OH (3.0 eq.) | HATU (2.85 eq.) and DIEA (6.0 eq.) |
| 9 | Fmoc-Leu-OH (3.0 eq.) | HATU (2.85 eq.) and DIEA (6.0 eq.) |
| 10 | Fmoc-Pro-OH (3.0 eq.) | HATU (2.85 eq.) and DIEA (6.0 eq.) |
| 11 | Fmoc-Asn(Trt)-OH (3.0 eq.) | HATU (2.85 eq.) and DIEA (6.0 eq.) |
| 12 | Fmoc-Val-OH (3.0 eq.) | HATU (2.85 eq.) and DIEA (6.0 eq.) |
| 13 | Fmoc-Leu-OH (3.0 eq.) | HATU (2.85 eq.) and DIEA (6.0 eq.) |
| 14 | Fmoc-Hyp-OH (3.0 eq.) | HATU (2.85 eq.) and DIEA (6.0 eq.) |
| 15 | Fmoc-HCys(Trt)-OH (2.0 eq.) | HATU (1.90 eq.) and DIEA (4.0 eq.) |
| 16 | Fmoc-Asp(OtBu)-OH (3.0 eq.) | HATU (2.85 eq.) and DIEA (6.0 eq.) |
| 17 | Fmoc-HArg(Pbf)-OH (2.0 eq.) | HATU (1.90 eq.) and DIEA (4.0 eq.) |
| 18 | Fmoc-Sar-OH (3.0 eq.) | HATU (2.85 eq.) and DIEA (6.0 eq.) |
| 19 | Fmoc-Sar-OH (3.0 eq.) | HATU (2.85 eq.) and DIEA (6.0 eq.) |
| 20 | Fmoc-Sar-OH (3.0 eq.) | HATU (2.85 eq.) and DIEA (6.0 eq.) |
| 21 | Fmoc-Sar-OH (3.0 eq.) | HATU (2.85 eq.) and DIEA (6.0 eq.) |
| 22 | Fmoc-Sar-OH (3.0 eq.) | HATU (2.85 eq.) and DIEA (6.0 eq.) |
| 23 | Fmoc-Sar-OH (3.0 eq.) | HATU (2.85 eq.) and DIEA (6.0 eq.) |

(continued)

| # | Raw materials | Coupling reagents |
|---|---|---|
| 24 | Fmoc-Sar-OH (3.0 eq.) | HATU (2.85 eq.) and DIEA (6.0 eq.) |
| 25 | Fmoc-Sar-OH (3.0 eq.) | HATU (2.85 eq.) and DIEA (6.0 eq.) |
| 26 | Fmoc-Sar-OH (3.0 eq.) | HATU (2.85 eq.) and DIEA (6.0 eq.) |
| 27 | Fmoc-Sar-OH (3.0 eq.) | HATU (2.85 eq.) and DIEA (6.0 eq.) |
| 28 | Fmoc-β-Ala-OH (3.0 eq.) | HATU (2.85 eq.) and DIEA (6.0 eq.) |

Removal of the Fmoc protecting agent was carried out using 20% piperidine/DMF solution for 30 minutes. The coupling reaction was monitored using ninhydrin, and the resin was washed with DMF 5 times.

Peptide cleavage and purification:

1. Side chain-protected polypeptide was added to a reaction flask, a cleavage buffer (90% TFA/2.5% TIS/2.5% $H_2O$/5.0% DTT) was added and the mixture was stirred at room temperature for 2 hours.
2. Ice-cold diisopropyl ether was added to precipitate out the polypeptide, and centrifugation (3 min at 3000 rpm) was carried out.
3. The polypeptide was washed with diisopropyl ether twice.
4. The resulting product was dried in vacuo to obtain crude peptide compound P30 (1.55 g, crude) as a white solid.

Synthetic method for HSK-P30:

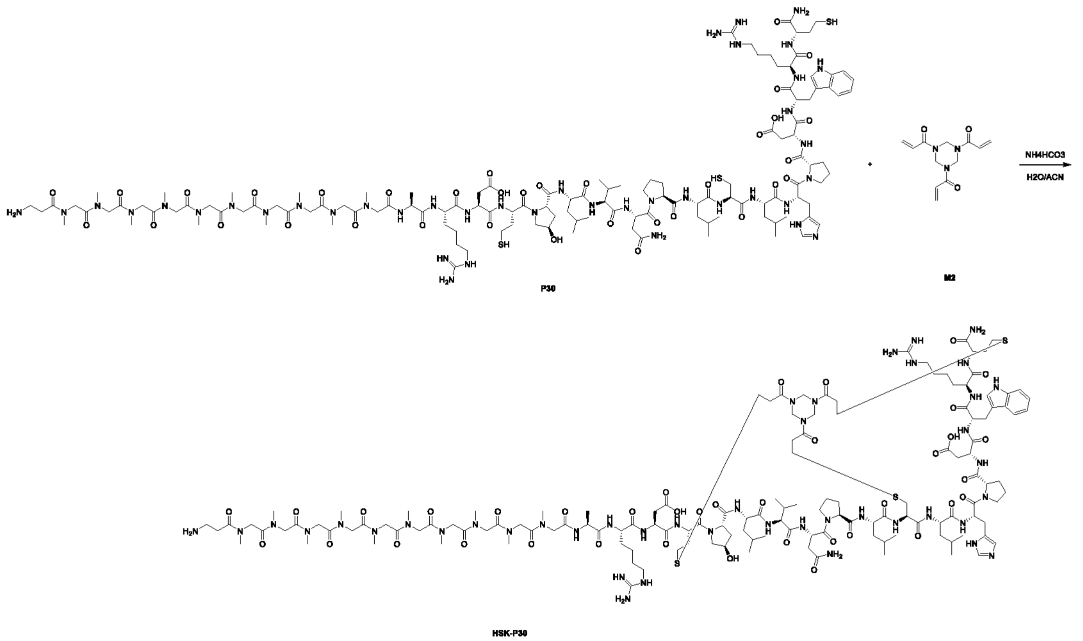


P30 M2

HSK-P30

The crude peptide P30 was dissolved in 50% MeCN/$H_2O$ (500 mL), and TATA (purchased from PharmaBlock Sciences (Nanjing), Inc., 150 mg, 0.60 mmol) was slowly added for at least 30 minutes at room temperature under stirring. After the addition, the mixture was stirred at room temperature for 30 minutes, ammonium bicarbonate was added to adjust the mixture to pH 8, and the reaction solution was stirred at room temperature for 12 hours. When the reaction was completed as shown by LC-MS, the resulting product was purified by preparative HPLC (mobile phase A: 0.075% TFA in $H_2O$, B: $CH_3CN$) to obtain HSK-P30 (115 mg, purity: 96.4%) as a white solid.

HSK-P16, HSK-P21, HSK-P23, HSK-P24, HSK-P25, HSK-P26, HSK-P27, HSK-P28, HSK-P29, HSK-P31, HSK-P32, and HSK-P33 were respectively synthesized according to the above-mentioned method.

HSK-P15: amino acid sequence: (β-Ala)-Sar10-Ala(hArg)Asp-SEQ ID NO:2 (Cys-HyP-Leu-Val-Asn-Pro-Leu-Cys-Leu-His-Pro-(D-Asp)-Trp-hArg-Hcys), molecular scaffold: TATA.

HSK-P16: amino acid sequence: (β-Ala)-Sar10-Ala(hArg)Asp-SEQ ID NO:3 (Cys-HyP-Leu-Val-Asn-Pro-Leu-Hcys-Leu-His-Pro-(D-Asp)-Trp-hArg-Cys), molecular scaffold: TATA.

HSK-P17: amino acid sequence: (β-Ala)-Sar10-Ala(hArg)Asp-SEQ ID NO:4 (Hcys-HyP-Leu-Val-Asn-Pro-Leu-Cys-Leu-His-Pro-(D-Asp)-Trp-hArg-Cys), molecular scaffold: TATA.

HSK-P21: amino acid sequence: (β-Ala)-Sar10-Ala(hArg)Asp-SEQ ID NO:1 (Hcys-HyP-Leu-Val-Asn-Pro-Leu-Hcys-Leu-His-Pro-(D-Asp)-Trp-hArg-Hcys), molecular scaffold: TATA.

HSK-P23: amino acid sequence: (β-Ala)-Sar10-Ala(hArg)Asp-SEQ ID NO:2 (Cys-HyP-Leu-Val-Asn-Pro-Leu-Cys-Leu-His-Pro-(D-Asp)-Trp-hArg-Hcys), molecular scaffold: TATB.

HSK-P24: amino acid sequence: (β-Ala)-Sar10-Ala(hArg)Asp-SEQ ID NO:3 (Cys-HyP-Leu-Val-Asn-Pro-Leu-Hcys-Leu-His-Pro-(D-Asp)-Trp-hArg-Cys), molecular scaffold: TATB.

HSK-P25: amino acid sequence: (β-Ala)-Sar10-Ala(hArg)Asp-SEQ ID NO:4 (Hcys-HyP-Leu-Val-Asn-Pro-Leu-Cys-Leu-His-Pro-(D-Asp)-Trp-hArg-Cys), molecular scaffold: TATB.

HSK-P26: amino acid sequence: (β-Ala)-Sar10-Ala(hArg)Asp-SEQ ID NO:7 (Hcys-HyP-Leu-Val-Asn-Pro-Leu-Hcys-Leu-His-Pro-(D-Asp)-Trp-hArg-Cys), molecular scaffold: TATB.

HSK-P27: amino acid sequence: (β-Ala)-Sar10-Ala(hArg)Asp-SEQ ID NO:6 (Hcys-HyP-Leu-Val-Asn-Pro-Leu-Cys-Leu-His-Pro-(D-Asp)-Trp-hArg-Hcys), molecular scaffold: TATB.

HSK-P28: amino acid sequence: (β-Ala)-Sar10-Ala(hArg)Asp-SEQ ID NO:5 (Cys-HyP-Leu-Val-Asn-Pro-Leu-Hcys-Leu-His-Pro-(D-Asp)-Trp-hArg-Hcys), molecular scaffold: TATB.

HSK-P29: amino acid sequence: (β-Ala)-Sar10-Ala(hArg)Asp-SEQ ID NO:7 (Hcys-HyP-Leu-Val-Asn-Pro-Leu-Hcys-Leu-His-Pro-(D-Asp)-Trp-hArg-Cys), molecular scaffold: TATA.

HSK-P30: amino acid sequence: (β-Ala)-Sar10-Ala(hArg)Asp-SEQ ID NO:6 (Hcys-HyP-Leu-Val-Asn-Pro-Leu-Cys-Leu-His-Pro-(D-Asp)-Trp-hArg-Hcys), molecular scaffold: TATA.

HSK-P31: amino acid sequence: (β-Ala)-Sar10-Ala(hArg)Asp-SEQ ID NO:5 (Cys-HyP-Leu-Val-Asn-Pro-Leu-Hcys-Leu-His-Pro-(D-Asp)-Trp-hArg-Hcys), molecular scaffold: TATA.

HSK-P32: amino acid sequence: (β-Ala)-Sar10-Ala(hArg)Asp-SEQ ID NO:1 (Hcys-HyP-Leu-Val-Asn-Pro-Leu-Hcys-Leu-His-Pro-(D-Asp)-Trp-hArg-Hcys), molecular scaffold:

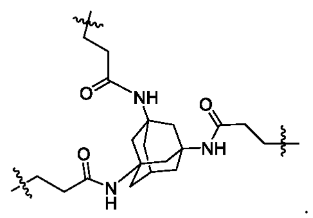

HSK-P33: amino acid sequence: (β-Ala)-Sar10-Ala(hArg)Asp-SEQ ID NO:1 (Hcys-HyP-Leu-Val-Asn-Pro-Leu-Hcys-Leu-His-Pro-(D-Asp)-Trp-hArg-Hcys), molecular scaffold:

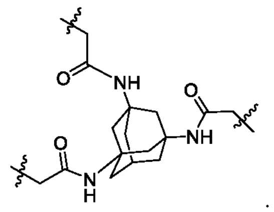

## Intermediate 13

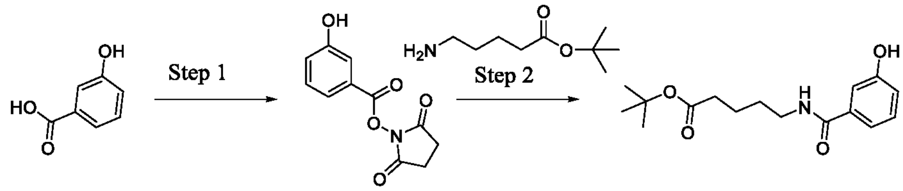


Intermediate 11 Intermediate 12 Intermediate 13

Step 1:

Intermediate 11 (6 g, 43.46 mmol), N-hydroxysuccinimide (5.50 g, 47.74 mmol) and 1-(3-dimethylaminopropyl)-3-ethylcarbodiimide hydrochloride (12.50 g, 65.19 mmol) were added to a mixed system of DCM (12 mL) and DMF (50 mL), and the mixture was reacted at room temperature for 3 h. As monitored by LC-MS, the reaction was completed. The reaction mixture was concentrated under reduced pressure to remove DCM. Water (300 ml) was added and the resulting mixture was extracted with ethyl acetate (100 ml×3). The organic phases were combined, washed with saturated sodium chloride solution, dried over anhydrous sodium sulphate and filtered and the filtrate was concentrated under reduced pressure to obtain intermediate 12 (10 g, 97.83%) as a white solid.

Step 2:

Tert-butyl 5-aminopentanoate (2 g, 11.54 mmol), intermediate 2 (2.71 g, 11.54 mmol) and N,N-diisopropylethylamine (4.48 g, 34.64 mmol) were sequentially added to DMF (20 ml). The mixture was reacted at room temperature for 5 h. Water (100 ml) was added, and the resulting mixture was extracted with ethyl acetate (50 ml×3). The organic phases were combined, washed with saturated sodium chloride solution, dried over anhydrous sodium sulphate and filtered. After the filtrate was concentrated under reduced pressure, the residue was separated and purified by silica gel column chromatography (EA : PE = 1 : 3) to obtain intermediate 13 (1.8 g, 53.17%) as an oil.

LCMS m/z =238.2 [M- tBu +1]$^+$.

**Example 1**

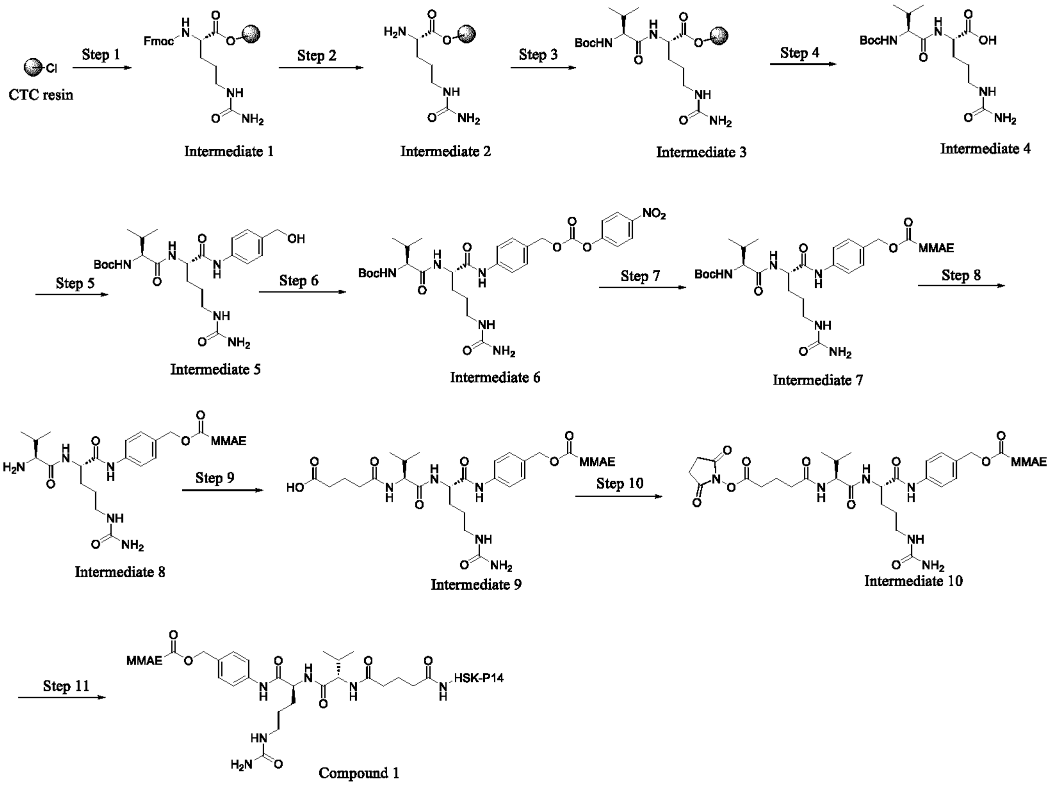


CTC resin, Intermediate 1, Intermediate 2, Intermediate 3, Intermediate 4, Intermediate 5, Intermediate 6, Intermediate 7, Intermediate 8, Intermediate 9, Intermediate 10, Compound 1

Steps 1-4:

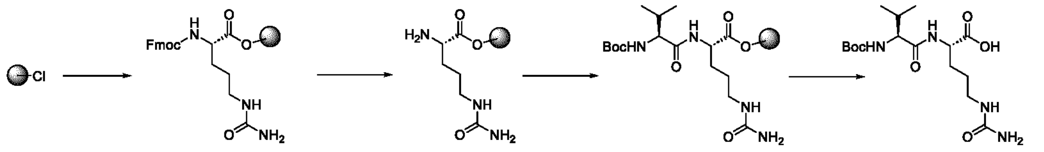

CTC resin Intermediate 1 Intermediate 2 Intermediate 3 Intermediate 4

CTC resin (75 g, 1.0 mmol/g) and Fmoc-L-citrulline (30.0 g, 75.4 mmol, 1.0 eq) were added to dichloromethane (600 mL) and then N,N-diisopropylethylamine (58.4 g, 453 mmol, 6.0 eq) was added. The mixture was reacted for 3 hours. Suction filtration was carried out and the resin was washed with DMF twice. To the resin mixture was added a prepared 20% piperidine/DMF solution, and the system was reacted for 2 hours. Suction filtration was carried out and the resin was washed with DMF twice. To the resin mixture were sequentially added DMF (600 mL), Boc-L-valine (48.0 g, 0.22 mmol, 3.0 eq), HBTU (85.0 g, 0.21 mmol, 2.85 eq) and N,N-diisopropylethylamine (58.4 g, 453 mmol, 6.0 eq), and the system was reacted for 3 hours. Suction filtration was carried out and the resin was washed with DMF (3 times), methanol (3 times) and dichloromethane (twice). To the resin mixture was added a prepared 20% hexafluoroisopropanol/dichloromethane solution and the system was reacted for 30 minutes. Suction filtration was carried out and the previous operation was repeated. The mother liquors were combined and concentrated to dryness to obtain intermediate 4 (25.0 g, yield over four steps = 88%).

LCMS m/z =374.9 $[M+1]^+$.

Step 5:

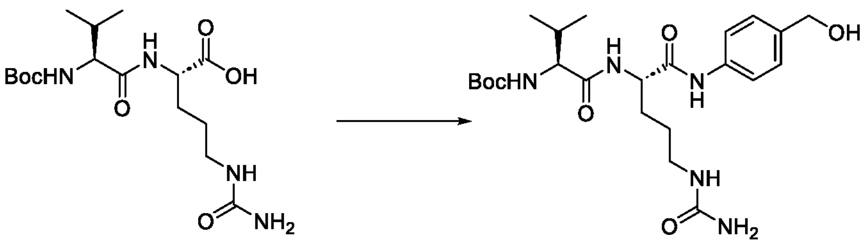

Intermediate 4 Intermediate 5

Intermediate 4 (25 g, 66.7 mmol) was added to dichloromethane (250 mL) and methanol (250 mL) and then p-aminobenzyl alcohol (9.84 g, 80.0 mmol) and EEDQ (39.5 g, 80.0 mmol) were added. The mixture was stirred at room temperature for 16 h and concentrated to dryness. The residue was purified by column chromatography (DCM : MeOH = 10 : 1) to obtain intermediate 5 (10 g, yield = 31%).

LCMS m/z =480.1 $[M+1]^+$.

Step 6:

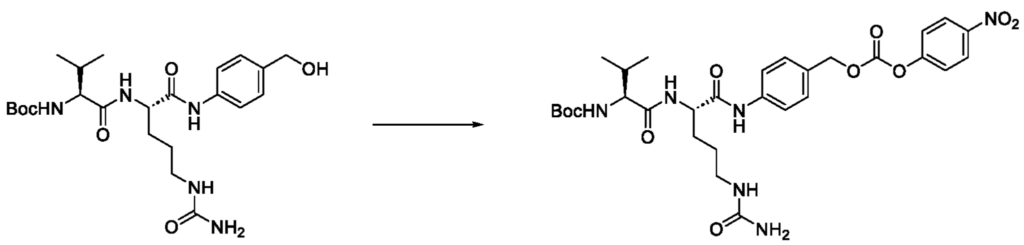

Intermediate 5 Intermediate 6

Intermediate 5 (10.0 g, 20.8 mmol) was added to dry dichloromethane (120 mL) and dry tetrahydrofuran (60 mL). Under nitrogen protection, p-nitrophenyl chloroformate (6.2 g, 31.2 mmol) and pyridine (3.3 g, 41.6 mmol) were then added and the mixture was stirred at room temperature for 5 h and filtered. The mother liquor was concentrated to

dryness and purified by column chromatography (DCM : MeOH = 10 : 1) to obtain intermediate 6 (2.3 g, yield = 16.6%).
LCMS m/z =664.3 [M+1]+.

Step 7:

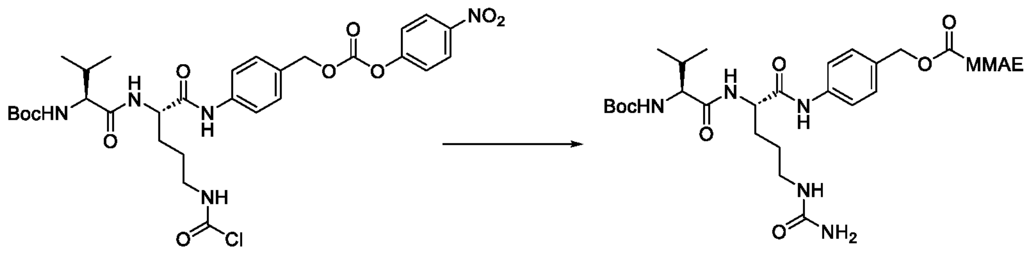

Intermediate 6 Intermediate 7

Intermediate 6 (2.1 g, 3.1 mmol) and N,N-diisopropylethylamine (3.6 g, 31 mmol) were added to DMF (40 mL). Under nitrogen protection, the mixture was stirred at room temperature for 10 minutes and then cooled to 0°C. Then MMAE (purchased from Chengdu Huajieming Biotechnology Co., Ltd., 2.0 g, 3.1 mmol) and HOBT (0.38 g, 3.1 mmol) were added and the mixture was stirred for 30 minutes while the temperature was maintained. The resulting mixture was stirred at room temperature for 18 h and then directly purified by a C18 reverse phase column (0.1% TFA) ($H_2O$ : ACN = 50 : 50) to obtain intermediate 7 (2.7 g, yield = 70%).
LCMS m/z =1223.4 [M+1]+.

Step 8:

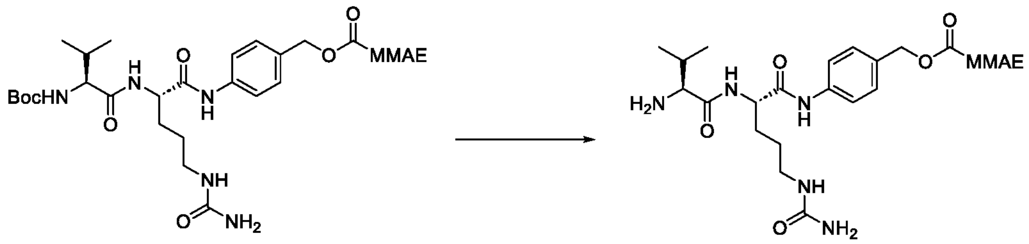

Intermediate 7 Intermediate 8

To intermediate 7 (2.0 g, 1.6 mmol) was added a mixed solution of TFA/DCM = 1 : 10 (34 mL, uniformly mixed) and the mixture was stirred at room temperature for 2 h and concentrated at 25°C to remove the solvent. Tetrahydrofuran (110 mL) and potassium carbonate (2.26 g, 16 mmol) were added and stirred at room temperature for 3 h. The reaction mixture was concentrated to dryness and directly purified by a C18 reverse phase column (0.1% TFA) ($H_2O$ : ACN = 60 : 40) to obtain intermediate 8 (1.4 g, yield = 76.5%).
LCMS m/z =1123.4 [M+1]+.

Step 9:

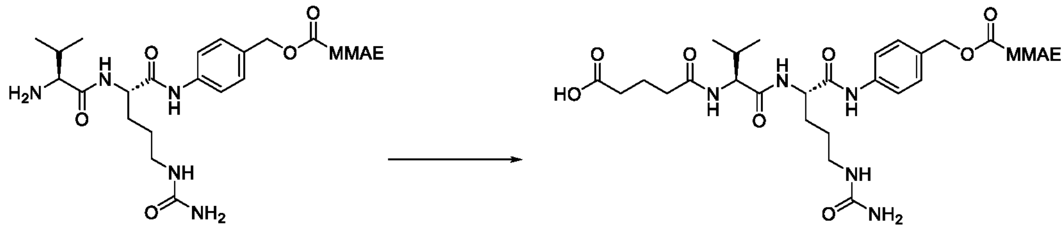

Intermediate 8 Intermediate 9

Intermediate 8 (1.4 g, 1.25 mmol), glutaric anhydride (0.29 g, 2.5 mmol) and N,N-diisopropylethylamine (0.33 g, 2.5 mmol) were added to DMA (14 mL). The mixture was stirred at room temperature for 18 h and purified by a C18 reverse phase preparative column (0.1% TFA) ($H_2O$ : ACN = 50 : 50) to obtain intermediate 9 (1.3 g, yield = 84.4%).
LCMS m/z =1237.4 $[M+1]^+$.

Step 10:

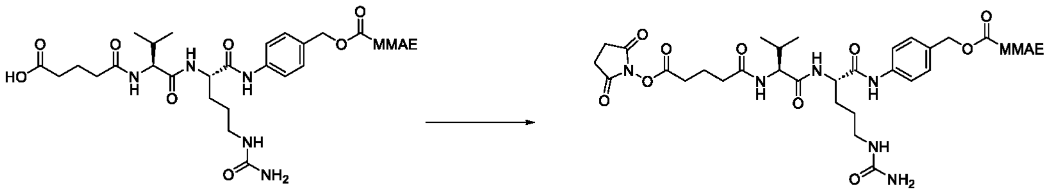

Intermediate 9 Intermediate 10

Intermediate 9 (1.3 g, 1.05 mmol) was added to DMA (58 mL) and dichloromethane (20 mL). Under nitrogen protection, the mixture was cooled to 0°C and N-hydroxysuccinimide (0.37 g, 3.15 mmol) and EDCI (0.61 g, 3.15 mmol) were added. The resulting mixture was stirred at room temperature for 18 h and purified by a C18 reverse phase preparative column (0.1% TFA) ($H_2O$ : ACN = 50 : 50) to obtain intermediate 10 (1.0 g, yield = 71.4%).
LCMS m/z =1334.5 $[M+1]^+$.

Step 11:

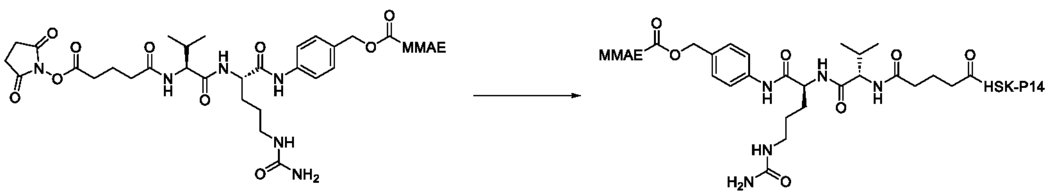

Intermediate 10 Compound 1

Intermediate 10 (10 mg, 7.5 μmol) and HSK-P14 (20 mg, 6.2 μmol) were added to DMA (1 mL) and then N,N-diisopropylethylamine (2.4 mg, 18.6 μmol) was added. The mixture was stirred at room temperature for 18 h. The reaction mixture was separated and purified by a liquid phase preparative column (liquid phase preparative conditions: C18 reverse phase preparative column; mobile phase: deionized water containing 0.1% trifluoroacetic acid (A) and acetonitrile containing 0.1% trifluoroacetic acid (B); gradient elution: B = 5%-70%; elution time: 15 min; flow rate: 12 mL/min; column temperature: 30°C, retention time: 4.56 min) to obtain compound 1 (10 mg, yield = 37%).
LCMS m/z =881.3 $[M/5+1]^+$, 1101.3 $[M/4+1]^+$, 1467.9 $[M/3+1]^+$.

## Example 2

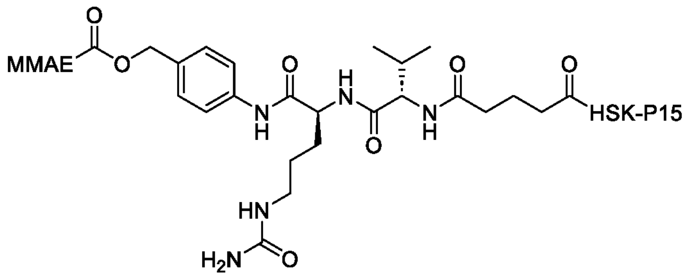

Compound 2

Intermediate 10 (10 mg, 7.5 μmol) and HSK-P15 (20 mg, 6.2 μmol) were added to DMA (1 mL) and then N,N-diisopropylethylamine (2.4 mg, 18.6 μmol) was added. The mixture was stirred at room temperature for 18 h. The reaction mixture was separated and purified by a liquid phase preparative column (liquid phase preparative conditions: C18 reverse phase preparative column; mobile phase: deionized water containing 0.1% trifluoroacetic acid (A) and acetonitrile containing 0.1% trifluoroacetic acid (B); gradient elution: B = 5%-70%; elution time: 15 min; flow rate: 12 mL/min; column temperature: 30°C, retention time: 4.66 min) to obtain compound 2 (10 mg).

LCMS m/z =884.2 $[M/5+1]^+$, 1104.8 $[M/4+1]^+$, 1472.5$[M/3+1]^+$.

**Example 3**

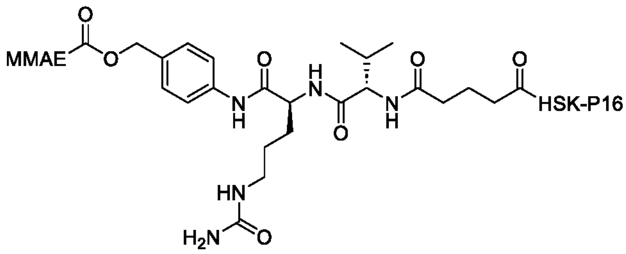

Compound 3

Reference can be made to Example 1 for the synthetic method:
Intermediate 10 (10 mg, 7.5 μmol) and HSK-P16 (20 mg, 6.2 μmol) were added to DMA (1 mL) and then N,N-diisopropylethylamine (2.4 mg, 18.6 μmol) was added. The mixture was stirred at room temperature for 18 h. The reaction mixture was separated and purified by a liquid phase preparative column to obtain compound 3 (10 mg).

LCMS m/z =884.2 $[M/5+1]^+$, 1104.8 $[M/4+1]^+$, 1472.8$[M/3+1]^+$.

**Example 4**

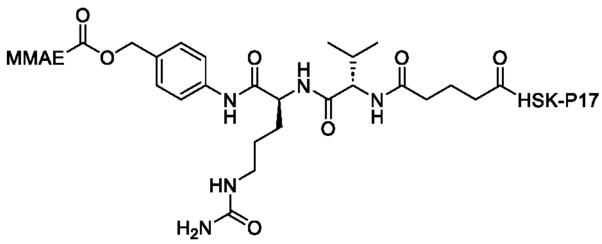

Compound 4

Intermediate 10 (10 mg, 7.5 μmol) and HSK-P17 (20 mg, 6.2 μmol) were added to DMA (1 mL) and then N,N-diisopropylethylamine (2.4 mg, 18.6 μmol) was added. The mixture was stirred at room temperature for 18 h. The reaction mixture was separated and purified by a liquid phase preparative column (liquid phase preparative conditions: C18 reverse phase preparative column; mobile phase: deionized water containing 0.1% trifluoroacetic acid (A) and acetonitrile containing 0.1% trifluoroacetic acid (B); gradient elution: B = 5%-70%; elution time: 15 min; flow rate: 12 mL/min; column temperature: 30°C, retention time: 4.54 min) to obtain compound 4 (10 mg).

LCMS m/z =884.2 $[M/5+1]^+$, 1104.8 $[M/4+1]^+$, 1472.6$[M/3+1]^+$.

**Example 5**

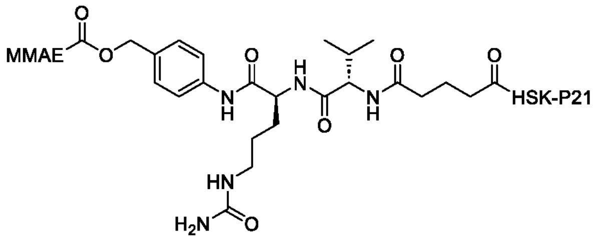

Compound 5

Reference can be made to Example 1 for the synthetic method:
Intermediate 10 (10 mg, 7.5 μmol) and HSK-P21 (20 mg, 6.2 μmol) were added to DMA (1 mL) and then N,N-diisopropylethylamine (2.4 mg, 18.6 μmol was added. The mixture was stirred at room temperature for 18 h. The reaction mixture was separated and purified by a liquid phase preparative column to obtain compound 5 (10 mg).

LCMS m/z =889.8 $[M/5+1]^+$, 1111.9 $[M/4+1]^+$, 1482.3$[M/3+1]^+$.

**Example 6**

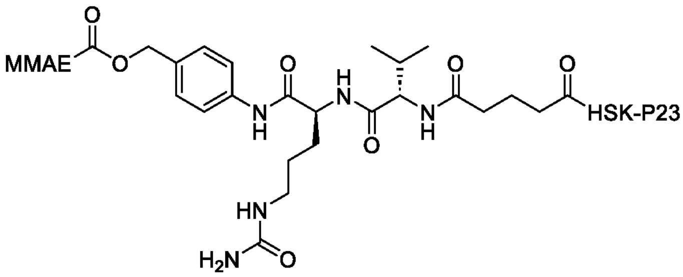

Compound 6

Reference can be made to Example 1 for the synthetic method:
Intermediate 10 (15 mg, 11.4 μmol and HSK-P23 (30 mg, 9.5 μmol were added to DMA (1 mL) and then N,N-diisopropylethylamine (3.7 mg, 28.5 μmol was added. The mixture was stirred at room temperature for 18 h. The reaction mixture was separated and purified by a liquid phase preparative column to obtain compound 6 (15 mg).

LCMS m/z =875.7 $[M/5+1]^+$, 1094.3 $[M/4+1]^+$, 1458.8 $[M/3+1]^+$.

**Example 7**

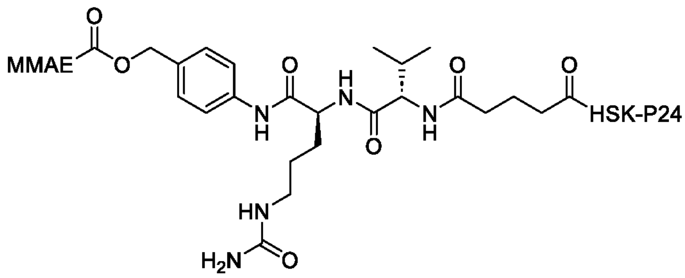

Compound 7

Reference can be made to Example 1 for the synthetic method:
Intermediate 10 (15 mg, 11.4 μmol and HSK-P24 (30 mg, 9.5 μmol were added to DMA (1 mL) and then N,N-diisopropylethylamine (3.7 mg, 28.5 μmol was added. The mixture was stirred at room temperature for 18 h. The reaction mixture was separated and purified by a liquid phase preparative column to obtain compound 7 (15 mg).

LCMS m/z =875.8 $[M/5+1]^+$, 1094.4 $[M/4+1]^+$, 1458.9 $[M/3+1]^+$.

**Example 8**

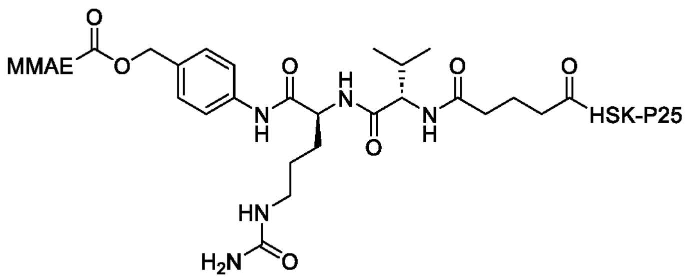

Compound 8

Reference can be made to Example 1 for the synthetic method:
Intermediate 10 (15 mg, 11.4 μmol and HSK-P25 (30 mg, 9.5 μmol were added to DMA (1 mL) and then N,N-diisopropylethylamine (3.7 mg, 28.5 μmol was added. The mixture was stirred at room temperature for 18 h. The reaction mixture was separated and purified by a liquid phase preparative column to obtain compound 8 (15 mg).

LCMS m/z =875.7 $[M/5+1]^+$, 1094.3 $[M/4+1]^+$, 1458.7 $[M/3+1]^+$.

**Example 9**

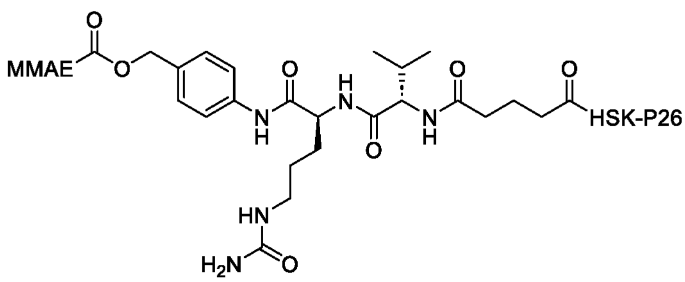

Compound 9

Reference can be made to Example 1 for the synthetic method:
Intermediate 10 (15 mg, 11.3 μmol and HSK-P26 (30 mg, 9.4 μmol were added to DMA (1 mL) and then N,N-diisopropylethylamine (3.7 mg, 28.5 μmol was added. The mixture was stirred at room temperature for 18 h. The reaction mixture was separated and purified by a liquid phase preparative column to obtain compound 9 (14 mg).
LCMS m/z =878.6 $[M/5+1]^+$, 1097.9 $[M/4+1]^+$, 1463.4 $[M/3+1]^+$.

**Example 10**

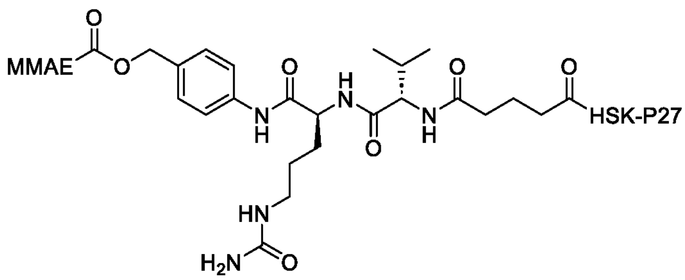

Compound 10

Reference can be made to Example 1 for the synthetic method:
Intermediate 10 (15 mg, 11.3 μmol and HSK-P27 (30 mg, 9.4 μmol were added to DMA (1 mL) and then N,N-diisopropylethylamine (3.7 mg, 28.5 μmol was added. The mixture was stirred at room temperature for 18 h. The reaction mixture was separated and purified by a liquid phase preparative column to obtain compound 10 (16 mg).
LCMS m/z =878.6 $[M/5+1]^+$, 1097.9 $[M/4+1]^+$, 1463.4 $[M/3+1]^+$.

**Example 11**

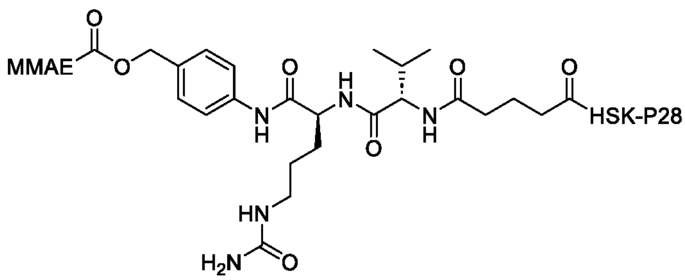

Compound 11

Reference can be made to Example 1 for the synthetic method:
Intermediate 10 (15 mg, 11.3 μmol and HSK-P28 (30 mg, 9.4 μmol were added to DMA (1 mL) and then N,N-diisopropylethylamine (3.7 mg, 28.5 μmol was added. The mixture was stirred at room temperature for 18 h. The reaction mixture was separated and purified by a liquid phase preparative column to obtain compound 11 (15 mg).
LCMS m/z =878.5 $[M/5+1]^+$, 1097.8 $[M/4+1]^+$, 1463.4 $[M/3+1]^+$.

**Example 12**

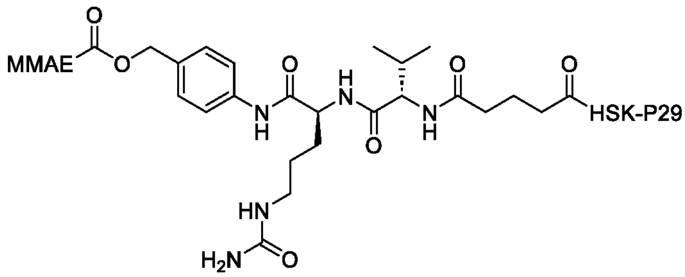

Compound 12

Reference can be made to Example 1 for the synthetic method: Intermediate 10 (15 mg, 11.2 μmol and HSK-P29 (30 mg, 9.3 μmol were added to DMA (1 mL) and then N,N-diisopropylethylamine (3.7 mg, 28.5 μmol was added. The mixture was stirred at room temperature for 18 h. The reaction mixture was separated and purified by a liquid phase preparative column to obtain compound 12 (14 mg).

LCMS m/z =887.0 $[M/5+1]^+$, 1108.4 $[M/4+1]^+$, 1477.4 $[M/3+1]^+$.

**Example 13**

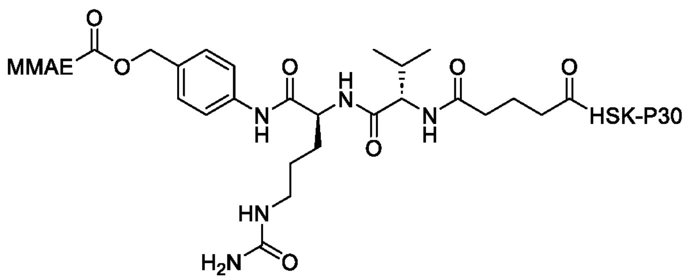

Compound 13

Intermediate 10 (15 mg, 11.2 μmol and HSK-P30 (30 mg, 9.3 μmol were added to DMA (1 mL) and then N,N-diisopropylethylamine (3.7 mg, 28.5 μmol was added. The mixture was stirred at room temperature for 18 h. The reaction mixture was separated and purified by a liquid phase preparative column (liquid phase preparative conditions: C18 reverse phase preparative column; mobile phase: deionized water containing 0.1% trifluoroacetic acid (A) and acetonitrile containing 0.1% trifluoroacetic acid (B); gradient elution: B = 5%-70%; elution time: 15 min; flow rate: 12 mL/min; column temperature: 30°C, retention time: 4.75 min) to obtain compound 13 (15 mg).

LCMS m/z =887.0 $[M/5+1]^+$, 1108.4 $[M/4+1]^+$, 1477.3 $[M/3+1]^+$.

**Example 14**

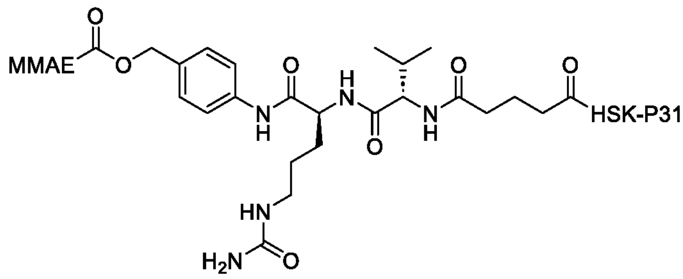

Compound 14

Reference can be made to Example 1 for the synthetic method:
Intermediate 10 (15 mg, 11.2 μmol and HSK-P31 (30 mg, 9.3 μmol were added to DMA (1 mL) and then N,N-diisopropylethylamine (3.7 mg, 28.5 μmol was added. The mixture was stirred at room temperature for 18 h. The reaction mixture was separated and purified by a liquid phase preparative column to obtain compound 14 (17 mg).
LCMS m/z =886.9 $[M/5+1]^+$, 1108.3 $[M/4+1]^+$, 1477.6 $[M/3+1]^+$.

**Example 15**

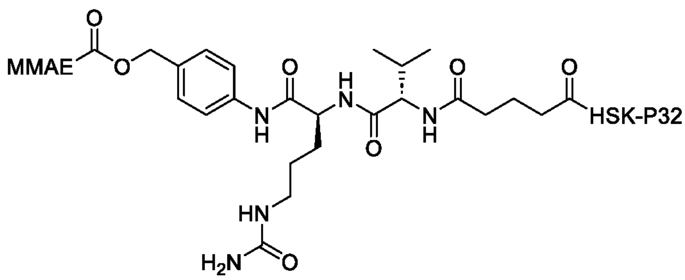

Compound 15

Reference can be made to Example 1 for the synthetic method:
Intermediate 10 (15 mg, 11.2 μmol and HSK-P32 (30 mg, 9.3 μmol were added to DMA (1 mL) and then N,N-diisopropylethylamine (3.7 mg, 28.5 μmol was added. The mixture was stirred at room temperature for 18 h. The reaction mixture was separated and purified by a liquid phase preparative column to obtain compound 15 (17 mg).
LCMS m/z =908.6 [M/5+1], 1135.5 [M/4+1],

**Example 16**

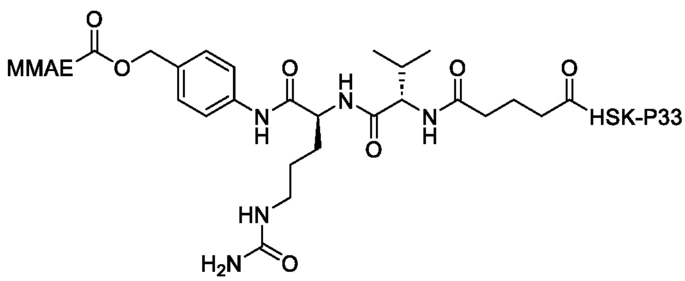

Compound 16

Reference can be made to Example 1 for the synthetic method: Intermediate 10 (15 mg, 11.2 μmol and HSK-P33 (30 mg, 9.3 μmol were added to DMA (1 mL) and then N,N-diisopropylethylamine (3.7 mg, 28.5 μmol was added. The mixture was stirred at room temperature for 18 h. The reaction mixture was separated and purified by a liquid phase preparative column to obtain compound 16 (15 mg).

LCMS m/z =900.2 $[M/5+1]^+$, 1125.1 $[M/4+1]^+$, 1499.7 $[M/3+1]^+$.

**Example 17**

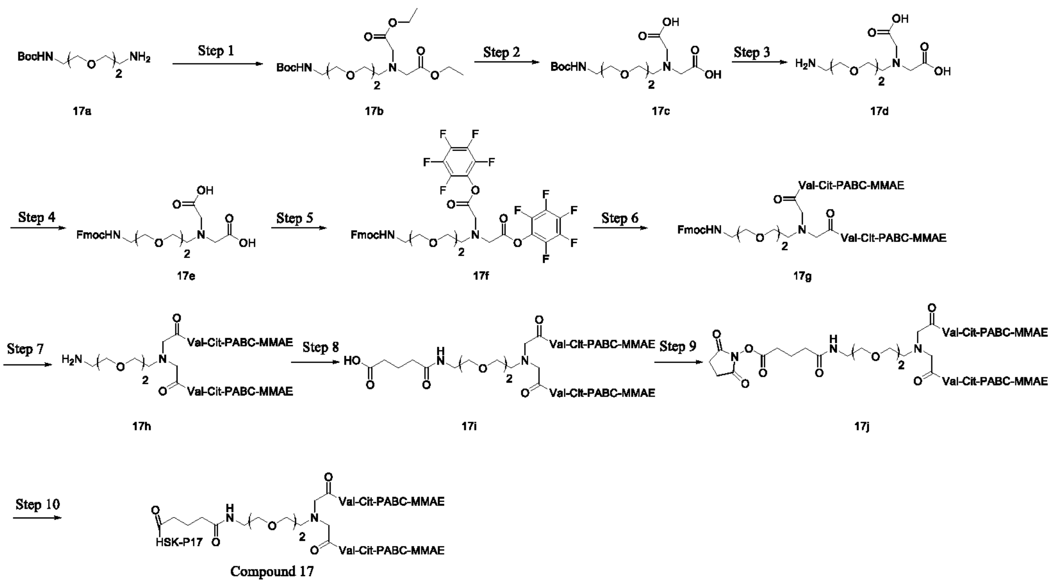


17a 17b 17c 17d 17e 17f 17g 17h 17i 17j

Compound 17

Step 1:

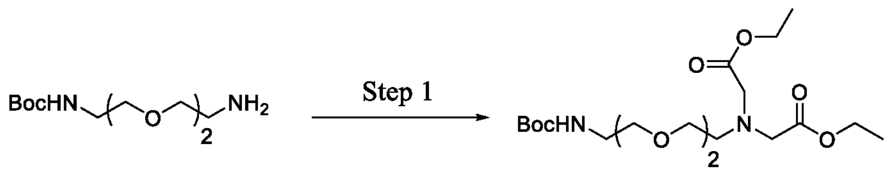


17a 17b

The known compound tert-butyl 2-(2-(2-aminoethoxy)ethoxy)ethylcarbamate (25.0 g, 0.1 mol), ethyl bromoacetate (43.2 g, 0.22 mol), and sodium carbonate (40.5 g, 0.25 mol) were sequentially added to acetonitrile (1.2 L) and the mixture was warmed to 50°C and stirred for 18 h. Then the mixture was cooled to room temperature and filtered and the mother liquor was concentrated to dryness to obtain crude **17b** (42 g, yield = 99%).

LCMS m/z =421.3 $[M+1]^+$.

Steps 2 and 3:

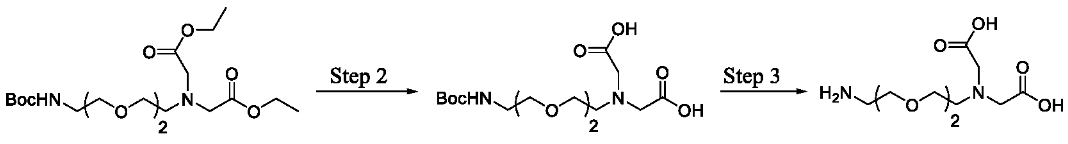


17b 17c 17d

Compound **17b** (42.0 g, 0.1 mol) was added to methanol (500 mL) and water (500 mL), then sodium hydroxide (40.3 g, 1.0 mol) was added and the mixture was stirred at room temperature for 4 h, adjusted to pH 1-2 with 6N hydrochloric acid and stirred for 0.5 h. The reaction solution was directly used in the next reaction.

LCMS m/z =265.2 $[M+1]^+$.

Step 4:

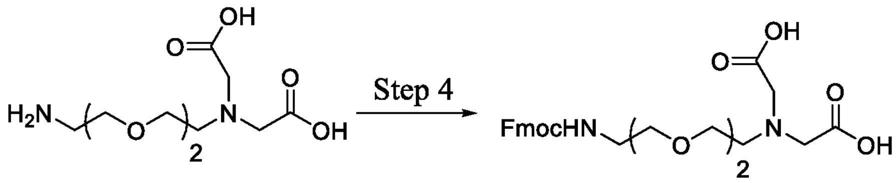


17d 17e

To the reaction solution of 17d were sequentially added 9-fluorenylmethyl-N-succinimidyl carbonate (34.0 g, 0.1 mol) and sodium bicarbonate (42.0 g, 0.5 mol). The mixture was stirred at room temperature for 4 h and purified by a C18 reverse phase column (0.1% TFA) ($H_2O$ : ACN = 60 : 40) to obtain **17e** (22 g, yield over two steps = 45.2%).

LCMS m/z =487.2 $[M+1]^+$.

Step 5:

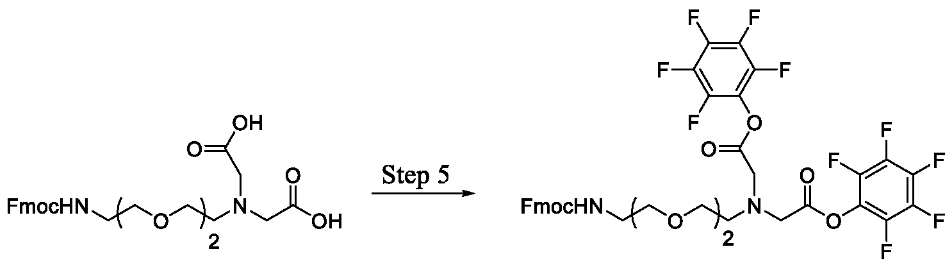


17e 17f

**17e** (5.0 g, 0.01 mol), pentafluorophenol (3.86 g, 0.02 mol) and N,N-diisopropylcarbodiimide (2.65 g, 0.02 mol) were sequentially added. The mixture was stirred at room temperature for 2 h and purified by column chromatography (PE : EA = 2 : 1) to obtain **17f** (4.0 g, yield = 47.6%).

LCMS m/z =819.1 $[M+1]^+$.

Step 6:

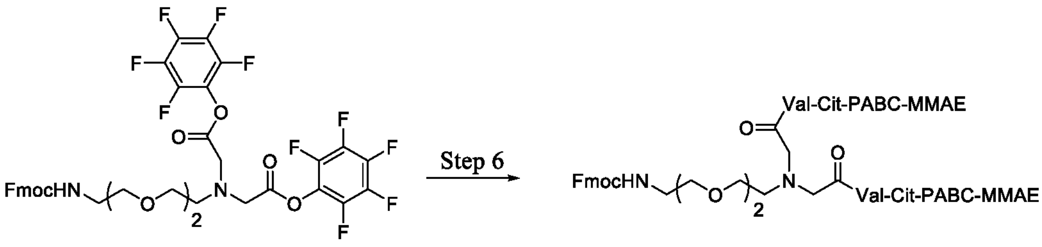


17f 17g

**17f** (140 mg, 0.17 mmol), intermediate 8 (390 mg, 0.34 mmol) and N,N-diisopropylethylamine (152 mg, 1.02 mmol) were sequentially added to DMF (5 mL). The mixture was stirred at room temperature for 12 h and purified by a Cis reverse phase column (0.1% TFA) ($H_2O$ : ACN = 40 : 60) to obtain **17g** (300 mg, yield = 65.1%).
LCMS m/z =899.8 $[M/3+1]^+$, 1349.5 $[M/2+1]^+$.

Step 7:

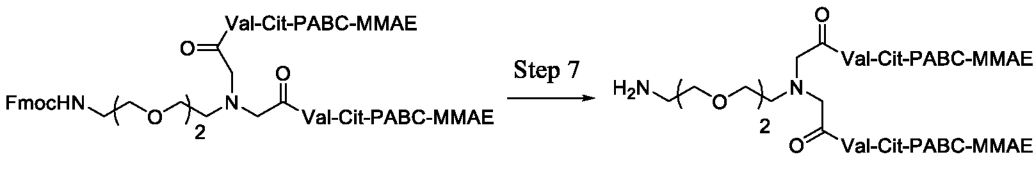


17g 17h

**17g** (300 mg, 0.11 mmol) was added to a mixed solution of piperidine : DMF = 1 : 4 (5 mL). The mixture was stirred at room temperature for 2 h and purified by a C18 reverse phase column (0.1% TFA) ($H_2O$ : ACN = 50 : 50) to obtain **17h** (200 mg, yield = 72.5%).
LCMS m/z =825.8 $[M/3+1]^+$, 1238.1 $[M/2+1]^+$.

Step 8:

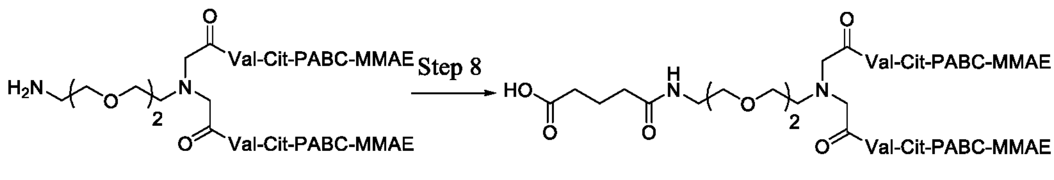


17h 17i

**17h** (200 mg, 0.08 mmol), glutaric anhydride (19 mg, 0.16 mmol) and N,N-diisopropylethylamine (21 mg, 0.16 mmol) were added to DMA (2 mL). The mixture was stirred at room temperature for 18 h and purified by a C18 reverse phase preparative column (0.1% TFA) ($H_2O$ : ACN = 50 : 50) to obtain **17i** (140 mg, yield = 66.9%).
LCMS m/z =863.8 $[M/3+1]^+$, 1295.3 $[M/2+1]^+$.

Step 9:

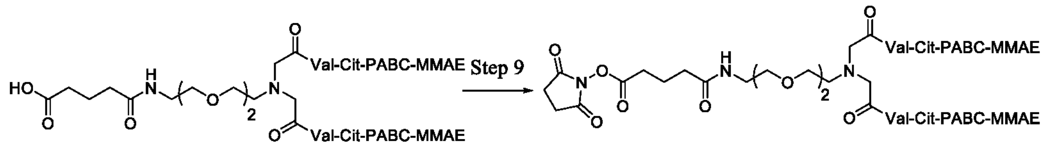


17i 17j

**17i** (46 mg, 0.017 mmol) was added to DMA (1 mL) and dichloromethane (0.3 mL). Under nitrogen protection, the mixture was cooled to 0°C and N-hydroxysuccinimide (6 mg, 0.051 mmol) and EDCI (10 mg, 0.051 mmol) were added. The resulting mixture was stirred at room temperature for 18 h and purified by a C18 reverse phase preparative column (0.1% TFA) ($H_2O$ : ACN = 50 : 50) to obtain **17j** (40 mg, yield = 83.8%).

LCMS m/z =896.3 $[M/3+1]^+$, $1343.6+1]^+$.

Step 10:

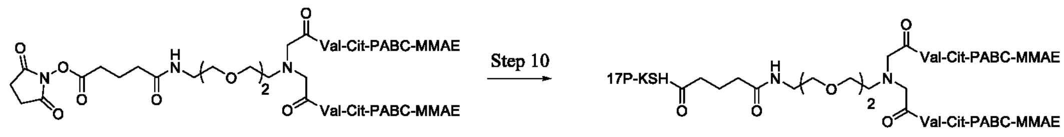


17j Compound 17

**17j** (40 mg, 15 μmol) and HSK-P17 (30 mg, 13 μmol) were added to DMA (1 mL) and then N,N-diisopropylethylamine (6 mg, 45 μmol) was added. The mixture was stirred at room temperature for 18 h. The reaction mixture was separated and purified by a liquid phase preparative column (liquid phase preparative conditions: C18 reverse phase preparative column; mobile phase: deionized water containing 0.1% trifluoroacetic acid (A) and acetonitrile containing 0.1% trifluoroacetic acid (B); gradient elution: B = 5%-70%; elution time: 15 min; flow rate: 12 mL/min; column temperature: 30°C, retention time: 5.32 min) to obtain compound 17 (20 mg, yield = 47%).

LCMS m/z =962.2 $[M/6+1]^+$, 1154.6 $[M/5+1]^+$, 1442.9 $[M/4+1]^+$.

**Example 18:**

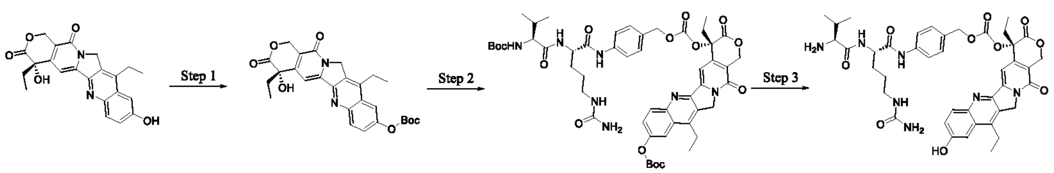


SN38 18a 18b 18c

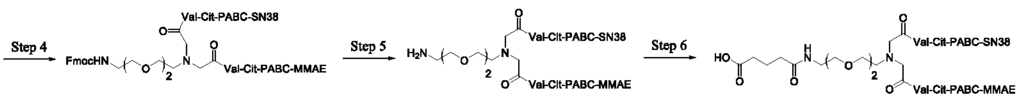


18d 18e 18f

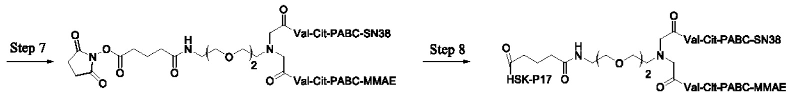


18g Compound 18

Step 1:

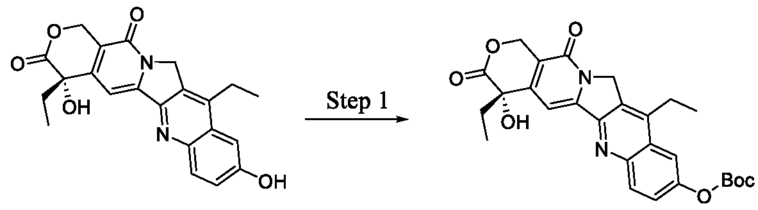


SN38 18a

The known compound SN38 (4.0 g, 0.01 mol) and di-tert-butyl dicarbonate (3.1 g, 0.013 mol) were sequentially added to dichloromethane (300 mL) and then pyridine (26.0 g, 0.3 mol) was added. The mixture was stirred at room temperature for 18 h and concentrated to dryness to obtain crude **18a** (5.0 g, yield = 99%).

LCMS m/z =493.1 $[M+1]^+$.

Step 2:

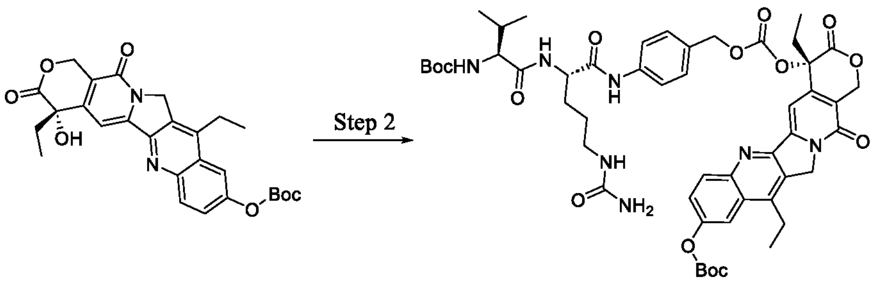


18a 18b

**18a** (2.0 g, 4 mmol), N,N-diisopropylethylamine (2.63 g, 20 mmol) and 4-dimethylaminopyridine (0.5 g, 4 mmol) were sequentially added to dry dichloromethane (40 mL). Under nitrogen protection, the mixture was cooled to 0°C and a solution of triphosgene (0.52 g, 1.72 mmol) in dichloromethane (10 mL) was slowly added dropwise. After the addition, the resulting mixture was stirred at 0°C for 5 minutes and stirred at room temperature for 10 minutes. Then a mixed solution of Boc-Val-Cit-PABC (1.75 g, 3.6 mmol) in dimethyl sulphoxide (10 mL) and dichloromethane (10 mL) was added dropwise. After the dropwise addition, the mixture was stirred for 2 hours, extracted with water (100 mL) and dichloromethane (50 mL), dried over anhydrous sodium sulphate, filtered and concentrated to dryness. The residue was purified by column chromatography (DCM : MeOH = 20 : 1) to obtain compound **18b** (1.4 g, yield = 35%).

LCMS m/z =998.4 $[M+1]^+$.

Step 3:

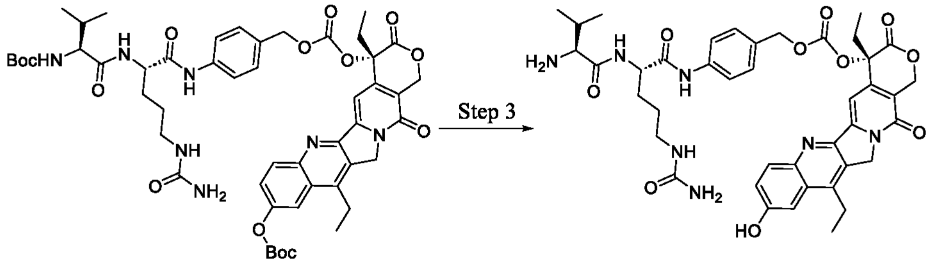


18b 18c

**18b** (1.26 g, 0.126 mmol) was added to a mixed solution of dichloromethane (13 mL) and trifluoroacetic acid (13 mL). The mixture was stirred at room temperature for 30 minutes and concentrated to dryness at 30°C to obtain crude **18c** (1.0 g, yield = 99%).

LCMS m/z =798.4 $[M+1]^+$.

Step 4:

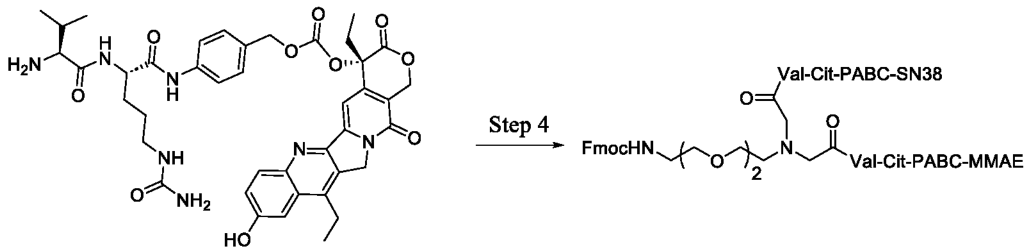


**18c** **18d**

**17f** (150 mg, 0.18 mmol) was added to N,N-dimethylformamide (2 mL) and then N,N-diisopropylethylamine (60 mg, 0.46 mmol) was added. A solution of Val-Cit-PABC-MMAE (200 mg, 0.16 mmol) in N,N-dimethylformamide (1 mL) was added dropwise and the mixture was stirred at room temperature for 30 minutes. Then N,N-diisopropylethylamine (60 mg, 0.46 mmol) was added and a solution of **18c** (165 mg, 0.18 mmol) in N,N-dimethylformamide (1 mL) was added dropwise. The resulting mixture was stirred at room temperature for 2 h and purified by a C18 reverse phase preparative column (0.1% TFA) ($H_2O$ : ACN = 35 : 65) to obtain **18d** (80 mg, yield = 23.2%).

LCMS m/z =1186.5 $[M/2+1]^+$.

Step 5:

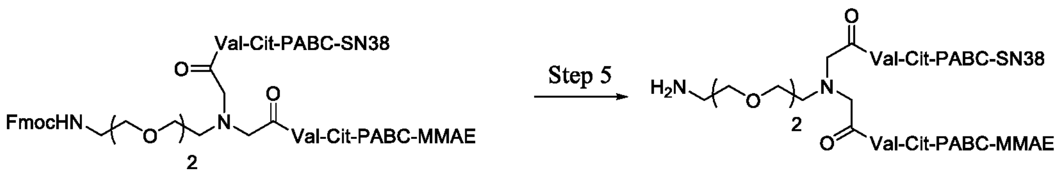


**18d** **18e**

**18d** (80 mg, 0.034 mmol) was added to a solution of 20% piperidine in N,N-dimethylformamide (1 mL). The mixture was stirred at room temperature for 1 minute and purified by a C18 reverse phase preparative column (0.1% TFA) ($H_2O$ : ACN = 50 : 50) to obtain **18e** (40 mg, yield = 55.5%).

LCMS m/z =1075.3 $[M/2+1]^+$.

Step 6:

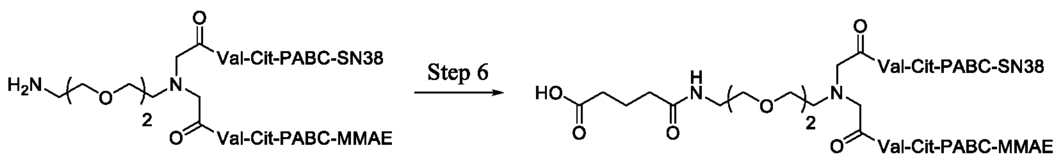


**18e** **18f**

**18e** (40 mg, 0.018 mmol) and glutaric anhydride (2.4 mg, 0.018 mmol) were added to N,N-dimethyl acetamide

(1 mL) solution. Then N,N-diisopropylethylamine (5 mg, 0.038 mmol) was added and the mixture was stirred at room temperature for 2 hours and purified by a C18 reverse phase preparative column (0.1% TFA) ($H_2O$ : ACN = 50 : 50) to obtain **18f** (40 mg, yield = 95%).

LCMS m/z =1132.7 $[M/2+1]^+$.

Step 7:

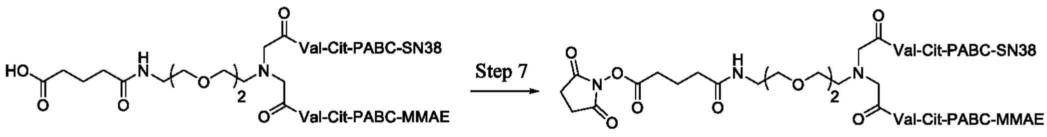


18f 18g

**18f** (40 mg, 0.018 mmol), N-hydroxysuccinimide (6 mg, 0.054 mmol) and EDCI (11 mg, 0.054 mmol) were sequentially added to a solution of N,N-dimethyl acetamide (1 mL) and dichloromethane (0.3 mL). Under nitrogen protection, the mixture was stirred at room temperature for 18 hours and concentrated at 20°C to remove dichloromethane. The residue was purified by a C18 reverse phase preparative column (0.1% TFA) ($H_2O$ : ACN = 45 : 55) to obtain **18g** (40 mg, yield = 96%).

LCMS m/z =1181.1 $[M/2+1]^+$.

Step 8:

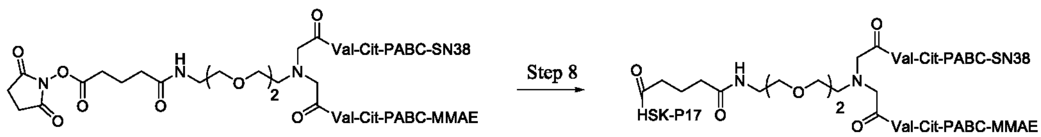


18g Compound 18

**18g** (20 mg, 8 μmol) and HSK-P17 (19 mg, 6 μmol) were sequentially added to DMA (1 mL) and then N,N-diisopropylethylamine (3 mg, 18 μmol) was added. The mixture was stirred at room temperature for 18 h. The reaction mixture was separated and purified by a liquid phase preparative column (liquid phase preparative conditions: C18 reverse phase preparative column; mobile phase: deionized water containing 0.1% trifluoroacetic acid (A) and acetonitrile containing 0.1% trifluoroacetic acid (B); gradient elution: B = 5%-70%; elution time: 15 min; flow rate: 12 mL/min; column temperature: 30°C, retention time: 5.54 min) to obtain compound 18 (5 mg, yield = 15%).

LCMS m/z =908.0 $[M/6+1]^+$, 1089.3 $[M/5+1]^+$, 1361.4 $[M/4+1]^+$.

**Example 19**

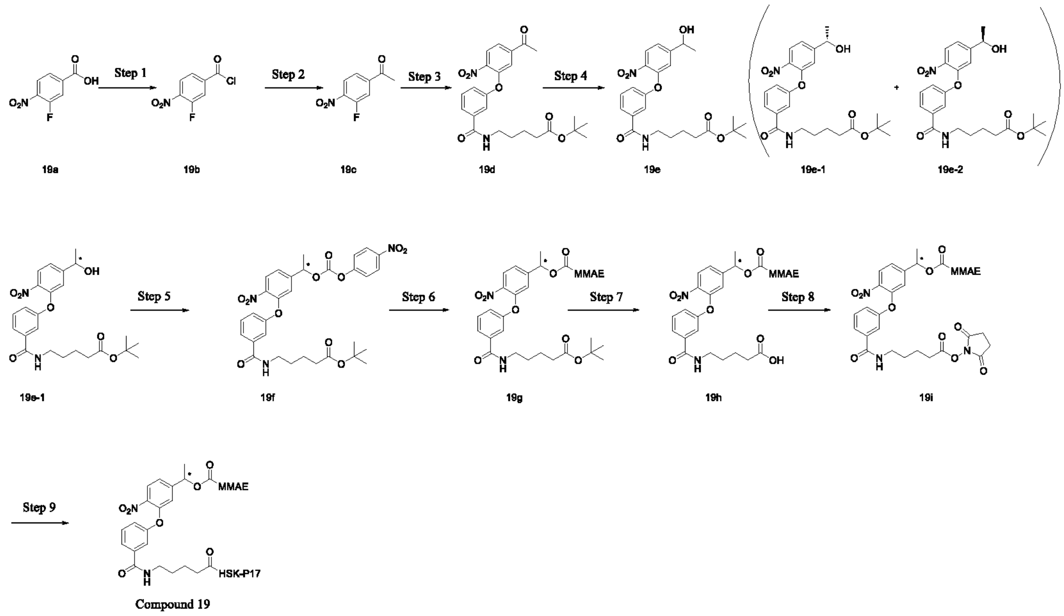


19a 19b 19c 19d 19e 19e-1 19e-2

19e-1 19f 19g 19h 19i

Compound 19

Step 1:

**19a** (20 g, 108.05 mmol) was added to thionyl chloride (80 mL) and then DMF (1 ml) was added. The mixture was reacted at 90°C for 3 h. The reaction mixture was concentrated under reduced pressure to remove $SOCl_2$, and the residue was diluted with toluene (40 ml) and then directly used in the next reaction.

Step 2:

To a single-neck bottle (500 ml) were sequentially added triethylamine (21.8 g, 216.1 mmol), 1,3-dimethyl malonate (17.13 g, 129.70 mmol) and magnesium chloride (7.20 g, 75.54 mmol). The mixture was stirred at room temperature for 1.5 h (the system solidified during stirring; a small amount of toluene was added to facilitate stirring). A solution of **19b** in toluene was slowly added dropwise, and after the addition, the mixture was stirred at room temperature overnight. The resulting reaction mixture was washed with water (200 ml) and extracted with ethyl acetate (100 ml×3). The organic phase was washed with dilute aqueous hydrochloric acid solution (4 mol/L). Liquid separation was carried out and the organic phase was concentrated under reduced pressure to dryness. Then 6N hydrochloric acid (200 ml) was added and the mixture was refluxed overnight. The mixture was cooled to room temperature and extracted with ethyl acetate (100 ml×3). The organic phase was concentrated under reduced pressure and the residue was separated and purified by silica gel column chromatography (EA : PE = 1/20) to obtain **19c** (14 g, yield over two steps: 70%).

LCMS m/z =184.1 $[M+1]^+$.

Step 3:

To a single-neck bottle (100 ml) were added **19c** (1.1 g, 6.01 mmol), intermediate 13 (1.76 g, 6.01 mmol), acetonitrile (20 mL) and potassium carbonate (1.66 g, 12.01 mmol). The mixture was reacted at 80°C for 5 h. As monitored by LC-MS, the reaction was completed. The mixture was cooled to room temperature and filtered. The solid was washed with a small amount of ethyl acetate. The filtrate was directly concentrated under reduced pressure and the residue was separated and purified by silica gel column chromatography (EA : PE = 1 : 3) to obtain **19d** (1.8 g, 65.61%).

LCMS m/z =401.1 $[M- tBu +1]^+$.

Step 4:

To a single-neck bottle (100 ml) were added **19d** (1.8 g, 3.94 mmol) and methanol (20 mL). At 0°C, sodium borohydride (0.18 g, 4.73 mmol) was added in portions. The mixture was reacted for 30 min while the temperature was maintained at 0°C. As monitored by TLC, the reaction was completed. Water (50 ml) was added and the resulting mixture was extracted with ethyl acetate (30 ml×3). The organic phases were combined, washed with saturated sodium chloride solution, dried over anhydrous sodium sulphate and filtered. The filtrate was concentrated under reduced pressure to obtain **19e** (1.8 g, 99.64%).

**19e** (1.9 g, 4.14 mmol) was subjected to chiral preparation to obtain **19e-1** (0.64 g, 35.5%, retention time: 1.878 min) and 19e-2 (0.6g, 33.3%, retention time: 1.965 min). Chiral preparation method: instrument: Waters 150 MGM; chromatographic column: cellulose-2 Column (250 × 30 mm, I.D. 30 mm, 10 um particle size); mobile phase: A: carbon dioxide, and B: IPA; isocratic elution: 20% mobile phase B; flow rate: 100 mL/min; back pressure: 100 bar; column temperature: 25°C; wavelength: 220 nm; elution time: 5 min.

LCMS m/z =403.1 $[M\text{-}^{t}Bu+1]^+$.

Step 5:

**19e-1** (0.60 g, 1.31 mmol), bis(p-nitrophenyl)carbonate (0.40 g, 1.31 mmol) and triethylamine (0.13 g, 1.31 mmol) were added to dichloromethane (10 mL). The mixture was reacted at room temperature for 16 h. The reaction solution was directly concentrated under reduced pressure and the residue was separated and purified by silica gel column chromatography (EA/PE = 0-60%) to obtain **19f** (0.65 g, 79.57%).

LCMS m/z =568.2 $[M\text{-}tBu+1]^+$.

Step 6:

**19f** (0.6 g, 0.96 mmol), MMAE (0.69 g, 0.96 mmol), 1-hydroxybenzotriazole (0.13 g, 0.96 mmol) and N,N-diisopropylethylamine (0.62 g, 4.83 mmol) were sequentially added to DMF (10 mL). The mixture was reacted at room temperature for 16 h. As monitored by LC-MS, the reaction was completed. The resulting mixture was separated and purified by a C18 reverse phase column (composition of mobile phases A and B: mobile phase A: acetonitrile; mobile phase B: water (containing 0.1% TFA), (A/B = 60%/40%)) to obtain the title compound (0.8 g, 69%).

LCMS m/z =1202.8 $[M+1]^+$.

Step 7:

**19g** (0.8 g, 0.67 mmol) was added to dichloromethane (20 mL) and then trifluoroacetic acid (2 ml) was added. The mixture was stirred at room temperature for 4 h. As monitored by LC-MS, the reaction was completed. The mixture was concentrated under reduced pressure at room temperature to remove the solvent. Then tetrahydrofuran (20 ml) and potassium carbonate (0.93 g, 6.73 mmol) were sequentially added and the mixture was stirred at room temperature for 3 h. After tetrahydrofuran was removed by concentration under reduced pressure, water (10 ml) and methanol (10 ml) were added. The mixture was separated and purified by a C18 reverse phase column (composition of mobile phases A and B, mobile phase A: acetonitrile; mobile phase B: water (containing 0.1% TFA), (A/B = 60%/40%)) to obtain the title compound (0.45 g, 58%).

LCMS m/z =1147.4 $[M+1]^+$.

Step 8:

**19h** (0.30 g, 0.26 mmol), N-hydroxysuccinimide (0.089 g, 0.77 mmol) and 1-(3-dimethylaminopropyl)-3-ethylcarbodiimide hydrochloride (0.15 g, 0.78 mmol) were sequentially added to a mixed system of DMA (5 ml) and dichloromethane (1 ml). The reaction mixture was reacted at room temperature overnight. The reaction was monitored by LC-MS. Dichloromethane was removed by concentration under reduced pressure. The mixture was separated and purified by a C18 reverse phase column (composition of mobile phases A and B, mobile phase A: acetonitrile; mobile phase B: water (containing 0.1% TFA), (A/B = 65%/35%)) to obtain **19i** (0.2 g, 62%).

LCMS m/z =1243.7 $[M+1]^+$.

Step 9:

**19i** (14 mg, 0.011 mmol), HSK-P17 (30 mg, 0.0094 mmol) and N,N-diisopropylethylamine (7.11 mg, 0.055 mmol) were added to DMF (2 mL). The mixture was reacted at room temperature for 3 h. As monitored by LC-MS, the reaction was completed. The reaction solution was directly purified by HPLC to obtain compound 19 (30 mg, 63.09%). Preparative HPLC separation method: 1. Instrument: waters 2767 (preparative liquid phase chromatographic instrument); chromatographic column: SunFire@ Prep C18 (19 mm × 250 mm) 2. The sample was dissolved in DMF and filtered with a 0.45 μm filter to prepare a sample solution. 3. Preparative chromatographic conditions: a. composition of mobile phases A and B: mobile phase A: acetonitrile; mobile phase B: water (containing 0.1% TFA); b. gradient elution: mobile phase A: 5%-70%; c. flow rate: 12 mL/min. d. elution time: 20 min.

LCMS m/z =1082.1 $[M/4+1]^+$, 1442.4 $[M/3+1]^+$.

**Example 20**

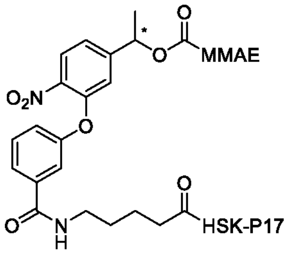

Compound 20

Reference can be made to Example 19 for the synthetic method: Compound 19e-2 was used as raw material and compound 20 (28 mg, 58.89%) was obtained after reaction and purification.

Preparative HPLC separation method: 1. Instrument: waters 2767 (preparative liquid phase chromatographic instrument); chromatographic column: SunFire@ Prep C18 (19 mm×250 mm) 2. The sample was dissolved in DMF and filtered with a 0.45 μm filter to prepare a sample solution. 3. Preparative chromatographic conditions: a. composition of mobile phases A and B: mobile phase A: acetonitrile; mobile phase B: water (containing 0.1% TFA); b. gradient elution: mobile phase A: 5%-70%; c. flow rate: 12 mL/min. d. elution time: 20 min.

LCMS m/z =1082.1 $[M/4+1]^+$, 1442.4 $[M/3+1]^+$.

## Biological test examples

### 1. PC3 cell proliferation inhibition assay

PC3 cell culture conditions: RPMI-1640 + 10% FBS + 1% penicillin-streptomycin solution. The cells were cultured in a 37°C, 5% $CO_2$ incubator. On the first day, the PC3 cells in the exponential growth phase were collected and plated in a 96-well cell culture plate at a density of 500 cells/well (90 μL/well). The cells were cultured overnight in a 37°C, 5% $CO_2$ incubator. The next day, 10 μL of the compound at different concentrations was added to each well so that the final concentration of DMSO in each well was 0.1%. The mixture was cultured in a 37°C, 5% $CO_2$ incubator for 3 days. After the completion of culture, 50 μL of detection solution (Cell Viability Assay, Promega, G7573) was added to each well. The mixture was mixed uniformly for 2 minutes and incubated at room temperature for 10 minutes. The chemiluminescence reading was detected by a microplate reader. The results were processed according to formula (1), the proliferation inhibition rate of the compound at different concentrations was calculated, and the $IC_{50}$ value of the compound with an inhibition rate of 50% was calculated using origin9.2 software, wherein $RLU_{compound}$ was the readout of the treated group, and RLU control was the average value of the solvent control group.

$$\text{Inhibition \%} = (1-(RLU_{compound}/RLU_{control})\times 100\% \quad \text{formula (1)}$$

The test results of some examples can be seen in Table 1.

**Table 1 Inhibitory activity of the compounds on the proliferation of PC3 cells**

| Compound | $IC_{50}$ (nM) | Max inh.% 10 μM |
|---|---|---|
| Compound 2 | A | 75.0 |
| Compound 4 | A | 73.5 |
| Compound 8 | B | 77.8 |
| Compound 9 | B | 71.7 |
| Compound 13 | B | 72.9 |
| Compound 17 | A | 69.4 |

(continued)

| Compound | $IC_{50}$ (nM) | Max inh.% 10 μM |
|---|---|---|
| Compound 18 | B | 92.7 |
| Notes: A ≤ 10 nM, 10 nM < B ≤ 50 nM, 50 nM < C ≤ 100 nM. | | |

Conclusion: the compounds of the present invention had a very excellent inhibitory activity with IC50 values of less than 100 nM, wherein compounds having some structures had IC50 values of less than 50 nM or even 10 nM; some compounds having excellent structures had IC50 values of less than 5 nM; and some compounds having more excellent structures had IC50 values of less than 2 nM. For example, compounds 2, 4 and 17 had $IC_{50}$ values of 3.1 nM, 2.9 nM and 1 nM, respectively. Control compound BT5528 had an $IC_{50}$ value of 7.4 nM.

**2. MOLP8 cell proliferation inhibition assay**

Human multiple myeloma MOLP8 cells purchased from DSMZ were placed in complete RPMI-1640 medium (supplemented with 20% foetal bovine serum) and cultured at 37°C and 5% $CO_2$. The cells in the exponential growth phase were collected, and the cell suspension was adjusted to 8000 cells/135 μL with the culture medium. 135 μL of the cell suspension was added to each well of a 96-well cell culture plate and incubated overnight. The next day, compounds at different concentrations were added, and the plate was placed in the incubator and incubated for 5 days. After the completion of culture, according to operation instructions for a CellTiter-Glo kit (Promega, Cat# G7573), 75 μL of CTG solution, which was already pre-melted and equilibrated to room temperature, was added to each well, and the mixture was uniformly mixed for 2 minutes using a microplate shaker. The plate was placed at room temperature for 10 minutes, and then fluorescence signal values were measured using an Envision 2104 plate reader (PerkinElmer). The cell proliferation inhibition rate was calculated according to the formula [(1- ($RLU_{compound}$ - $RLU_{blank}$)/($RLU_{control}$ - $RLU_{blank}$))×100%]. The $IC_{50}$ value was obtained by four-parameter nonlinear fitting using GraphPad Prism software. The test results of some examples can be seen in Table 2.

**Table 2 Inhibitory activity of the compound on MOLP8 cells**

| Compound | $IC_{50}$ (nM) | Max inh.% 10 μM | Compound 4 $IC_{50}$/MMAE $IC_{50}$ |
|---|---|---|---|
| Compound 4 | 143.6 | 99.1 | 120 |

Conclusion: the activity of compound 4 of the present invention on negative cell MOLP8 was much weaker than its activity on positive cell PC3, indicating that the compound had good cell selectivity.

**3. Pharmacokinetic test in rats**

**3.1 Experimental animals:** male SD rats, about 220 g, 6-8 weeks old, 3 rats/compound. Purchased from Chengdu Ddossy Experimental Animals Co., Ltd.

**3.2 Experimental design:** on the day of the test, 3 SD rats were randomly grouped according to their body weights. The animals were fasted with water available for 12 to 14 h one day before the administration, and were fed 4 h after the administration.

**Table 3. Administration information**

| Group | Quantity | Administration information | | | | | |
|---|---|---|---|---|---|---|---|
| | Male | Test compound | Administration dosage (mg/kg) | Administration concentration (mg/mL) | Administration volume (mL/kg) | Collected samples | Mode of administration |
| G1 | 3 | Compound 4 | 1 | 0.5 | 2 | Plasma | Intravenously |
| Notes: 5% DMA+95% (20% SBE-CD) | | | | | | | |

Before and after the administration, 0.15 ml of blood was taken from the orbit of the animals under isoflurane

anaesthesia and placed in an EDTAK2 centrifuge tube. The blood was centrifuged at 5000 rpm and 4°C for 10 min to collect plasma. The blood collection time points for the intravenous administration group and intragastric administration group were: 0, 5 min, 15 min, 30 min, 1 h, 2 h, 4 h, 6 h, 8 h and 24 h. Before analysis and detection, all samples were stored at -80°C, and a quantitative analysis of samples was performed using LC-MS/MS.

**Table 4. Pharmacokinetic parameters of test compound in plasma of rats**

| Test compound | Mode of administrati on | Compoun d | Cmax (ng/mL) | CL (mL/ minlk g) | $Vd_{ss}$ (L/kg) | $AUC_{0-t}$ (hr*ng/m L) | $T_{1/2}$ (h) |
|---|---|---|---|---|---|---|---|
| Compound 4 | i.v. (1 mg/kg) | PDC | 6531±106 3 | 11.0±1.5 | 0.233±0.0 65 | 1472±224 | 0.272±0 . 037 |
| Conclusion: compound 4 had good pharmacokinetic characteristics in rats. | | | | | | | |

**4. Pharmacokinetic test in monkeys**

**4.1 Experimental animals:** male cynomolgus monkeys, 3-5 kg, 3-6 years old, 3 monkeys/compound. Purchased from Suzhou Xishan Biotechnology Inc.

**4.2 Experimental method:** on the day of the test, 3 monkeys were randomly grouped according to their body weights. The animals were fasted with water available for 14 to 18 h one day before the administration, and were fed 4 h after the administration.

**Table 5. Administration information**

| Group | Quantity | Administration information | | | | | |
|---|---|---|---|---|---|---|---|
| | Male | Test compound | Administration dosage (mg/kg) | Administration concentration (mg/mL) | Administration volume (mL/kg) | Collected samples | Mode of administration |
| G1 | 3 | Compound 4 | 1 | 1 | 1 | Plasma | Intravenously |

Solvent for intravenous administration: 5% DMA+95% (20% SBE-CD).

Before and after the administration, 1.0 mL of blood samples were drawn from the limb veins and placed in an EDTAK2 centrifuge tube. Centrifugation was carried out at 5000 rpm at 4°C for 10 min, and the plasma was collected. The blood collection time points for the intravenous administration group were: 0, 2 min, 5 min, 15 min, 30 min, 1 h, 2 h, 4 h, 6 h, 8 h, and 24 h. Before analysis and detection, all samples were stored at -80°C, and a quantitative analysis of samples was performed using LC-MS/MS.

**Table 6. Pharmacokinetic parameters of test compound in plasma of monkeys**

| Test Compound | Mode of administration | Compound | Cmax (ng/mL) | CL (mL/min/kg) | $Vd_{ss}$ (L/kg) | $AUC_{0-t}$ (hr*ng/mL) | $T_{1/2}$ (h) | $AUC_{MMAF}$/ $AUC_{compound\ 4}$ |
|---|---|---|---|---|---|---|---|---|
| Compound 4 | i.v. (1 mg/kg) | PDC | 7634±72 | 5.57±1.1 | 3.179±0.0055 | 3026±573 | 0.643±0.1 7 | 0.00686 |
| | | MMAE | 3.71±0.16 | NC | NC | 20.8±0.57 | 24.3±1.8 | |

NC: unable to calculate;
Conclusion: compound 4 had good pharmacokinetic characteristics in monkeys.

### 5. Mouse PC3 cells subcutaneous in vivo transplanted tumour model

Human prostate cancer PC3 cells were placed in an RPMI-1640 medium (supplemented with 10% foetal bovine serum and 1% penicillin-streptomycin solution) and cultured at 37°C. Conventional digestion treatment with trypsin was carried out twice a week for passage. When the cell saturation was 80%-90%, and the number reached the requirement, the cells were collected, counted, and inoculated. Male CB-17 SCID mice (from Beijing Vital River Laboratory Animal Technology Co., Ltd.) were subcutaneously inoculated with 0.1 mL ($2 \times 10^6$) of PC3 cells (plus matrigel, with the volume ratio of 1 : 1) on the right back. When the average tumour volume reached about 80 - 120 $mm^3$, grouping and administration were performed (marked as Day 0). For the solvent group, 50 mM of acetic acid and 10% sucrose solution (pH = 5) were administered; and for the administration group, 0.5 mg/kg of compound 2, compound 4 or compound 13 was intravenously administered. The administration frequency was once a week and the administration cycle was 35 days (Day 0-Day 34). After grouping, the tumour diameter was measured twice a week with a vernier calliper. The formula for calculating the tumour volume was: $V = 0.5 \times a \times b^2$, where a and b represented the long and short diameters of the tumour, respectively. The tumour inhibitory effect of the compound was evaluated by TGI (%) = [1- (average tumour volume at the end of administration in the treatment group - average tumour volume at the beginning of administration in the treatment group)/(average tumour volume at the end of treatment in the solvent control group - average tumour volume at the beginning of treatment in the solvent control group)] $\times$ 100%. The tumour growth curve and the animal body weight change curve were as shown in Figure 1 and Figure 2, respectively.

Test results: after 35 days (administration frequency: once a week), the TGIs of the groups administered with compound 2, compound 4 and compound 13 were 90%, 92% and 88%, respectively; the maximum body weight losses of the animals in all administration groups were obviously lower than that of the solvent group.

Conclusion: in the mouse PC3 subcutaneous in vivo transplanted tumour model, compound 2, compound 4 and compound 13 of the present invention had good efficacy in inhibiting tumour growth, and were well tolerated.

## Claims

1. A peptide ligand specific for EphA2, comprising a polypeptide and a non-aromatic molecular scaffold, **characterised in that** the polypeptide comprises at least three residues independently selected from Cys and Hcys, and wherein at least one of the residues is a Hcys residue, the three Cys and Hcys residues are separated by two loop sequences, and the Cys or Hcys residue of the polypeptide is connected to the scaffold via a thioether bond, thereby forming two polypeptide loops on the scaffold.

2. The peptide ligand according to claim 1, **characterised in that** the peptide ligand comprises the following amino acid sequence:
   Xa1-A1- Xa2-A2- Xa3

   wherein A1 and A2 represent the amino acid residue sequences between Xa1, Xa2 and Xa3, and A1 and A2 each independently comprise 5 or 6 amino acid residues;
   Xa1, Xa2 and Xa3 are independently a Cys or Hcys residue, and at least one of Xa1, Xa2 and Xa3 is a Hcys residue, and the non-aromatic molecular scaffold and Xa1, Xa2 and Xa3 of the polypeptide form a thioether bond, thereby forming two polypeptide loops on the molecular scaffold.

3. The peptide ligand according to claim 2, **characterised in that** the polypeptide ligand comprises the amino acid sequence as shown below:
   Xa1-Hyp-Leu-Val-Asn-Pro-Leu-Xa2-Leu-His-Pro-(D-Asp)-Trp-(hArg)-Xa3.

4. The peptide ligand according to claim 3, **characterised in that** the polypeptide comprises an amino acid sequence as shown in any one of SEQ ID NO:1-SEQ ID NO:7:

   Hcys-Hyp-Leu-Val-Asn-Pro-Leu-Hcys-Leu-His-Pro-(D-Asp)-Trp-(hArg)-Hcys (SEQ ID NO: 1);
   Cys-Hyp-Leu-Val-Asn-Pro-Leu-Cys-Leu-His-Pro-(D-Asp)-Trp-(hArg)-Hcys (SEQ ID NO:2);
   Cys-Hyp-Leu-Val-Asn-Pro-Leu-Hcys-Leu-His-Pro-(D-Asp)-Trp-(hArg)-Cys (SEQ ID NO:3);
   Hcys-Hyp-Leu-Val-Asn-Pro-Leu-Cys-Leu-His-Pro-(D-Asp)-Trp-(hArg)-Cys (SEQ ID NO:4);
   Cys-Hyp-Leu-Val-Asn-Pro-Leu-Hcys-Leu-His-Pro-(D-Asp)-Trp-(hArg)-Hcys (SEQ ID NO:5);
   Hcys-Hyp-Leu-Val-Asn-Pro-Leu-Cys-Leu-His-Pro-(D-Asp)-Trp-(hArg)-Hcys (SEQ ID NO:6);
   Hcys-Hyp-Leu-Val-Asn-Pro-Leu-Hcys-Leu-His-Pro-(D-Asp)-Trp-(hArg)-Cys (SEQ ID NO:7).

**5.** The peptide ligand according to claim 4, **characterised in that** the polypeptide sequence of the peptide ligand is selected from one of SEQ ID NO:8-SEQ ID NO:14:

(β-Ala)-$Sar_{10}$-Ala-hArg-Asp-Hcys-Hyp-Leu-Val-Asn-Pro-Leu-Hcys-Leu-His-Pro-(D-Asp)-Trp-(hArg)-Hcys (SEQ ID NO:8);
(β-Ala)-$Sar_{10}$-Ala-hArg-Asp-Cys-Hyp-Leu-Val-Asn-Pro-Leu-Cys-Leu-His-Pro-(D-Asp)-Trp-(hArg)-Hcys (SEQ ID NO:9);
(β-Ala)-$Sar_{10}$-Ala-hArg-Asp-Cys-Hyp-Leu-Val-Asn-Pro-Leu-Hcys-Leu-His-Pro-(D-Asp)-Trp-(hArg)-Cys (SEQ ID NO:10);
(β-Ala)-$Sar_{10}$-Ala-hArg-Asp-Hcys-Hyp-Leu-Val-Asn-Pro-Leu-Cys-Leu-His-Pro-(D-Asp)-Trp-(hArg)-Cys (SEQ ID NO:11);
(β-Ala)-$Sar_{10}$-Ala-hArg-Asp-Cys-Hyp-Leu-Val-Asn-Pro-Leu-Hcys-Leu-His-Pro-(D-Asp)-Trp-(hArg)-Hcys (SEQ ID NO:12);
(β-Ala)-$Sar_{10}$-Ala-hArg-Asp-Hcys-Hyp-Leu-Val-Asn-Pro-Leu-Cys-Leu-His-Pro-(D-Asp)-Trp-(hArg)-Hcys (SEQ ID NO:13);
(β-Ala)-$Sar_{10}$-Ala-hArg-Asp-Hcys-Hyp-Leu-Val-Asn-Pro-Leu-Hcys-Leu-His-Pro-(D-Asp)-Trp-(hArg)-Cys (SEQ ID NO:14).

**6.** The peptide ligand according to claim 4, **characterised in that** the peptide ligand is a bicyclic peptide containing one of the following structures:

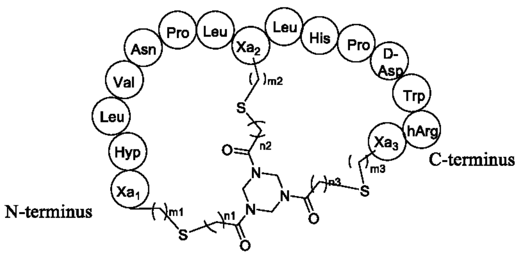

I-1

,

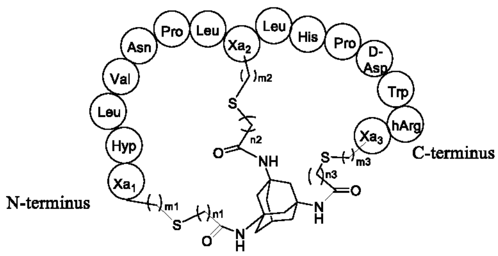

II-1

wherein, when Xa1, Xa2 or Xa3 is Cys, correspondingly, m1, m2 or m3 is 1; when Xa1, Xa2 or Xa3 is Hcys, correspondingly, m1, m2 or m3 is 2;
n1, n2 and n3 are independently 1 or 2.

**7.** The peptide ligand according to claim 6, **characterised in that** the peptide ligand is a bicyclic peptide having one of the following structures:

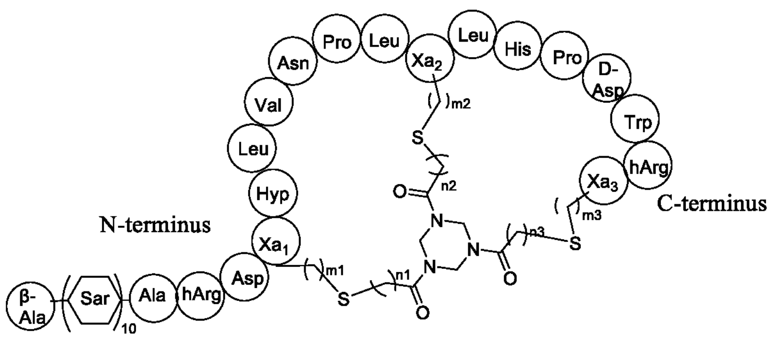

,

I-2

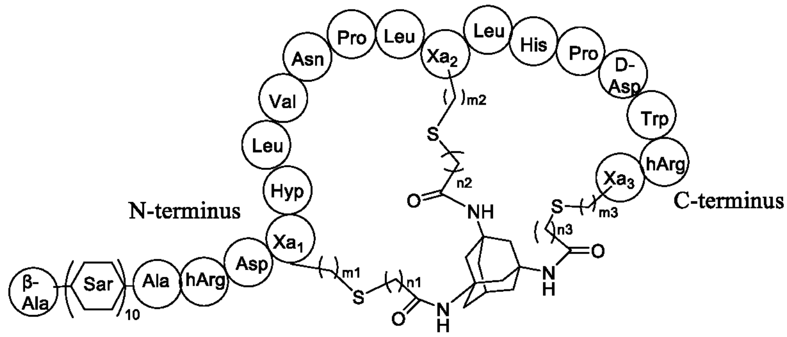

II-2.

8. The peptide ligand according to claim 7, **characterised in that**

(1) Xa1 is Hcys, m1 is 2, and n1 is 1 or 2; or
(2) Xa2 is Hcys, m2 is 2, and n2 is 1 or 2; or
(3) Xa3 is Hcys, m3 is 2, and n3 is 1 or 2.

9. A drug conjugate or a pharmaceutically acceptable salt thereof, **characterised in that** the conjugate comprises the peptide ligand according to any one of claims 1-8 conjugated to one or more effectors and/or functional groups via a linker.

10. The drug conjugate or the pharmaceutically acceptable salt thereof according to claim 9, **characterised in that** the effector is a cytotoxic agent.

11. The drug conjugate or the pharmaceutically acceptable salt thereof according to claim 10, **characterised in that** the cytotoxic agent is selected from the following group: alkylating agents, antimetabolites, plant alkaloids, terpenoids, podophyllotoxins and a derivative thereof, taxanes and a derivative thereof, a topoisomerase inhibitor, and antitumour antibiotics.

12. The drug conjugate or the pharmaceutically acceptable salt thereof according to claim 10, **characterised in that** the cytotoxic agent is selected from the following group: cisplatin, carboplatin, oxaliplatin, mechlorethamine, cyclophosphamide, chlorambucil, ifosfamide, azathioprine, mercaptopurine, a pyrimidine analogue, vincristine, vinblastine, vinorelbine, vindesine, etoposide, teniposide, taxol, camptothecine, irinotecan, topotecan, amsacrine, etoposide, etoposide phosphate, teniposide, actinomycin D, doxorubicin, epirubicin, epothilone and a derivative thereof, bleomycin and a derivative thereof, dactinomycin and a derivative thereof, plicamycin and a derivative thereof, mitomycin C, calicheamycin, maytansine and a derivative thereof, and auristatin and a derivative thereof.

13. The drug conjugate or the pharmaceutically acceptable salt thereof according to claim 10, **characterised in that** the cytotoxic agent is selected from DM1, MMAE or SN38.

14. The drug conjugate or the pharmaceutically acceptable salt thereof according to claim 9, **characterised in that** the linker is a peptide linker, a disulphide linker, an enzyme-dependent linker, or a pH-dependent linker.

15. The drug conjugate or the pharmaceutically acceptable salt thereof according to claim 14, **characterised in that** the disulphide linker is selected from DMDS, MDS, DSDM, NDMDS or a structure of formula III:

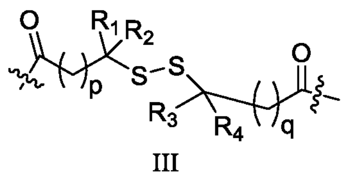

III

wherein $R_1$, $R_2$, $R_3$ and $R_4$ are independently selected from H, methyl, ethyl, propyl and isopropyl;
p and q are independently 1, 2, 3, 4 or 5;

the peptide linker is selected from: -Cit-Val-, -Phe-Lys- and -Val-Lys-; the enzyme-dependent linker is selected from:

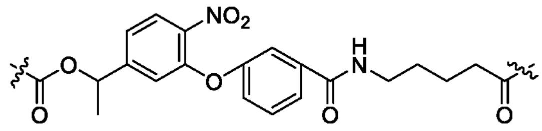

;

the pH-dependent linker is selected from a cis-aconitic anhydride.

**16.** The drug conjugate or the pharmaceutically acceptable salt thereof according to claim 14, **characterised in that** the linker is -PABC-Cit-Val-glutaryl-, - PABC-cyclobutyl-Ala-Cit-βAla-, (-PABC-Cit-Val-CO-$CH_2$)$_2$N-$CH_2$($CH_2OCH_2$)$_2$$CH_2$NH-glutaryl-, or

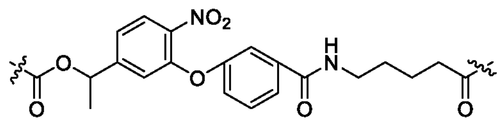

,

wherein PABC represents p-aminobenzylcarbamate.

**17.** The drug conjugate or the pharmaceutically acceptable salt thereof according to claim 9, **characterised in that** the drug conjugate has a structure of formula I, formula II, formula III, formula IV, formula V or VI:

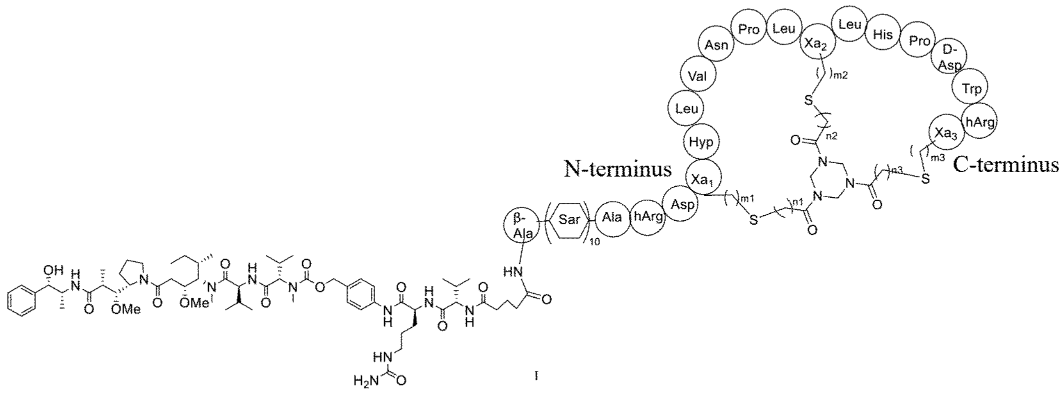

I

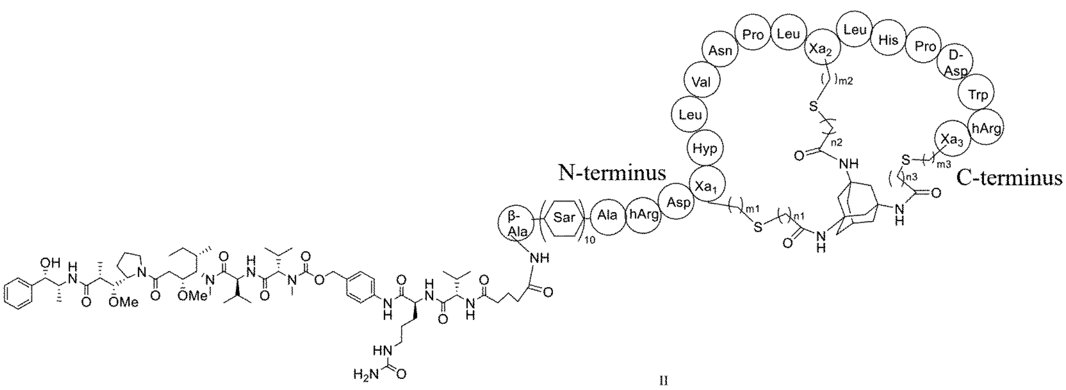

II

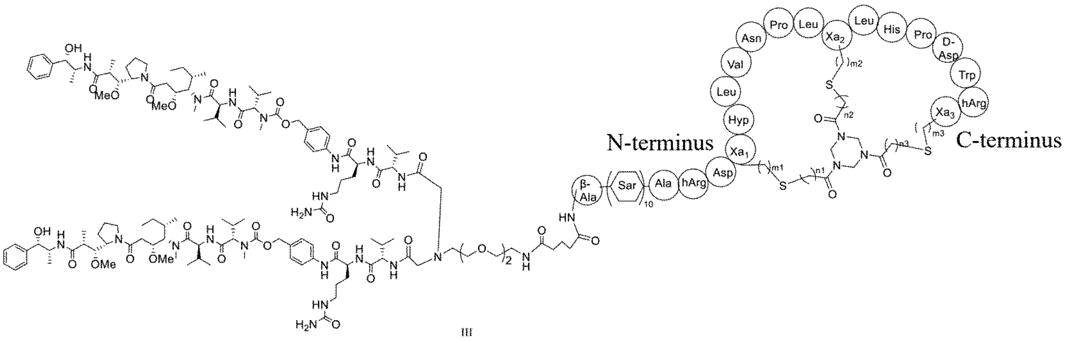

III

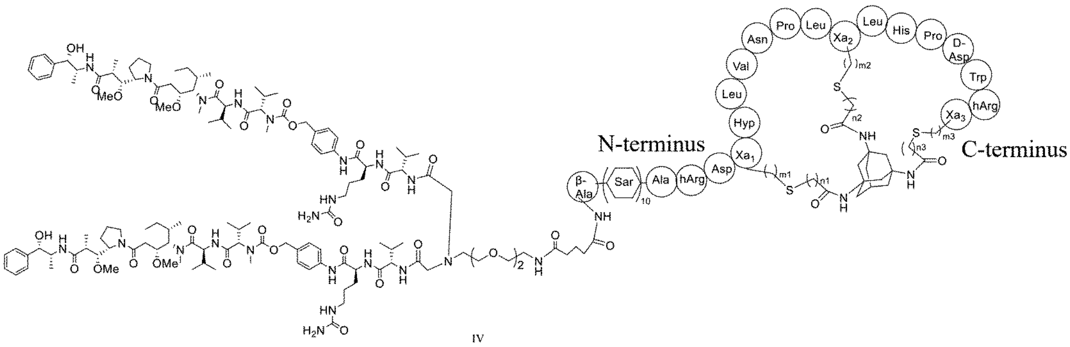

IV

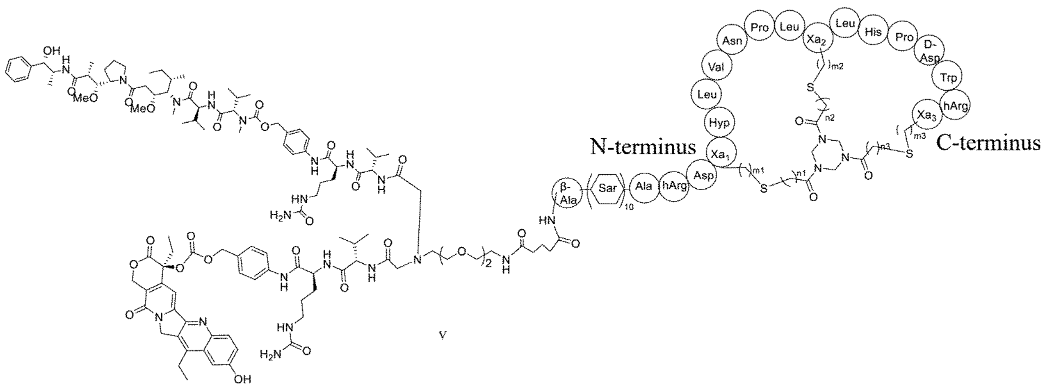

V

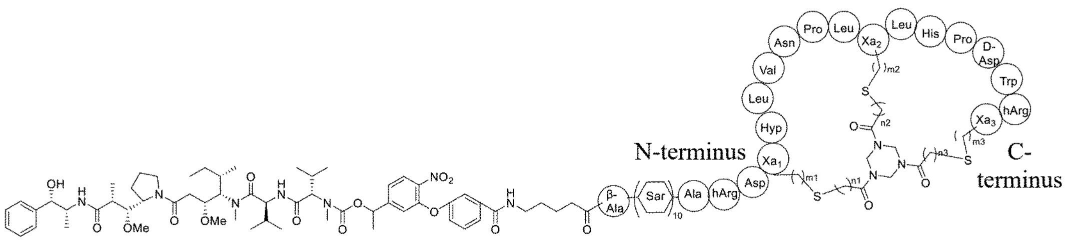

VI

wherein Xa1, Xa2 and Xa3 are independently a Cys or Hcys residue, and at least one of Xa1, Xa2 and Xa3 is a Hcys residue;
when Xa1, Xa2 or Xa3 is Cys, correspondingly, m1, m2 or m3 is 1;
when Xa1, Xa2 or Xa3 is Hcys, correspondingly, m1, m2 or m3 is 2;
n1, n2 and n3 are independently 1 or 2.

**18.** A drug conjugate or a pharmaceutically acceptable salt thereof, **characterised in that** the drug conjugate is selected from one of the following structures:

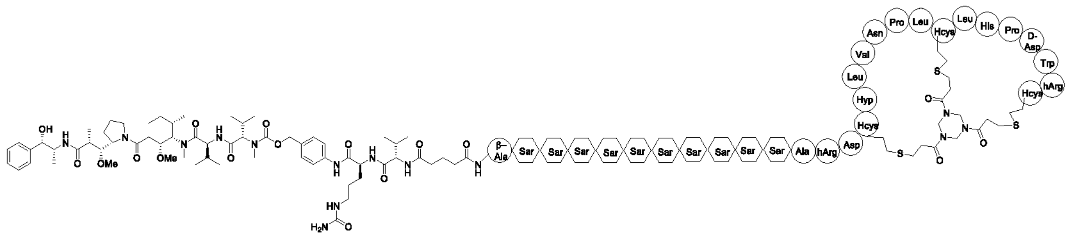

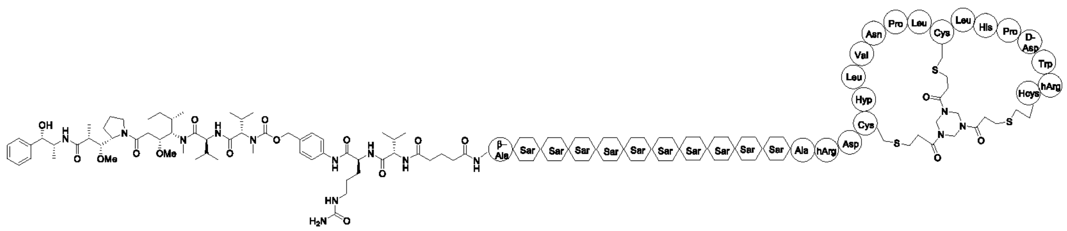

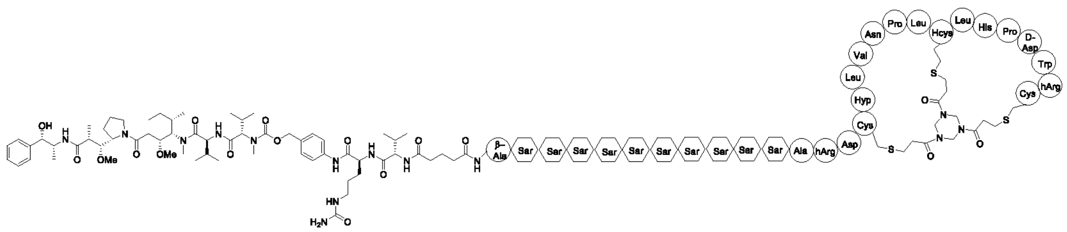

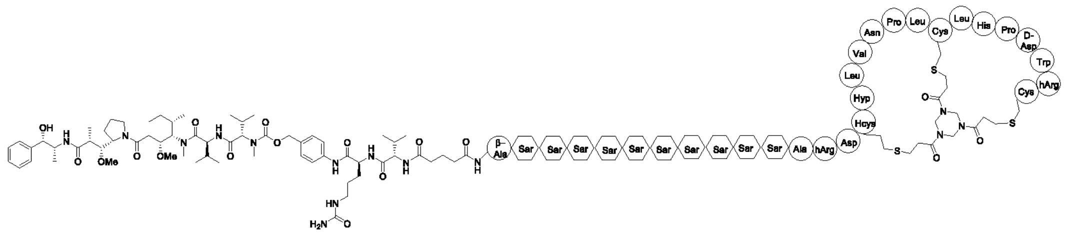

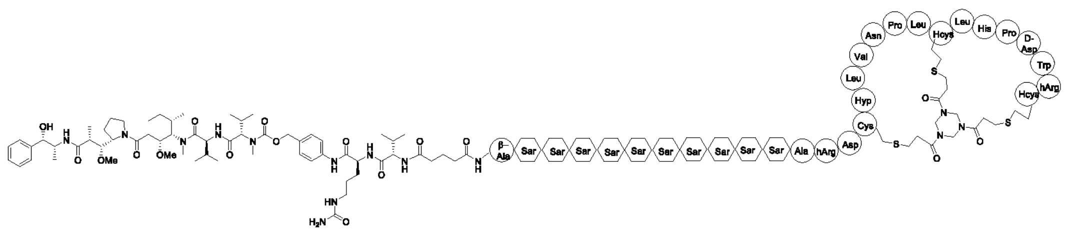

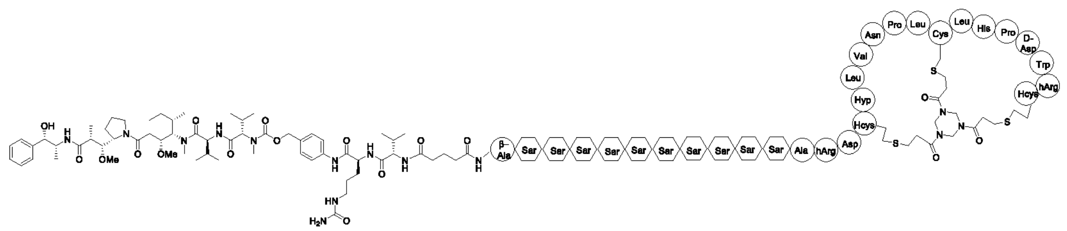

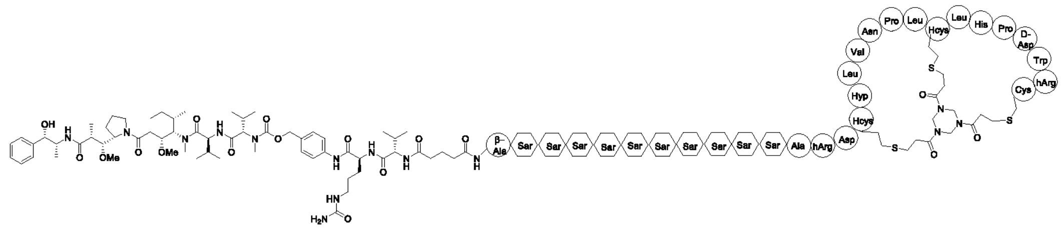

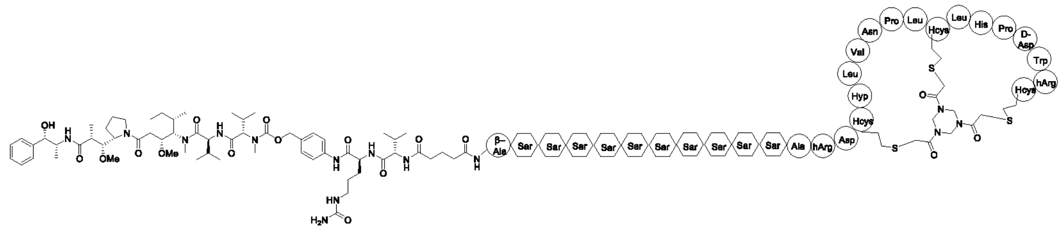

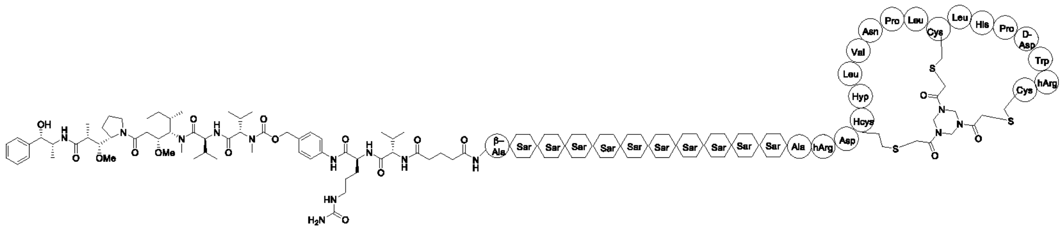

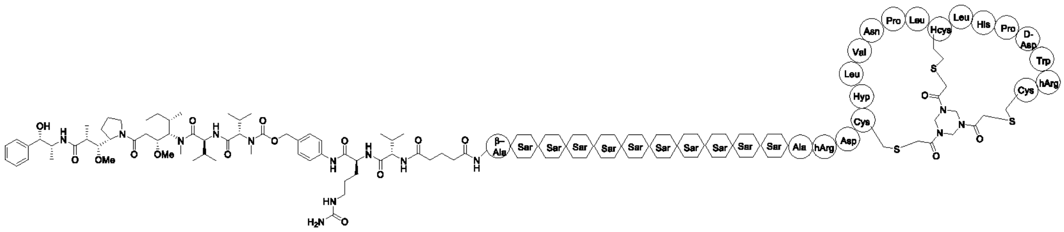

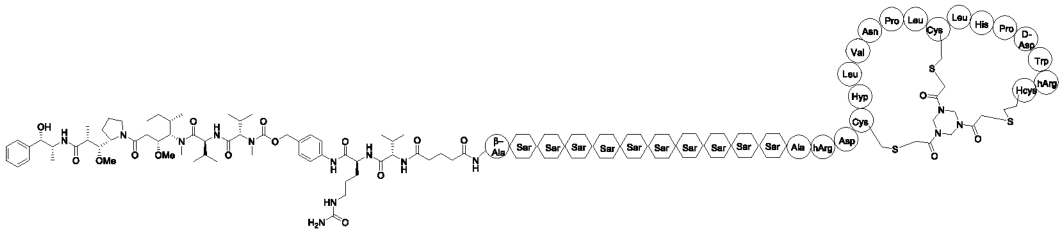

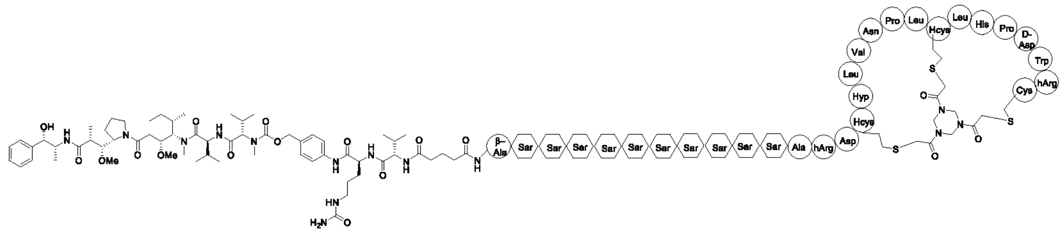

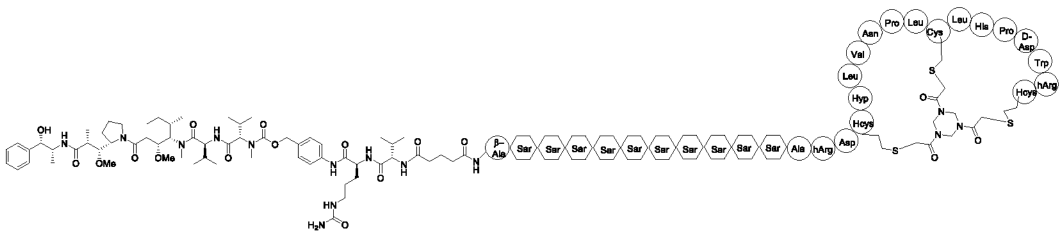

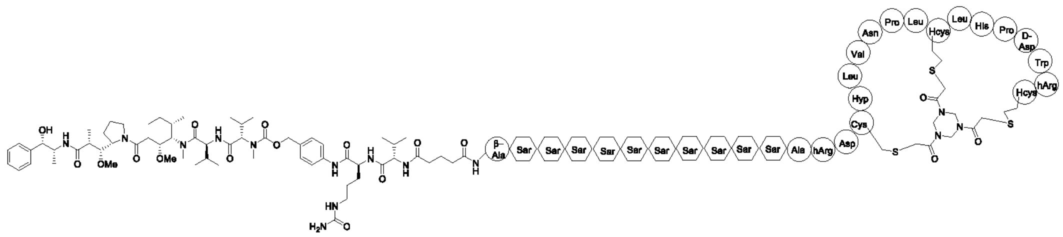

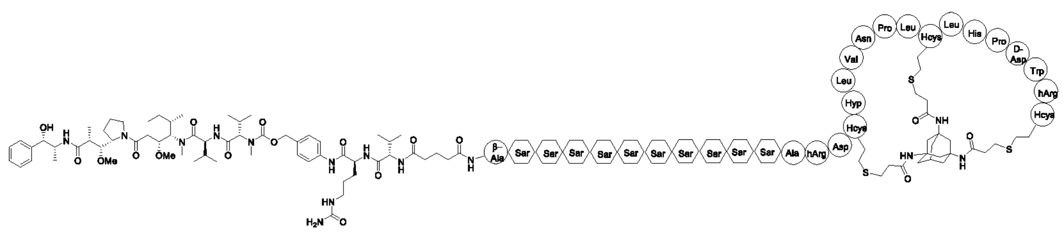

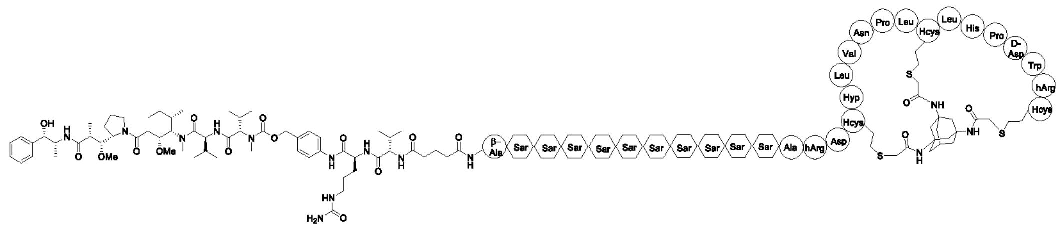

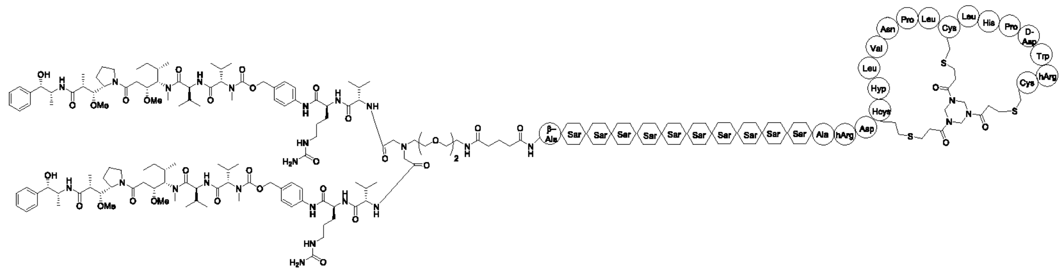

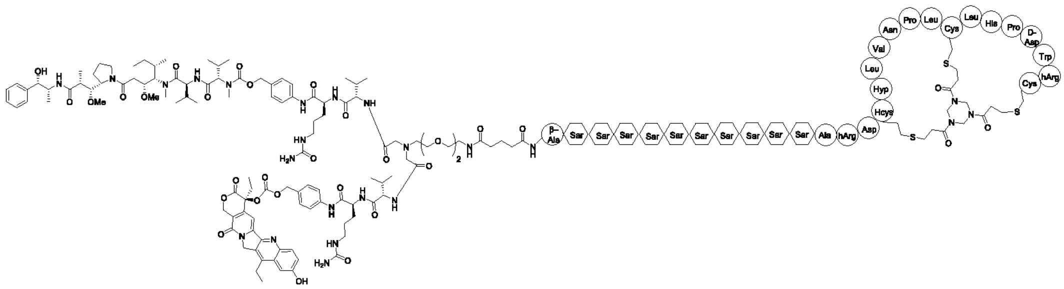

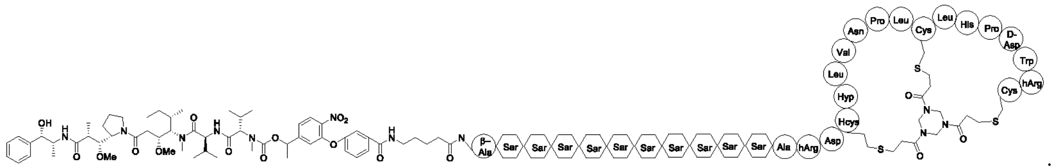

.

**19.** A pharmaceutical composition, comprising the drug conjugate or the pharmaceutically acceptable salt thereof according to any one of claims 9-18, and a pharmaceutically acceptable carrier and/or excipient.

**20.** Use of the drug conjugate or the pharmaceutically acceptable salt thereof according to any one of claims 9-18 or the composition according to claim 19 in the preparation for preventing and/or treating EphA2 overexpressed disease, **characterised in that** preferably, the EphA2 overexpressed disease is selected from: prostate cancer, lung cancer, breast cancer, gastric cancer, ovarian cancer, oesophageal cancer, multiple myeloma and fibrosarcoma.

**21.** A pharmaceutical composition or pharmaceutical preparation, comprising 1-1500 mg of the drug conjugate or the pharmaceutically acceptable salt thereof according to any one of claims 9-18, and a pharmaceutically acceptable carrier and/or excipient.

**22.** A method for treating a tumour, comprising administering to a subject a therapeutically effective amount of the drug conjugate or the pharmaceutically acceptable salt thereof according to any one of claims 9-18, **characterised in that** the subject comprises a mammal or human, the therapeutically effective amount is preferably 1-1500 mg, and the tumour is preferably prostate cancer, lung cancer, breast cancer, gastric cancer, ovarian cancer, oesophageal cancer, multiple myeloma and fibrosarcoma.

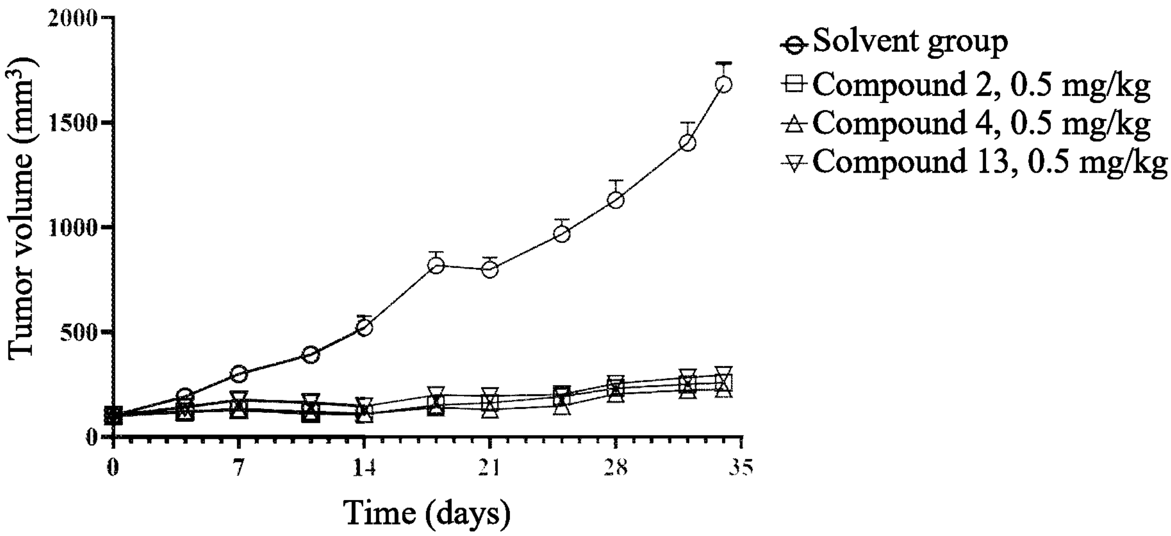
Tumor volume (mm$^3$)
2000
1500
1000
500
0
0
7
14
21
28
35
Time (days)
Solvent group
Compound 2, 0.5 mg/kg
Compound 4, 0.5 mg/kg
Compound 13, 0.5 mg/kg

*FIG. 1*

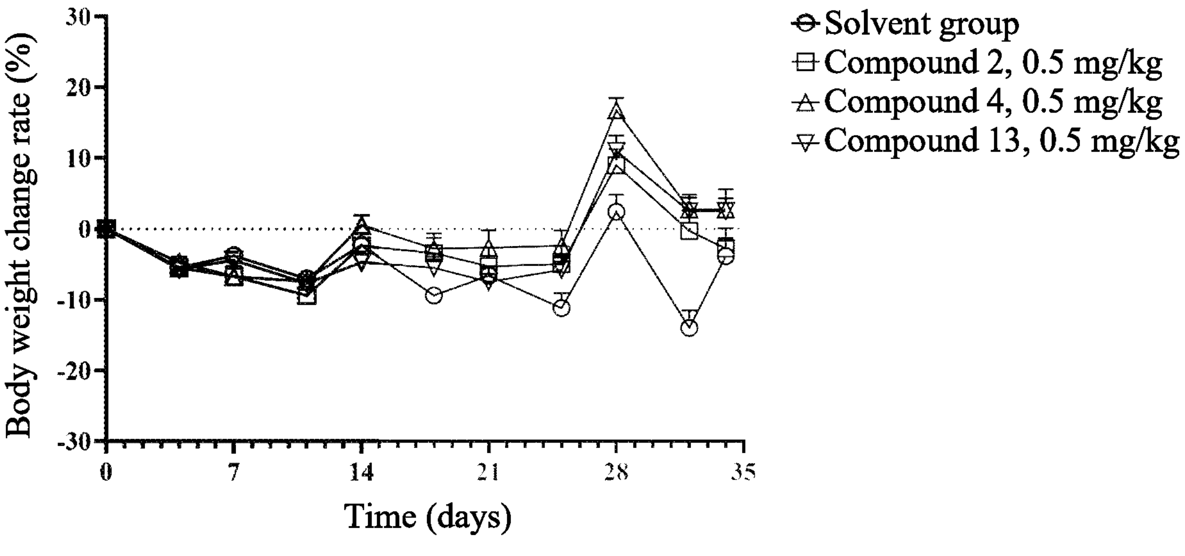
Body weight change rate (%)
30
20
10
0
-10
-20
-30
0
7
14
21
28
35
Time (days)
Solvent group
Compound 2, 0.5 mg/kg
Compound 4, 0.5 mg/kg
Compound 13, 0.5 mg/kg

*FIG. 2*

## INTERNATIONAL SEARCH REPORT

International application No.
**PCT/CN2022/125377**

**A. CLASSIFICATION OF SUBJECT MATTER**

C07K 7/08(2006.01)i； A61P 35/00(2006.01)i； A61K 47/64(2017.01)i； C07K 14/00(2006.01)i

According to International Patent Classification (IPC) or to both national classification and IPC

**B. FIELDS SEARCHED**

Minimum documentation searched (classification system followed by classification symbols)

C07K; A61P; C07K; A61K

Documentation searched other than minimum documentation to the extent that such documents are included in the fields searched

Electronic data base consulted during the international search (name of data base and, where practicable, search terms used)

CNABS, VEN, DWPI, CJFD, CNKI, CNTXT, USTXT, ISI WEB OF SCIENCE, PUBMED, GENBANK: 申请人, 发明人, Eph受体酪氨酸激酶A2, 肽配体, 双环肽, 双环, 硫醚键, EphA2, bicyclic peptide, bicycle, peptide ligand, hcys, homocysteine, hcy, SEQ ID NOs: 1-14.

**C. DOCUMENTS CONSIDERED TO BE RELEVANT**

| Category* | Citation of document, with indication, where appropriate, of the relevant passages | Relevant to claim No. |
|---|---|---|
| Y | CN 111787955 A (BICYCLETX LTD.) 16 October 2020 (2020-10-16)<br>claims 1-21, and description, paragraphs [0051]-[0062] | 1-22 |
| Y | CN 112585158 A (BICYCLERD LTD.) 30 March 2021 (2021-03-30)<br>claims 1-19, and description, paragraphs [0016] and [0025] | 1-22 |
| A | WO 2007022494 A2 (ENDOCYTE, INC.) 22 February 2007 (2007-02-22)<br>entire document | 1-22 |
| A | WO 2008010101 A2 (SANOFI-AVENTIS) 24 January 2008 (2008-01-24)<br>entire document | 1-22 |
| A | CN 111712264 A (BICYCLERD LTD.) 25 September 2020 (2020-09-25)<br>entire document | 1-22 |
| A | WO 2017191460 A1 (BICYCLE THERAPEUTICS LTD.) 09 November 2017 (2017-11-09)<br>entire document | 1-22 |
| A | MUDD, G. E. et al. "Identification and Optimization of EphA2-Selective Bicycles for the Delivery of Cytotoxic Payloads"<br>*Journal of Medicinal Chemistry,* Vol. 63, 23 March 2020 (2020-03-23),<br>pages 4107-4116 | 1-22 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **10 December 2022** | **20 December 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

INTERNATIONAL SEARCH REPORT

International application No.
PCT/CN2022/125377

C. DOCUMENTS CONSIDERED TO BE RELEVANT

| Category* | Citation of document, with indication, where appropriate, of the relevant passages | Relevant to claim No. |
|---|---|---|
| A | BENNETT, G. et al. "MMAE Delivery Using the Bicycle Toxin Conjugate BT5528" *Molecular Cancer Therapeutics,* Vol. 19, No. 7, 31 July 2020 (2020-07-31), pages 1385-1394 | 1-22 |

Form PCT/ISA/210 (second sheet) (January 2015)

INTERNATIONAL SEARCH REPORT

International application No.
PCT/CN2022/125377

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

   [1] The actually submitted sequence table is an XML file in the standard ST.26.

Form PCT/ISA/210 (continuation of first sheet) (January 2015)

**INTERNATIONAL SEARCH REPORT**

International application No.
**PCT/CN2022/125377**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **22**
because they relate to subject matter not required to be searched by this Authority, namely:

[1] Claim 22 relates to a method for treating tumor, belonging to a method for treatment of a disease defined in PCT Rule 39.1(iv). The present report is formed on the basis of a corresponding pharmaceutical use of a peptide ligand specific for EphA2. Claim 22 relates to a method for treating a tumor, belonging to a method for treatment of a disease defined in PCT Rule 39.1(iv). The present report was formed on the basis of a corresponding pharmaceutical use of a peptide ligand specific to EphA2.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2022/125377**

| Patent document cited in search report | Publication date (day/month/year) | Patent family member(s) | Publication date (day/month/year) |
|---|---|---|---|
| CN 111787955 A | 16 October 2020 | PL 3727460 T3 | 22 August 2022 |
| | | DK 3727460 T3 | 11 July 2022 |
| | | EP 3727461 A1 | 28 October 2020 |
| | | AU 2018387418 A1 | 30 July 2020 |
| | | IL 275437 A | 31 August 2020 |
| | | KR 20200105839 A | 09 September 2020 |
| | | EP 3727460 A1 | 28 October 2020 |
| | | EP 4053145 A1 | 07 September 2022 |
| | | WO 2019122863 A1 | 27 June 2019 |
| | | ES 2922632 T3 | 19 September 2022 |
| | | PH 12020550929 A1 | 17 May 2021 |
| | | IL 275440 A | 31 August 2020 |
| | | WO 2019122860 A1 | 27 June 2019 |
| | | SG 11202005494 Q A | 29 July 2020 |
| | | HU E059126 T2 | 28 October 2022 |
| | | FI 3727460 T3 | 15 July 2022 |
| | | US 2019184025 A1 | 20 June 2019 |
| | | CA 3086257 A1 | 27 June 2019 |
| | | CA 3085253 A1 | 27 June 2019 |
| | | AU 2018387417 A1 | 30 July 2020 |
| | | TW 201927306 A | 16 July 2019 |
| | | PT 3727460 T | 14 July 2022 |
| | | KR 20200105840 A | 09 September 2020 |
| | | SG 11202005495 U A | 29 July 2020 |
| | | US 2022289792 A1 | 15 September 2022 |
| | | JP 2021506910 A | 22 February 2021 |
| | | LT 3727460 T | 25 July 2022 |
| | | BR 112020012246 A2 | 24 November 2020 |
| | | BR 112020012349 A2 | 24 November 2020 |
| | | JP 2021506936 A | 22 February 2021 |
| | | CN 111741771 A | 02 October 2020 |
| CN 112585158 A | 30 March 2021 | None | |
| WO 2007022494 A2 | 22 February 2007 | US 2008248052 A1 | 09 October 2008 |
| | | BR PI0615354 A2 | 17 May 2011 |
| | | KR 20130113543 A | 15 October 2013 |
| | | CA 2617660 A1 | 22 February 2007 |
| | | EP 1948241 A2 | 30 July 2008 |
| | | US 2014058064 A1 | 27 February 2014 |
| | | EP 2374480 A2 | 12 October 2011 |
| | | RU 2012136587 A | 10 March 2014 |
| | | US 2013184435 A1 | 18 July 2013 |
| | | ES 2468240 T3 | 16 June 2014 |
| | | JP 2013079248 A | 02 May 2013 |
| | | RU 2008110495 A | 27 September 2009 |
| | | CN 103893779 A | 02 July 2014 |
| | | CN 103893778 A | 02 July 2014 |
| | | JP 2009504784 A | 05 February 2009 |
| | | AU 2006279304 A1 | 22 February 2007 |
| | | KR 20080041223 A | 09 May 2008 |
| WO 2008010101 A2 | 24 January 2008 | JP 2009543575 A | 10 December 2009 |

Form PCT/ISA/210 (patent family annex) (January 2015)

**INTERNATIONAL SEARCH REPORT**
**Information on patent family members**

International application No.
**PCT/CN2022/125377**

| Patent document cited in search report | Publication date (day/month/year) | Patent family member(s) | Publication date (day/month/year) |
|---|---|---|---|
| | | IL 243376 D0 | 29 February 2016 |
| | | KR 20090059110 A | 10 June 2009 |
| | | PE 20120431 A1 | 04 May 2012 |
| | | NI 200900005 A | 26 January 2010 |
| | | NO 20090758 L | 17 March 2009 |
| | | SV 2009003151 A | 21 January 2010 |
| | | TN 2009000007 A1 | 19 August 2010 |
| | | BR PI0714871 A2 | 07 May 2013 |
| | | IN 586KOLNP2009 A | 15 May 2009 |
| | | EC SP099074 A | 27 February 2009 |
| | | CA 2658276 A1 | 24 January 2008 |
| | | US 2015274824 A1 | 01 October 2015 |
| | | AU 2007274738 A1 | 24 January 2008 |
| | | UY 30492 A1 | 29 February 2008 |
| | | CO 6160239 A2 | 20 May 2010 |
| | | CR 10558 A | 13 September 2010 |
| | | PE 30 April 2008 A1 | 19 May 2008 |
| | | ZA 200900545 B | 31 March 2010 |
| | | CN 101622276 A | 06 January 2010 |
| | | EP 2044122 A2 | 08 April 2009 |
| | | CL 2012001387 A1 | 28 September 2012 |
| | | JP 2014221757 A | 27 November 2014 |
| | | MA 30634 B1 | 03 August 2009 |
| | | EA 200970130 A1 | 30 June 2009 |
| | | MY 157757 A | 15 July 2016 |
| | | AR 061911 A1 | 01 October 2008 |
| | | CL 2007002085 A1 | 18 April 2008 |
| | | US 2013302316 A1 | 14 November 2013 |
| | | GT 200900002 A | 03 November 2010 |
| | | IL 196470 D0 | 01 August 2011 |
| | | NZ 574215 A | 27 July 2012 |
| | | ES 2673822 T3 | 25 June 2018 |
| | | US RE47123 E | 13 November 2018 |
| | | MX 2009000709 A | 04 February 2009 |
| | | ME 00581 B | 20 December 2011 |
| | | US 2010047257 A1 | 25 February 2010 |
| | | TW 200821329 A | 16 May 2008 |
| CN 111712264 A | 25 September 2020 | WO 2019122861 A1 | 27 June 2019 |
| | | GB 201721265 D0 | 31 January 2018 |
| | | EP 3727462 A1 | 28 October 2020 |
| | | US 2020338203 A1 | 29 October 2020 |
| | | JP 2021507909 A | 25 February 2021 |
| WO 2017191460 A1 | 09 November 2017 | JP 2019523214 A | 22 August 2019 |
| | | EP 3452096 A1 | 13 March 2019 |
| | | GB 201607827 D0 | 15 June 2016 |
| | | CN 109414510 A | 01 March 2019 |
| | | US 2019134213 A1 | 09 May 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **TIMMERMAN et al.** *ChemBioChem,* 2005
- **HEINIS et al.** Angewandte Chemie. 2014, vol. 53, 1602-1606
- Angewandte Chemie. 2014, vol. 53, 1602-1606